# EUROPEAN PATENT APPLICATION

(11) **EP 4 742 268 A2**
(43) Date of publication of application: **13.05.2026**
(21) Application number: 25216773.9
(22) Date of filing: 11.02.2019
(51) Int. Cl.: G16H 50/30

(54) **A METHOD OF GENERATING A STATISTICAL MODEL TO DETECT, OR PREDICT THE RISK OF, AN OUTCOME**

(30) Priority: 09.02.2018 GB 201802206; 16.05.2018 EP 18172720
(62) Divisional of application: 19708778.6
(71) Applicant: Metabolomic Diagnostics Limited, Co. Cork (IE)
(72) Inventor: TUYTTEN, Robin, Cork (IE); THOMAS, Gregoire, Lokeren (BE)
(74) Representative: Purdylucey Intellectual Property

(57) **Abstract**

A system and method of generating a model M that can detect or predict the predisposition to an outcome, for example a person's predisposition to developing a health condition such as preeclampsia, with a positive or/and negative predictive value better or equal to a predefined predictive value target, where the method factors in the impact of prevalence on test performance. The system and method employ an iterative population segregation methodology, involving at least two population segregations steps in which each step, independently, employs a probability-defined model. The first segregation step is selected from one of a rule-in probability defined model and a rule-out probability defined model, and the second segregation step is selected from the other of a rule-in probability defined model and a rule-out probability defined model. The present invention overcomes the technical limitations of existing predictive models and provides a solution to achieve a superior predictive model delivering an accurate risk prediction result, and in a less computationally intensive way. By isolating and segmenting particular population subsets according to the invention a much more robust and accurate way of detecting, or predict risk of, an outcome is achieved.

## Description

### Field of the Invention

The present invention relates to a system and method of generating a model M to detect, or predict the risk of, an outcome. In particular, the invention relates to a method of generating a model M to detect, or predict an outcome, in particular the risk of a health condition in a subject. Also contemplated are uses of the model to detect or predict the outcome, and in particular predict risk of a subject developing a health condition.

### Background to the Invention

Classically, to identify a population at high risk of a defined outcome (such as developing a specific health condition), it is common to lock the false positive rate (FPR, 1-specificty) to a target value and then, for any given classifier, to observe at which sensitivity (detection rate of future cases) the ROC curve crosses the specificity criterion. Conversely, to identify a population at low risk, it is common to lock the false negative rate (FNR, 1-sensitivity) to a target value and then, for any given classifier, to observe at which specificity (detection for future non-cases) the ROC curve crosses the FNR criterion to identify a population at low risk. Typically, one will first develop a prognostic model which maximizes AUROC and then establish its estimated detection rate at the set criterion. However, prognostic models with high AUROC are not necessarily the best models when the intended clinical application is either rule-in or rule-out [1]. What is more, prognostic models typically do not have high AUROC; for instance, one of the most used prognostic models is the Framingham risk model for prediction of cardiovascular risk, discriminates only reasonably in certain (sub)populations, with a receiver-operating characteristic (ROC) curve area of little over 0.70 [2][3].

The statistics AUROC, Sn, and Sp are considered prevalence-independent statistics, yet prevalence (or incidence; depending on the application) is important when assessing the clinical usefulness of a prognostic test. When a prognostic test is assessed / applied in its clinically relevant context, metrics like positive and negative predictive value (PPV and NPV), which take the disease prevalence (or incidence) into account, are more appropriate. Here, PPV corresponds the fraction of patients that will actually develop the condition (TP, True Positives) within the group of all patients that have a positive test result (True Positives + False Positives (FP)); or in other words, PPV is the probability that the disease is present when the test is positive. NPV corresponds to the fraction of patients that will actually not develop the conditions (TN, True Negatives) within the group of all patients that have a negative test result (True Negatives + False Negatives (FN)), or in other words NPV is the probability that the disease is not present when the test is negative.

Typically, a prognostic rule-in test should 1) identify a minimum proportion of the patients that will actually develop the disease and 2) ensure that this true positive group has a sufficiently large proportion of the patients testing positive. In other words, such prognostic tests must reach a minimum sensitivity and minimum PPV. Likewise, a prognostic rule-out test should 1) identify a minimum proportion of patients that will certainly not develop the disease and 2) ensure that of the patients testing negative, sufficiently few will develop the disease (false negatives). Such test must therefore reach a minimum specificity and minimum negative predictive value (NPV).

It is easily understood that predictive values are important determinants of the performance of a classifier, as it allows quantifying the "cost" associated with a change of clinical pathway following a prognostic test result. For illustrative purposes, consider the following hypothetical scenario. If the total monetary cost (and/or health cost as a result of an undesired side effects) of an available prophylactic treatment is high, a health care system might determine, based on a cost-benefit analysis, that it can support treatment of a high-risk group with a 1:5 chance of developing the condition (i.e., where minimally 1 in 5 of those treated will eventually develop the condition (and therefore warrants treatment) and maximally 4 in 5 are false positives, and hence will be needlessly offered the treatment). This criterion translates to a prognostic test which should select a high-risk group with a PPV = 0.2.

In this scenario, a test which classifies 50% of future cases into a high-risk group with a PPV = 0.2 is considered better (from the health economics point of view) than a test which classifies 75% of future cases in a high-risk group with a PPV = 0.1. The latter would amount to a "cost" of treating 9 False Positives for each True Positive, which would be deemed not fit-for-purpose by the health care system where the cost of the prophylactic treatment is high. The limitations of defining rule-in or rule-out tests using either a fixed FPR (rule-in) or a fixed FNR (rule-out) are exemplified in Example 1. This also limits the utility of AUROC analysis as widely applied for the assessment of prognostic tests.

Prediction, prevention and early intervention, are cornerstones of modern healthcare. To deliver upon these concepts, healthcare providers need to firstly identify people at risk of developing disease or who already have a subclinical disease. It is only when such groups can be identified early that patients can benefit maximally from appropriate treatments. Typically, healthcare systems will utilise prognostic, pre-disposition or screening tests to identify these target groups. Screening approaches can be very effective for those diseases which have a well-defined cause like viral infections (e.g., early diagnosis of HIV infection as disclosed in Lucas A, Armbruster B. "The cost-effectiveness of expanded HIV screening in the United States" [4], or genetic disorders (e.g., non-invasive prenatal testing for aneuploidy in pregnancy as disclosed in Dondorp W, De Wert G, Bombard Y, et al. "Non-invasive prenatal testing for aneuploidy and beyond: Challenges of responsible innovation in prenatal screening" [5]. In these examples, effective healthcare can be provided because the tests to identify the target populations combine
- very high test sensitivity (Sn), whereby Sn is a measure of the ability to actually identify these people will develop the disease / have subclinical disease in the population which will truly develop the disease / truly have subclinical, and
- very high test specificity (Sp), whereby Sp is a measure of ability to actually identify these people that will not develop the disease / not have subclinical disease in the population which will truly not develop the disease / truly not have subclinical disease.

As a result, these tests also deliver:
- good positive predictive value (PPV), whereby PPV is the fraction of people that have or will develop the disease within the population of people who all tested positive on the test, i.e. a positive test result corresponds to developing the disease / having the disease, and
- good negative predictive value (NPV), whereby NPV is the fraction of people that do not have and will not develop the disease within the population of people who all tested negative on the test.

However, for many diseases, a problem with current tests to identify those people who have or are at risk of developing a disease are characterised by sub-optimal test characteristics (Sn, Sp, PPV & NPV). In these cases, health care systems have to balance health care resources, the wishes of clinicians, and the expectations of the public.

There is a trend within health care systems to use cost-benefit analyses to establish an economically viable PPV for a test rather than relying on Sensitivity (Sn), as highlighted in a publication entitled "Prevention of cardiovascular disease: guidelines for assessment and management of cardiovascular risk" by the World Health Organisation[6]. What is more for technically implemented model solutions, when the prevalence (or pre-test probability) of a disease is very low, even tests with "good" Sensitivity and Specificity, can have a surprisingly low post-test probability (PPV) as demonstrated in this table;

| Disease prevalence | Sensitivity (%) | False Positive Rate (1-Specificty) (%) | Positive Predictive Value |
|---|---|---|---|
| 1% | 90% | 10% | 0.083 |
| 10% | 90% | 10% | 0.500 |

It is desirable to mitigate the trade-off between the need to limit the number of False positives per True positive (expressed as a PPV threshold) and to identify as many of the people who have or are at risk of developing, a disease (increasing Sensitivity).

When developing technically implemented models for medical use, one should also take the purpose of the model into account. In diagnostic models, the goal is the accurate classification of individuals into their true disease states. When developing prognostic models, however, the goal is more technically complex. In this context, correctly predicting whether a future event will occur is more difficult owing to its stochastic nature. It is more appropriate therefore to seek to classify people into risk strata to ensure that those at risk can avail of mitigating treatments.

In order to generate good prediction tests, test developers will often try to combine multiple predictors into a prediction model, to improve test performance. However, especially in prognosis, the addition of more predictors often results in futile test improvements, especially when assessed solely by means of estimating the area under the ROC curve (or the C-statistic), which is the metric most used to assess prognostic and/or diagnostic tests. What is more, Royston et al. noted that the AUROC of prognostic models is typically between 0.6 and 0.85[7], an AUROC range which is generally insufficient to achieve good PPV and good Sn at the same time. There is therefore a need based on current techniques, that merely considering more predictors (or variables) in prognostic algorithms will deliver the often sought-for increments in prognostic performance.

From the above, it is clear that developing "good" prognostic tests is non-trivial and complicated task. Taken together with the fact that achieving adequate post-test probabilities for tests for low prevalent diseases is even more difficult to achieve.

It is an object of the invention to overcome at least one of the above-referenced problems. It is a further object of the invention to leverage sets of available predictors differently, in order to overcome the limited prognostic performance gains typically achieved by combining predictors in increasingly complex models.

### Summary of the Invention

The invention provides, as set out in the appended claims, a system, a computer program product and method of generating a model that can be employed to detect, or predict risk of, an outcome, for example the risk of a subject developing a health condition in the context of a diagnostic or prognostic test.

In particular, the invention provides a method of generating a model that can detect or predict the predisposition to an outcome, with a positive or/and negative predictive value better or equal to a predefined predictive value target, and hence the method factors in the impact of prevalence on test performance. The methods employ an iterative population segregation methodology, involving at least two population segregations steps in which each step, independently, employs a probability-defined model.

The first segregation step is selected from one of a rule-in probability defined model and a rule-out probability defined model, and the second segregation step is selected from the other of a rule-in probability defined model and a rule-out probability defined model.

The method employs iterative population segregation methodology, to obtain enriched sub-populations of subjects having (a predisposition for) an outcome, or /and enriched sub-populations of subjects not having (a predisposition for) an outcome. The method comprises the generation of PPV-defined models with high detection rates of subjects with the (predisposition for) the outcome, or/and NPV-defined models with high detections rates of subjects not having (a predisposition for) the outcome.

The present invention overcomes the technical limitations of existing predictive models and provides a solution to achieve a superior predictive model delivering an accurate risk prediction result, and this in a less computationally intensive way. By isolating and segmenting particular population subsets in a way as defined in claim 1 a much more robust and accurate way of detecting, or predict risk of, an outcome is achieved. A significant improvement in the accuracy of prognostic models is achieved when comparing the figures of merit for two prediction models established with and without the application of the invention, whereby both models use the same four variables:

| **Prognostic model type** | **Variables used in model** | **PPV target (fixed)** | **Sn (detection rate of future disease cases (%))** | **Reference to exemplification section** |
|---|---|---|---|---|
| Single step stratification (classic, without application of invention) | PLGF, DIG, sENG, L-ERG | 0.071 | 0.65 (65%) | Example 4B |
| Stratification using segregation methodology as per this invention | PLGF, DIG, sENG, L-ERG | 0.071 | 0.91 (91%) | Example 4D |

It is clear that for a given PPV-target, increasing the detection rate of future disease cases from 65% to 1 % is exceptional in the context of a prognostic model.

The iterative population segregation methodology includes at least two segregation steps. The first segregation step employs a first model to segregate the test population (or a subset thereof) to generate two sub-populations A1 and B1 in which sub-population A1 is enriched in subjects that either have the (predisposition for the) condition or do not have the (predisposition for the) condition (i.e. one of a rule-in or rule-out model). The second segregation step employs a second model to segregate sub-population B1 to generate two sub-populations A2 and B2 in which sub-population A2 is enriched in subjects that either have the (predisposition for the) condition or do not have (predisposition for the) the condition (i.e. the other of the rule-in or rule-out models). Each model is configured to generate a sub-population whose subjects have one of two probabilities selected from rule-in cut-off and rule-out cut-off probabilities.

For example, the first model may be configured to generate a sub-population A1 whose subjects have a probability to have (/develop) a health condition lower than or equal to a predefined rule-out cut-off probability, and the second model may be configured to generate a sub-population A2 whose subjects have a probability to have (/develop) a health condition higher than or equal to a predefined rule-in cut-off probability. Alternatively, the first model may be configured to generate a sub-population A1 whose subjects have a probability to have(/develop) the health condition higher than or equal to a predefined rule-in cut-off probability, and the second model may be configured to generate a sub-population A2 whose subjects have a probability to have(/develop) the health condition lower than or equal to a predefined rule-out cut-off probability.

Taking a "rule-out then rule-in" example, the first segregation step employs a rule-out model configured to generate a sub-population A1 whose subjects have a probability to have (/develop) the health condition lower than or equal to a predefined rule-out cut-off probability. Sub-population A1, which is enriched in subjects that do not have (the predisposition for) the condition, is removed and the next segregation step is performed on the remaining subjects, i.e., sub-population B1, which now has a higher prevalence of subjects with (a predisposition for) the health condition than the starting population. In other words sub-population B1 is enriched in subjects that have (the predisposition for) the condition. The second segregation step employs a rule-in model on sub-population B1 that is configured to generate a sub-population A2 whose subjects have a probability to have (/develop) the health condition higher than or equal to a predefined rule-in cut-off probability. These two segregation steps in combination generate a sub-population that is further enriched in subjects having the (predisposition for) the health condition (i.e. has a much higher prevalence of (predisposition for) the health condition than the starting population).

The method of the invention may employ three or more segregation steps, in which at least two successive steps conform to the rule-in/rule-out or rule-out/rule-in directed segregation steps described above. Possible embodiments of the invention include rule-in/rule-out/rule-in and rule-out/rule-in/rule-out, segregation steps. The method of the invention may employ consecutive segregation steps until an n^{th} generation population sub-set is generated which cannot be segregated any further. This "stopping" event will occur
1) when no successful segregation model can be found, based on the variables available for the subjects in the n^{th} generation population; or
2) when the n^{th} generation population in its entirety meets the pre-set PPV rule-in cut-off probability or the pre-set NPV rule-out cut-off probability;or 3) when the n^{th} generation population in its entirety is added to the composite constituting the combined earlier generation population rule-in subsets, the resulting composite rule-in population meets the pre-set PPV rule-in cut-off probability, or when the n^{th} generation population in its entirety is added to the composite constituting the combined earlier generation population rule-out subsets, the resulting composite rule-out population meets the NPV rule-out cut-off probability.

When the methods are employed with the aim of finding a diagnostic / prognostic rule-in test with a PPV equal or better than a pre-set cut-off, the final segregation step generally employs a PPV-defined model.

When the methods are employed with the aim of finding a diagnostic / prognostic rule-out test with a NPV equal to or better than a pre-set cut-off, the final segregation step generally employs a NPV-defined model.

When the methods are employed with the aim of finding a combined rule-in - rule-out diagnostic / prognostic test with a PPV equal or better than a pre-set cut-off, and a NPV equal or better than a pre-set cut-off, any of the segregation steps may consider either a PPV-or NPV-defined model, in such a way that the composite rule-in populations, as obtained at different segregation steps, at least meet the pre-set PPV cut-off, and the composite rule-out populations, as obtained at different segregation steps, at least meet the pre-set NPV cut-off.

When the methods of the invention are directed to detecting or predicting risk of a health condition, the method generally employs a test population of subjects, at least some of whom have or will develop the condition. Measurement data for a plurality of diagnostic or prognostic variables are obtained from each of the subjects. The variables may be selected from biometric, life-style and physiological characteristics, and in one particular embodiment, the variables will include biological molecules such as proteins, metabolites or combinations thereof. The measurement data may be obtained from a test population in which some of the subjects have the health condition at the time of measurement, or a test population in which none of the subjects have the condition (or symptoms of the condition) at the time of measurement but subsequently develop the condition (i.e. a nested study for use in generating a model for predicting the risk of developing the health condition). An example of the latter would be a test population used in generating a model for predicting risk of developing preeclampsia in a woman at an early stage of pregnancy, or a model for predicting risk of developing secondary cancers in patients with primary cancer. In both cases, the subjects in the test population do not have the target health condition at the time of measurement.

In a first aspect, the invention provides a method of generating a model M for detecting, or predicting the risk of, an outcome, the method comprising the steps of providing a population of test samples (i.e. subjects, scenarios, products) and measurement data for a plurality (n) of variables for each of the test samples, and from this, generating a first model M1, comprising 1 to n variables, configured to predict the presence or risk of development of the outcome. The model M1 is configured to segregate the population of test samples into first generation population subsets A and B in which the samples in the first generation population subset A have a probability selected from one of a rule-out probability (i.e. a probability of the outcome lower than or equal to a predefined rule-out cut-off probability) and a rule-in probability (i.e. a probability of the outcome higher than or equal to a predefined rule-in cut-off probability). The population of samples is then segregated based on the first model M1 into first generation population subsets A and B.

When the first generation population subset B comprises a sufficient number of samples, a second model M2 is generated comprising 1 to n variables, that is configured to predict the presence or risk of the outcome in the first generation population subset B. The model M2 is configured to segregate the first generation population subset B into second generation population subsets A and B in which the samples the second generation population subset A have a probability selected from the other of a rule-out probability (i.e. a probability of the outcome lower than or equal to a predefined rule-out cut-off probability) and a rule-in probability (i.e. a probability of the outcome higher than or equal to a predefined rule-in cut-off probability). The first generation population subset B is then segregated based on the second model M2 into second generation population subset A and B. The model M comprises the first model M1 and the second model M2 that is generally applied to a sample sequentially. In use, the two models may be combined into a single algorithm, or two algorithms employed in sequence. A value can be outputted representative or indicative of a detection, or a prediction of risk, of the health condition in the subject based on said model M.

The method of the invention can be applied to generate models for predicting different types of outcome, in a number of different fields including medicine, radiology, biometrics, forecasting of natural hazards, meteorology, machine learning, and data mining research. One particular area of application is the area of medical diagnostics and prognostic, especially predicting the risk of a subject having or developing a particular health condition, especially syndromic conditions such as preeclampsia.

In one aspect, the invention provides a method of generating a model M for detecting, or predicting risk of, a health condition in a subject, the method comprising the steps of providing a population of test subjects and measurement data for a plurality (n) of variables for each of the test subjects selected from biometric, life-style and physiological characteristics, and from this, generating a first model M1, comprising 1 to n variables, configured to predict the presence or predisposition of developing a health condition in the population of test subjects. The model M1 is configured to segregate the population of test subjects into first generation population subsets A and B in which the subjects in the first generation population subset A have a probability selected from one of a rule-out probability (i.e. a probability to have/develop the health condition lower than or equal to a predefined rule-out cut-off probability) and a rule-in probability (i.e. a probability to have/develop the health condition higher than or equal to a predefined rule-in cut-off probability). The population of subjects is then segregated based on the first model M1 into first generation population subsets A and B.

When the first generation population subset B comprises a sufficient number of subjects, a second model M2 is generated comprising 1 to n variables, that is configured to predict the presence or predisposition of developing a health condition in the first generation population subset B. The model M2 is configured to segregate the first generation population subset B into second generation population subsets A and B in which the subjects the second generation population subset A have a probability selected from the other of a rule-out probability (i.e. a probability to have/develop the health condition lower than or equal to a predefined rule-out cut-off probability) and a rule-in probability (i.e. a probability to have/develop the health condition higher than or equal to a predefined rule-in cut-off probability). The first generation population subset B is then segregated based on the second model M2 into second generation population subset A and B. The model M comprises the first model M1 and the second model M2 applied sequentially. In use, the two models may be combined into a single algorithm, or two algorithms employed in sequence.

In one embodiment, the subjects in the first generation population subset A have a probability to have or to develop the health condition lower than or equal to a predefined rule-out cut-off probability, and the subjects in the second generation population subset A have a probability to have or to develop the health condition higher than or equal to a predefined rule-in cut-off probability. When the method is restricted to two steps, the model M2 is generally configured to segregate the first population subset B such that the subjects in the second generation population subset A have a probability to have or to develop the health condition higher than or equal to a predefined rule-in PPV cut-off.

In one embodiment, the subjects in the first generation population subset A have a probability to have or to develop the health condition higher than or equal to a predefined rule-in cut-off probability, and the subjects in the second generation population subset A have a probability to have or to develop the health condition lower than or equal to a predefined rule-out cut-off probability. When the method is restricted to two steps, the model M2 is generally configured to segregate the first population subset B such that the subjects in the second generation population subset A have a probability to have or to develop the health condition lower than or equal to a predefined rule-in NPV cut-off.

In one embodiment, the method includes an additional segregation step comprising:
When the second generation population subset B comprises a sufficient number of subjects, generating a third model M3 comprising 1 to n variables, configured to predict the presence or risk of developing a health condition in the second generation population subset B, wherein the model M3 is configured to segregate the second generation population subset B into third generation population subsets A and B in which the subjects the third generation population subset A have a probability selected from one of: a probability to have/develop the health condition lower than or equal to a predefined rule-out probability (for example a predefined NPV cut-off); or a probability to have/develop the health condition higher than or equal to a predefined rule-in probability (for example, a predefined PPV cut-off); and
segmenting the other of the second generation population subsets based on the third model M3 into third generation population subsets A and B.

In one embodiment, the first model M1 is configured to segment the population of test subjects such that the subjects the first generation population subset A have a probability to have/develop the health condition higher than a predefined rule-in cut-off probability;
the second model M2 is configured to segment the first generation population subset B such that the subjects in the second generation population subset A have a probability to have/develop the health condition lower than a predefined rule-out cut-off probability; and the third model M3 is configured to segment the second generation population subset B such that the subjects in the third generation population subset A have a probability to have/develop the health condition higher than a predefined rule-in PPV cut-off. [In > Out > In]

In one embodiment, the first model M1 is configured to segment the population of test subjects such that the subjects the first generation population subset A have a probability to have/develop the health condition lower than a predefined rule-out cut-off probability;
the second model M2 is configured to segment the first generation population subset B such that the subjects in the second generation population subset A have a probability to have/develop the health condition higher than a predefined rule-in cut-off probability; and the third model M3 is configured to segment the second generation population subset B such that the subjects in the third generation population subset A have a probability to have/develop the health condition lower than a predefined rule-out NPV cut-off. [Out > In > Out]

In one embodiment, the first model M1 is configured to segment the population of test subjects such that the subjects the first generation population subset A have a probability to have/develop the health condition higher than a predefined rule-in PPV cut-off;
the second model M2 is configured to segment the first generation population subset B such that the subjects in the second generation population subset A have a probability to have/develop the health condition lower than a predefined rule-out cut-off probability; and
the third model M3 is configured to segment the second generation population subset B such that the subjects in the third generation population subset A have a probability to have/develop the health condition so that the composite rule-in population constituting the combined first generation population subset A and the third generation population A have a probability higher than a predefined rule-in PPV cut-off. [In > Out > In -composite]

In one embodiment, the first model M1 is configured to segment the population of test subjects such that the subjects the first generation population subset A have a probability to have/develop the health condition lower than a predefined rule-out NPV cut-off;
the second model M2 is configured to segment the first generation population subset B such that the subjects in the second generation population subset A have a probability to have/develop the health condition higher than a predefined rule-in cut-off probability; and
the third model M3 is configured to segment the second generation population subset B such that the subjects in the third generation population subset A have a probability to have/develop the health condition so that the composite rule-out population constituting the combined first generation population subset A and the third generation population A have a probability lower than a predefined rule-out NPV cut-off. [Out > In > Out -composite]

In one embodiment, the method includes additional segregation steps, for example 1, 2, 3, 4, 5, 6, 7, 8, 9 or 10 additional steps. Each additional segmentation steps may comprise the steps of:
when the n^{th} generation population subset B comprises a sufficient number of subjects, generating a (n+1)^{th} model Mⁿ⁺¹ comprising 1 to n variables, configured to predict the presence or risk of developing a health condition in the n^{th} generation population subset B, wherein the model Mⁿ⁺¹ is configured to segregate the n^{th} generation population subset B into (n+1)^{th} generation population subset A and B such that the subjects in the (n+1)^{th} generation population subset A have a probability selected from one of: a probability to have/develop the health condition lower than or equal to a predefined rule-out NPV cut-off; or a probability to have/develop the health condition higher than or equal to a predefined rule-in PPV cut-off; and
segmenting the n^{th} generation population subset B based on the (n+1)^{th} multivariable model into (n+1)^{th} generation population subsets A and B.

Generally, the model employed in the final segregation step in the method is configured to segregate the population into subsets in which one of the subsets have a probability selected from one of: a probability to have/develop the health condition lower than or equal to a predefined rule-out NPV cut-off; or a probability to have/develop the health condition higher than or equal to a predefined rule-in PPV cut-off.

In one embodiment, successive segmentation steps are repeated until a population subset is generated that cannot be further segmented.

In one embodiment, the method includes additional segregation steps, for example 1, 2, 3, 4, 5, 6, 7, 8, 9 or 10 additional steps. Each additional segmentation steps may comprise the steps of:
when the n^{th} generation population subset B comprises a sufficient number of subjects, generating a (n+1)^{th} model Mⁿ⁺¹ comprising 1 to n variables, configured to predict the presence or risk of developing a health condition in the n^{th} generation population subset B, wherein the model Mⁿ⁺¹ is configured to segregate the n^{th} generation population subset B into (n+1)^{th} generation population subset A and B,
wherein when the subjects in the (n+1)^{th} generation population subset A are added to the combined earlier generation population rule-in subsets A, the composite rule-in population have a probability higher than or equal to a predefined rule-in PPV cut-off to have/develop the condition; or
wherein when the subjects in the (n+1)^{th} generation population subset A are added to the combined earlier generation population rule-out subsets A, the composite rule-out population have a probability lower than or equal to a predefined rule-out NPV cut-off to have/develop the condition; and
segmenting the n^{th} generation population subset B based on the (n+1)^{th} multivariable model into (n+1)^{th} generation population subsets A and B.

In one embodiment, successive segmentation steps are repeated until a population subset is generated that cannot be further segmented.

In one embodiment, the rule-in cut-off probability as applied to a rule-in model refers to the cut-off threshold for the probability parameter selected from PPV (Positive Predictive Value), Specificity (1-False Positive Rate; 1-FPR) or Positive Likelihood Ratio (PLR), determined by a person skilled in the art and taking into account the prevalence of the health condition.

In one embodiment, the rule-out cut-off probability as applied to a rule-out model refers to the cut-off threshold for the probability parameter selected from NPV (Negative Predictive Value), Sensitivity (1-False Negative Rate; 1-FNR) or Negative Likelihood Ratio (PLR), determined by a person skilled in the art and taking into account the prevalence of the health condition.

In one embodiment, when one of the population subsets does not comprise a sufficient number of subjects, the previous segregation step is repeated using an alternative model configured to generate a population subset with a sufficient number of subjects.

The model generated by the method of the invention comprises at least two models, namely at least a rule-in model and at least one rule-out model. The models, generated according to the method of the invention, may be applied to a subject to determine the probability of the subject having a health condition or not (diagnosis), or to determine the probability of the subject developing a health condition or not in the future (prognosis). For example, if the models are a first rule-out model, and a second rule-in model, if the subject does not conform to the rule-out model but does conform to the rule-in model, then the subject is at high risk of having (or developing) a health condition, in line with the probability parameter selected. Likewise, if the models are a first rule-in model, and a second rule-out model, if the subject does not conform to the rule-in model but does conform to the rule-out model, then the subject is at low risk of having (or developing) a health condition in line with the probability parameter selected. In some aspects, depending on whether the subject conforms to the first model, there may be no need to determine whether the subject conforms to the second model.

In another aspect, the invention provides a method of detecting risk of a subject having or developing a health condition using the model M generated according to a method of the invention. The method typically comprises the steps of:
using the first model M1 to determine whether the subject has a probability to have/develop the health condition selected from one of: a probability lower than or equal to a first predefined rule-out cut-off probability; and a probability higher than or equal to a first predefined rule-in cut-off probability; and
optionally, using the second model M2 to determine whether the subject has a probability to have/develop the health condition selected from the other of: a probability lower than or equal to a second predefined rule-out cut-off probability; or a probability higher than or equal to a second predefined rule-in cut-off probability.

In one embodiment, when the first model M1 determines that the subject has a probability to have or to develop the health condition that is higher than a first predefined rule-out cut-off probability, the second model M2 is employed to determine whether the subject has probability to have or to develop the health condition that is higher than or equal to a second predefined rule-in probability (for example, a PPV rule-in cut-off); wherein when the second model M2 determines that the subject has a probability to have or to develop the health condition that is higher than or equal to a second predefined rule-in probability (for example a PPV rule-in cut-off), the subject is determined to have a high probability to have or to develop the health condition; [Out > In]

In one embodiment, when the first model M1 determines that the subject has a probability to have or to develop the health condition that is lower than a first predefined rule-in cut-off probability, the second model M2 is employed to determine whether the subject has probability to have or to develop the health condition that is lower than or equal to a second predefined rule-out probability (for example a NPV rule-out cut-off); wherein when the second model M2 determines that the subject has a probability to have or to develop the health condition that is lower than or equal to a second predefined rule-out probability (for example a NPV rule-out cut-off), the subject is determined to have a low probability to have or to develop the health condition. [In > Out]

In one embodiment, when the first model M1 determines that the subject has a probability to have or to develop the health condition that is lower than or equal to a first predefined rule-out cut-off probability (for example a rule-out NPV cut-off), the subject is determined to have a low probability to have or to develop the health condition;

In one embodiment, when the first model M1 determines that the subject has a probability to have or to develop the health condition that is higher than or equal a first predefined rule-in cut-off probability (for example rule-in PPV cut-off, the subject is determined to have a high probability to have or to develop the health condition;

In one embodiment, the method comprises the steps of:
using the first model M1 to determine whether the subject has a probability to have or to develop the health condition selected from one of: a probability lower than or equal to a predefined NPV rule-out cut-off; and a probability higher than or equal to a predefined rule-in PPV cut-off;
when the first model M1 determines that the subject has a probability to have or to develop the health condition that is higher than the first predefined NPV rule-out cut-off OR the first model M1 determines that the subject has a probability to have or to develop the health condition that is lower than the first predefined PPV rule-in cut-off,
using the second model M2 to determine whether the subject has a probability to have or to develop the health condition selected from the other of: a probability lower than or equal to the predefined rule-out NPV cut-off; or a probability higher than or equal to the predefined rule-in PPV cut-off, and
when the second model M2 determines that the subject has a probability to have or to develop the health condition that is higher than the first predefined NPV rule-out cut-off OR the second model M2 determines that the subject has a probability to have or to develop the health condition that is lower than the first predefined PPV rule-in cut-off,
using the third model M3 to determine whether the subject has a probability to have or to develop the health condition selected from one of: a probability lower than or equal to the predefined NPV rule-out cut-off; or a probability higher than or equal to the predefined PPV rule-in probability. [In/Out > In/Out > In/Out]

In one embodiment, the method comprises the steps of:
using the first model M1 to determine whether the subject has a probability to have or to develop the health condition higher than or equal to a predefined rule-in PPV cut-off;
wherein when the first model M1 determines that the subject has a probability to have or to develop the health condition that is lower than the predefined rule-in PPV cut-off, the second model is employed to determine whether the subject has probability to have or to develop the health condition that is lower than or equal to a predefined NPV rule-out cut-off;
wherein when the second model M2 determines that the subject has a probability to have or to develop the health condition that is higher than the predefined rule-out NPV cut-off, the third model M3 is employed to determine whether the subject has probability to have or to develop the health condition that is lower than or equal to the predefined NPV rule-out cut-off;
wherein when the third model determines that the subject has a probability to have or to develop the health condition that is higher than the third predefined NPV rule-out cut-off, the subject is determined to have a high probability to have/develop the health condition.

In one embodiment, the plurality of variables include at least two variables selected from a metabolite, a protein and a clinical risk factor.

In one embodiment, the plurality of variables include a plurality of metabolites, optionally in combination with at least one protein or clinical risk factor.

In one embodiment, the health condition is a pregnancy related disorder. In one embodiment, the health condition is selected from preeclampsia, and the variables include metabolites and/or proteins.

There is also provided a computer program comprising program instructions for causing a computer program to carry out a method of the invention which may be embodied on a record medium, carrier signal or read-only memory.

There is also provided a computer implemented system for generating a model M for detecting or predicting risk of a health condition in a subject. In one embodiment, the computer implemented system comprises:
a module or means for providing a population of test subjects and measurement data for a plurality (n) of variables for each of the test subjects selected from biometric, life-style and/or physiological characteristics;
a module or means for providing a first model M1 configured to predict the presence or risk of the health condition in the population of test subjects comprising 1 to n variables, wherein the model M1 is configured to segregate the population of test subjects into first generation population subsets A and B in which the subjects in the first generation population subset A have a probability selected from one of: a probability to have/develop the health condition lower than or equal to a predefined rule-out cut-off probability; or a probability to have/develop the health condition higher than or equal to a predefined rule-in cut-off probability;
a module or means for segmenting the population of subjects based on the first model M1 into first generation population subsets A and B;
optionally, a module or means for determining when the first generation population subset B comprises a sufficient number of subjects;
a module or means for providing a second model M2 configured to predict the presence or risk of the health condition in the first generation population subset B, the model comprising 1 to n variables, wherein the model M2 is configured to segregate the first generation population subset B into second generation population subsets A and B in which the subjects in the second generation population subset A have a probability selected from the other of: a probability to have/develop the health condition lower than or equal to a predefined rule-out cut-off probability; or a probability to have/develop the health condition higher than or equal to a predefined rule-in cut-off probability; and
a module or means for segmenting the first generation population subset B based on the second model M2 into second generation population subset A and B;
wherein the model M comprises the first model M1 and the second model M2.

The embodiments in the invention described comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g. CD ROM, or magnetic recording medium, e.g. a floppy disk or hard disk. The carrier may be an electrical or optical signal which may be transmitted via an electrical or an optical cable or by radio or other means.

Other aspects and preferred embodiments of the invention are defined and described in the other claims set out below.

### Brief Description of the Figures

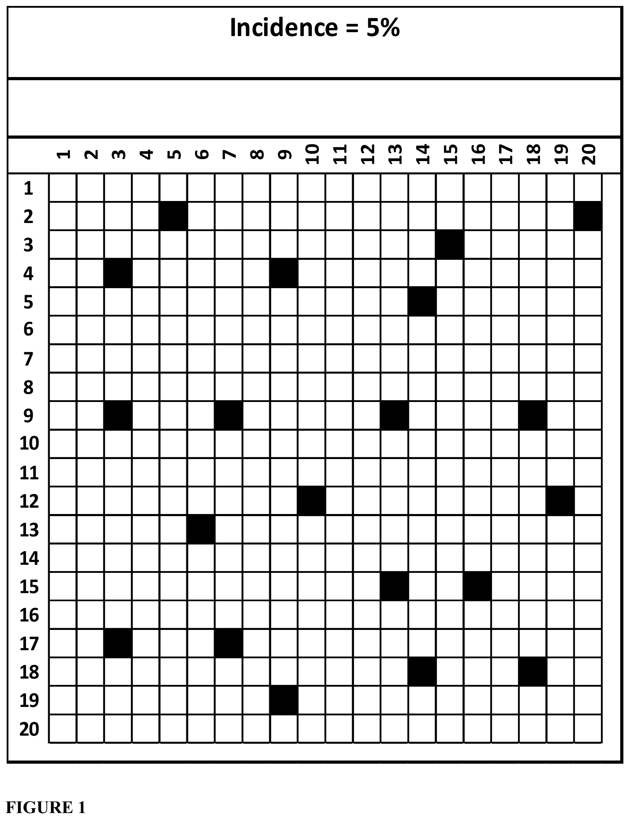

**Fig. 1** is a graphical representation of a hypothetical "base-case" population (pre-test) with a 5% outcome incidence (p= 0.05).

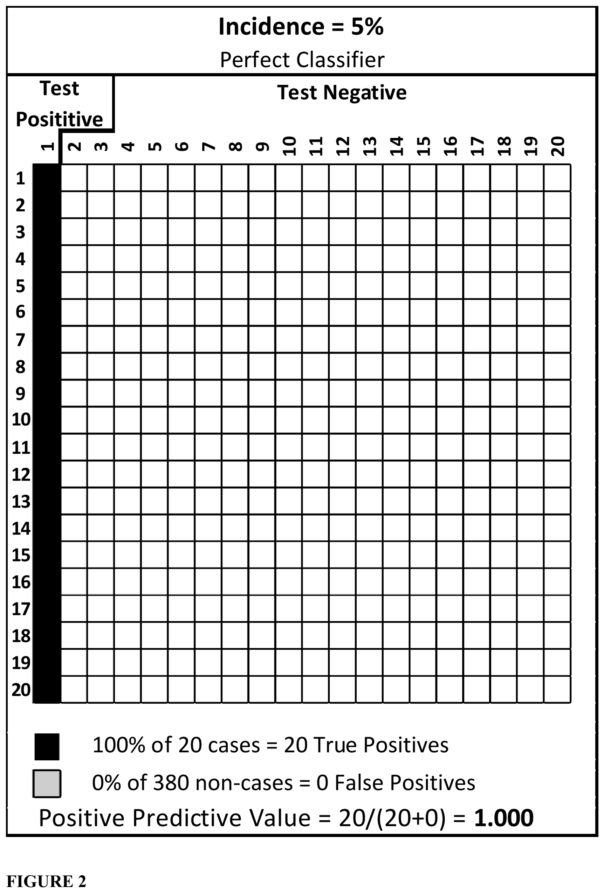

**Fig. 2** is a graphical representation of a hypothetical perfect outcome classifier for the hypothetical "base-case" population.

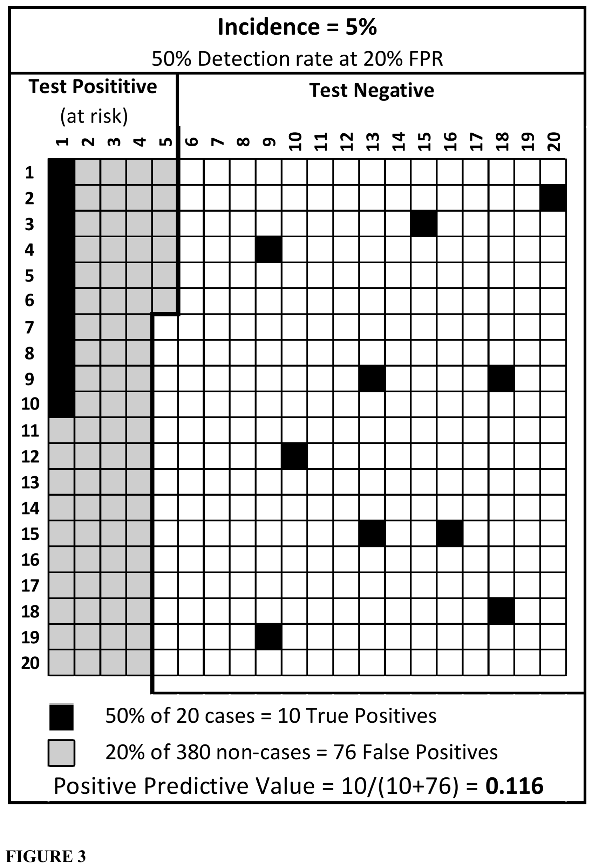

**Fig. 3** is a graphical representation of a rule-in classifier with 50% outcome detection rate at 20% FPR threshold for the hypothetical "base-case" population

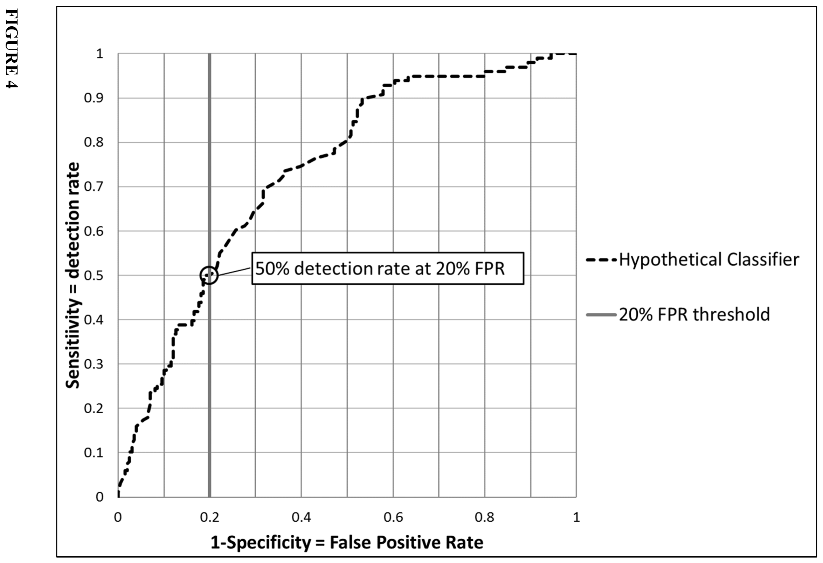

**Fig. 4** is a graphical representation of a hypothetical rule-in classifier with 50% outcome detection rate at 20% FPR for the hypothetical "base-case" population represented in the ROC space.

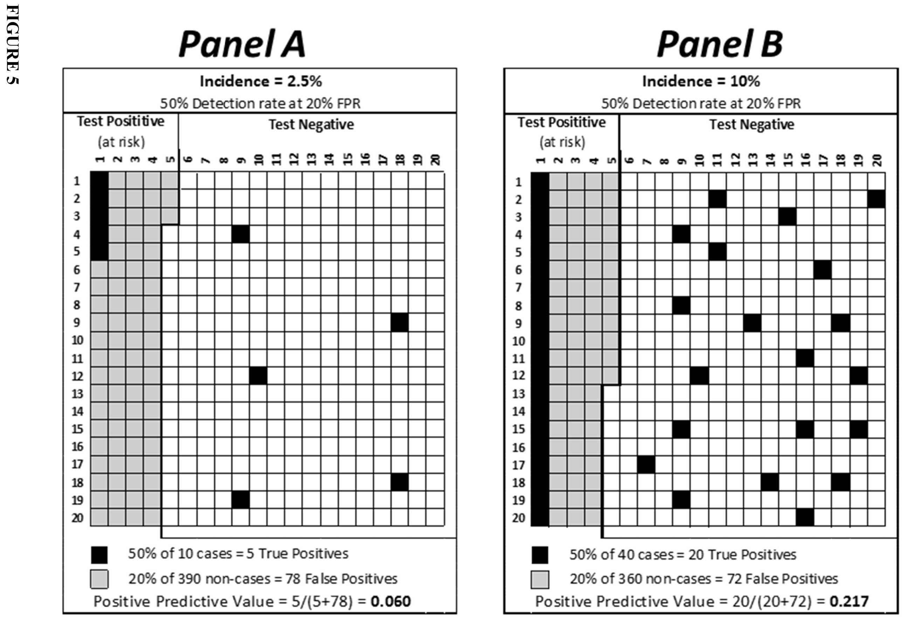

**Fig. 5** is a graphical representation of rule-in classifiers with 50% detection rate at 20% FPR: Panel A - outcome incidence p= 0.025. Panel B - outcome incidence p= 0.1

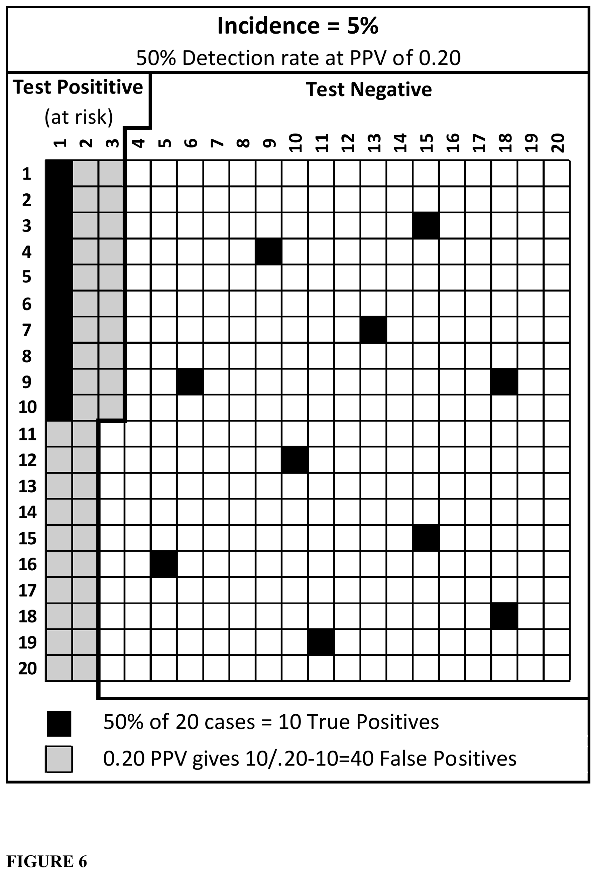

**Fig. 6** is a graphical representation of a rule-in classifier with 50% detection rate at PPV= 0.2 for the hypothetical "base-case" population.

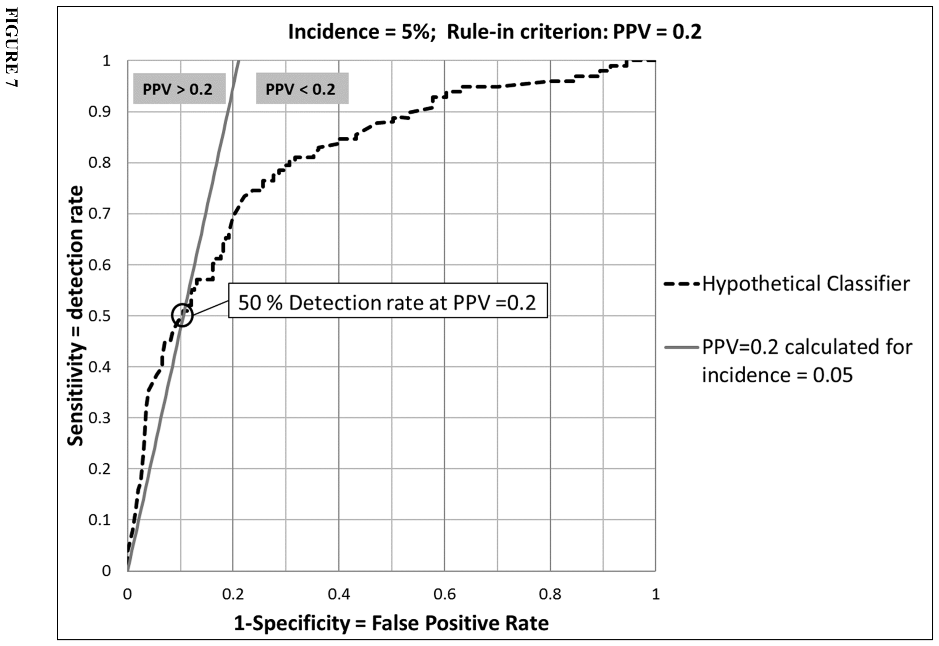

**Fig. 7** is an ROC curve of a hypothetical rule-in classifier delivering 50% detection rate at PPV= 0.2 for the hypothetical "base-case" population represented in the ROC space.

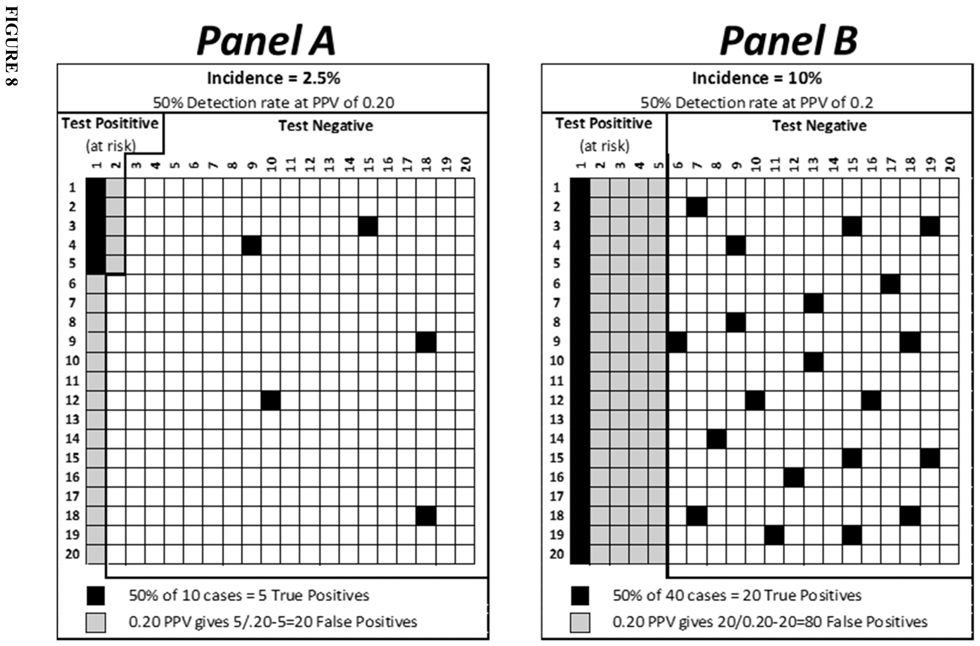

**Fig. 8** is a graphical representation of a rule-in classifier with 50% detection rate at PPV= 0.2 threshold. Panel A - outcome incidence p= 0.025. Panel B - outcome incidence p= 0.1.

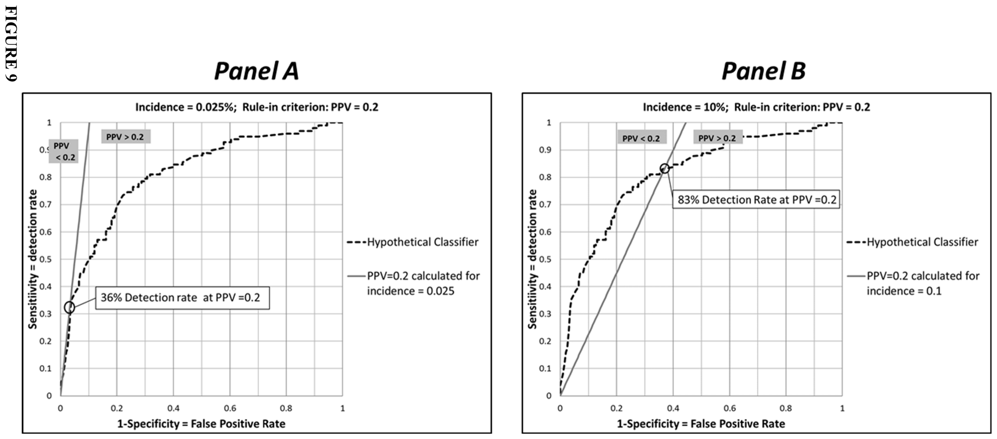

**Fig. 9****:** Hypothetical rule-in classifier (Fig.7) represented in the ROC space with a PPV= 0.2 threshold. Panel A: Outcome incidence p= 0.025; the classifier (barely) detects 36% of Future cases at the applied criterion. Panel B: Outcome incidence p= 0.1; the classifier detects 83% of Future Cases at the applied criterion.

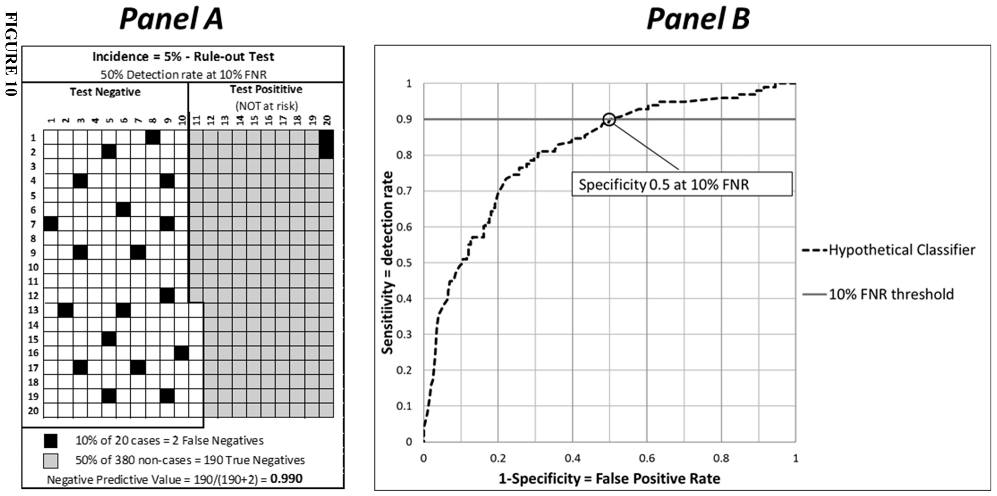

**Fig.10****:** Panel A: Rule-out classifier with Specificity = 0.5 (50 % detection Rate for future non-cases) at 10% FNR threshold for the hypothetical "base-case" population: Panel B Hypothetical rule-out classifier delivering Specificity = 0.5 (or 50% Detection Rate for future non-cases) at 10%NPR represented in the ROC space for the hypothetical "base-case" population

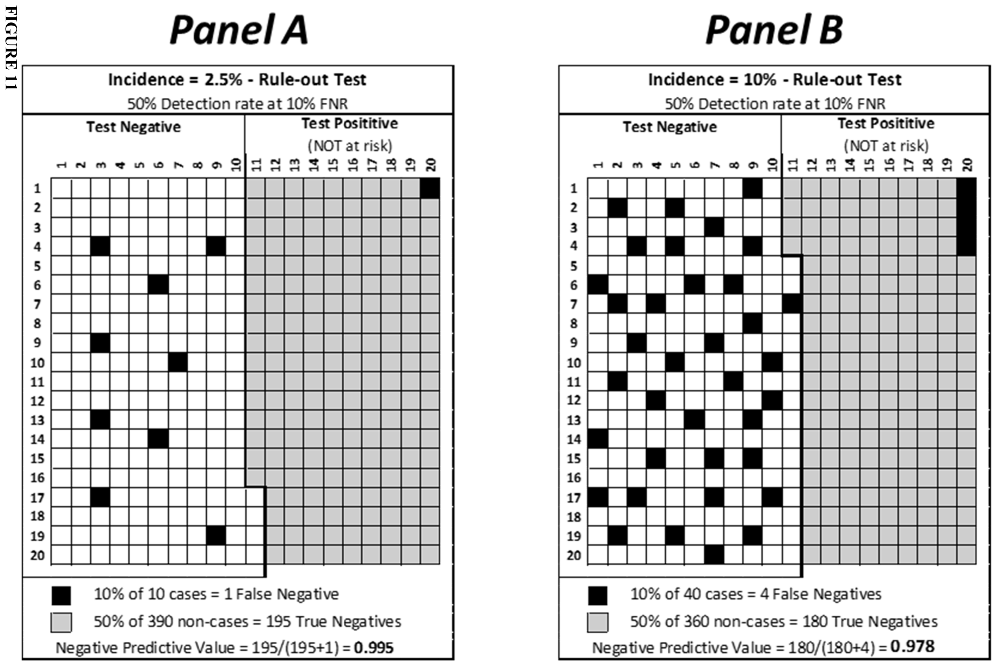

**Fig. 11****:** Rule-out classifiers with Specificity = 0.5 at 10% FNR threshold; Panel A: Outcome incidence p =0.025; Panel B: Outcome incidence p = 0.1.

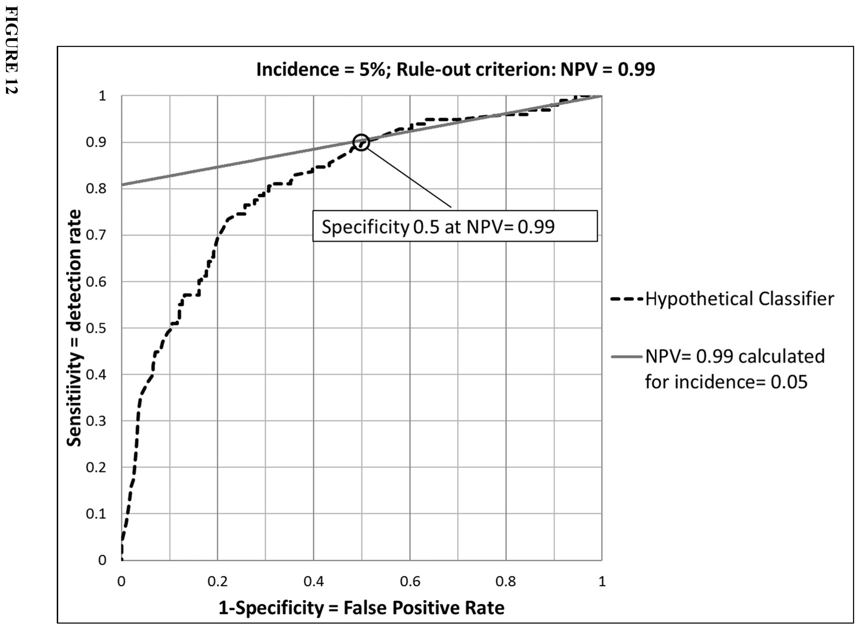

**Fig. 12****:** Hypothetical rule-out classifier delivering Specificity = 0.5 at NPV= 0.99 for the hypothetical "base-case" population represented in the ROC space.

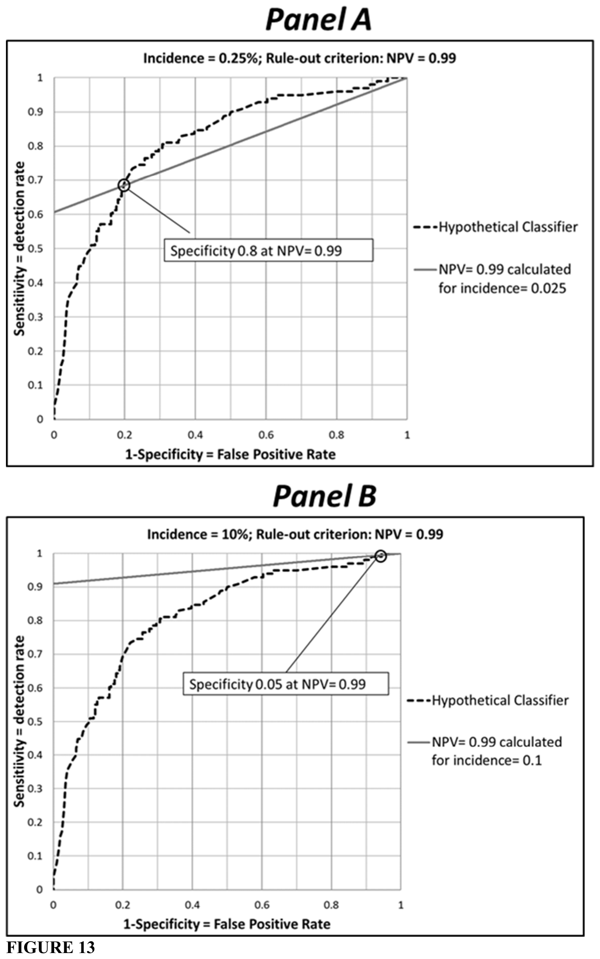

**Fig. 13****:** Hypothetical rule-out classifier represented in the ROC space with a NPV = 0.99 threshold (Fig. 12). Panel A: Outcome incidence p= 0.025; the detects 80% of Future non-cases (true negatives) at the applied criterion. Panel B: Outcome incidence p= 0.1; the classifier barely detects barely 5% of Future non-cases (true negatives) at the applied criterion.

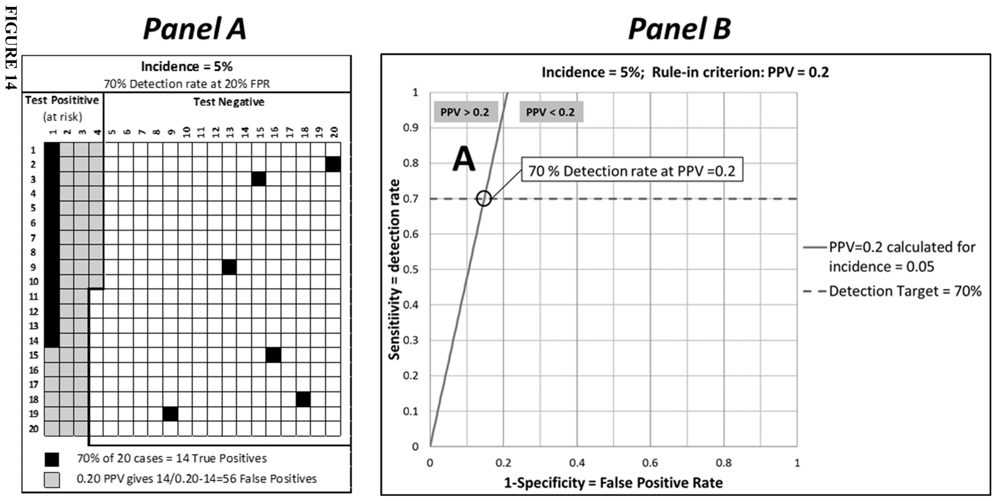

**Fig. 14****:** Illustration of the minimal requirements, i.e., 70% Detection rate for PPV = 0.2 for a clinically meaningful rule-in test (hypothetical) for an outcome incidence (or prevalence) of 5% (hypothetical "base-case" population). Panel A: Rule-in classifier delivering 70% Detection rate with PPV= 0.2 (or 4 False Positives / True positive). Panel B: Representation of the minimal requirements in the ROC space; intercept of PPV= 0.2 line and Sensitivity = 0.7 line. Any classifier with a ROC curve with points in Area "A" would meet the minimal criterion.

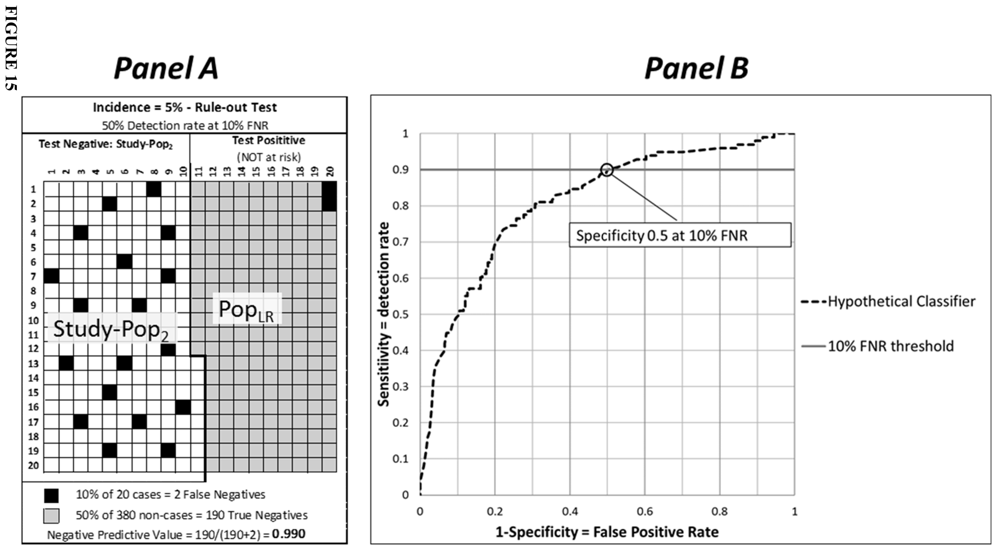

**Fig. 15****:** Establishing Rule-in classification by means of a sequential approach; Step 1. Application of a rule-out classification resulting in a (transient) Study-Pop2 enriched in Future-Cases. Panel A: Rule-out classifier with Specificity = 0.5 (50 % detection Rate for future non-cases) at 10% FNR threshold: Panel B Hypothetical rule-out classifier delivering Specificity = 0.5 (or 50% Detection Rate for future non-cases) at 10%NPR represented in the ROC space.

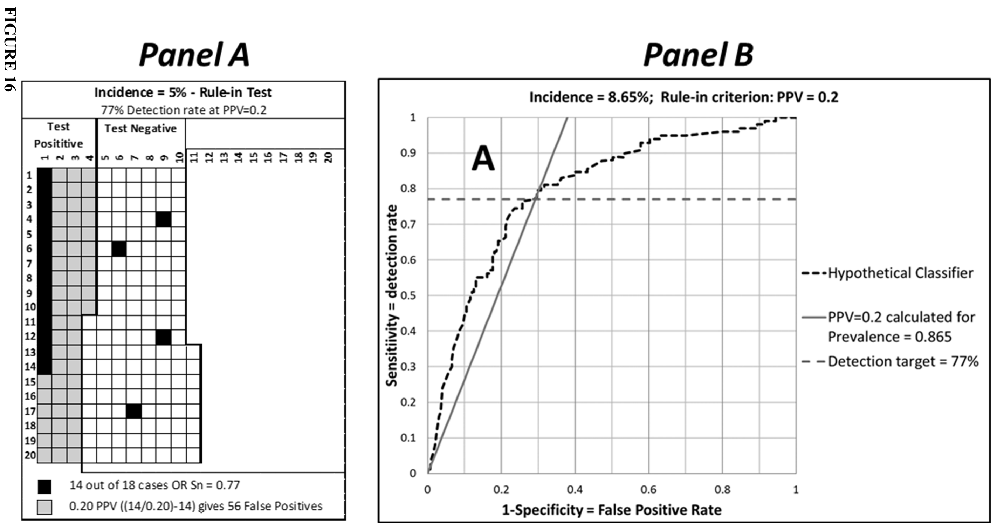

**Fig. 16****:** Establishing Rule-in classification by means of a sequential approach; Step 2. Application of a rule-in classification resulting in the final high-risk group (Test Positive). Panel A: Rule-in classifier with Sensitivity = 0.77 (77 % detection Rate for future cases) at PPV= 0.2 threshold: Panel B Hypothetical rule-in classifier delivering Sensitivity = 0.77 at PPV= 0.2 represented in the ROC space. Any classifier with a ROC curve with points in Area "A" would meet the minimal criterion.

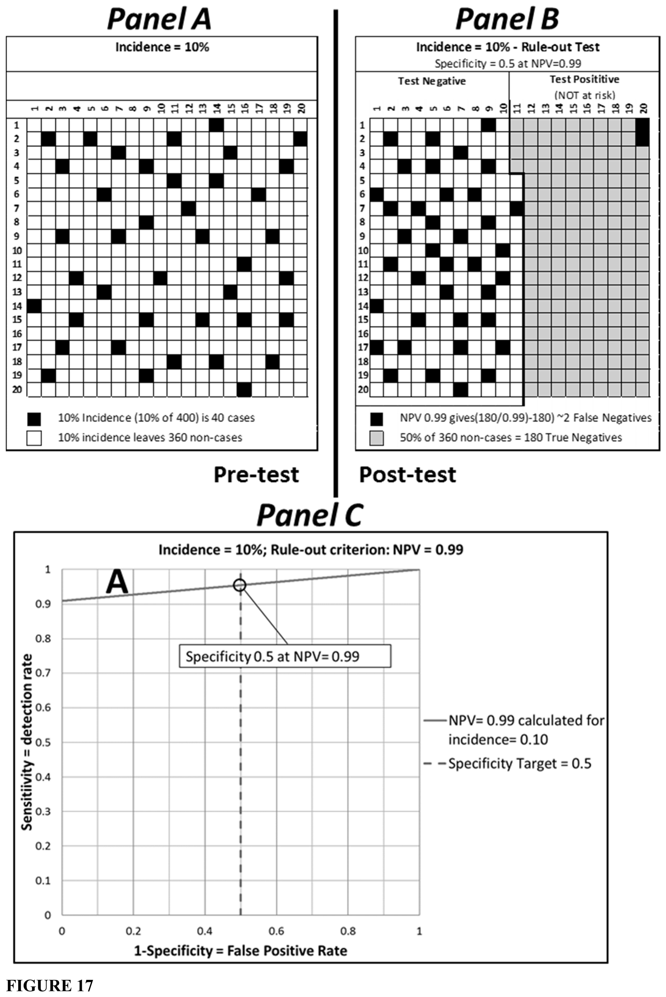

**Fig. 17****:** Illustration of the minimal requirements, i.e., Specificity=0.5 (i.e., 50% detection rate of future non-cases) for NPV = 0.99 for a clinically meaningful rule-out test (hypothetical) for an outcome incidence (or prevalence) of 10%. Panel A: Population "base-case 2" (pre-test). Panel B: Rule-out classifier delivering Specificity = 0.5 with NPV= 0.99 (or 1 False negative / 99 True Negatives). Panel C: Representation of the minimal requirements in the ROC space; intercept of NPV= 0.99 line and Specificity = 0.5% line. Any classifier with a ROC curve with points in Area "A" would meet the minimal criterion.

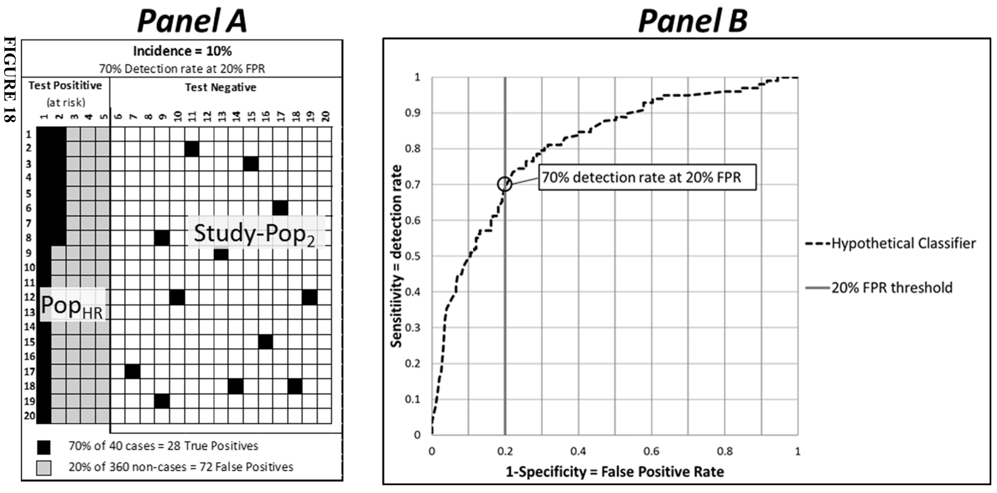

**Fig. 18****:** Establishing Rule-out classification by means of a sequential approach; Step 1. Application of a rule-in classification resulting in a (transient) Study-Pop2 enriched in Future non-Cases. Panel A: Rule-in classifier with Sensitivity = 0.7 (70 % detection Rate for future cases) at 20% FPR threshold: Panel B Hypothetical rule-in classifier delivering Sensitivity = 0.7 (or 70% Detection Rate for future non-cases) at 20% FPR represented in the ROC space.

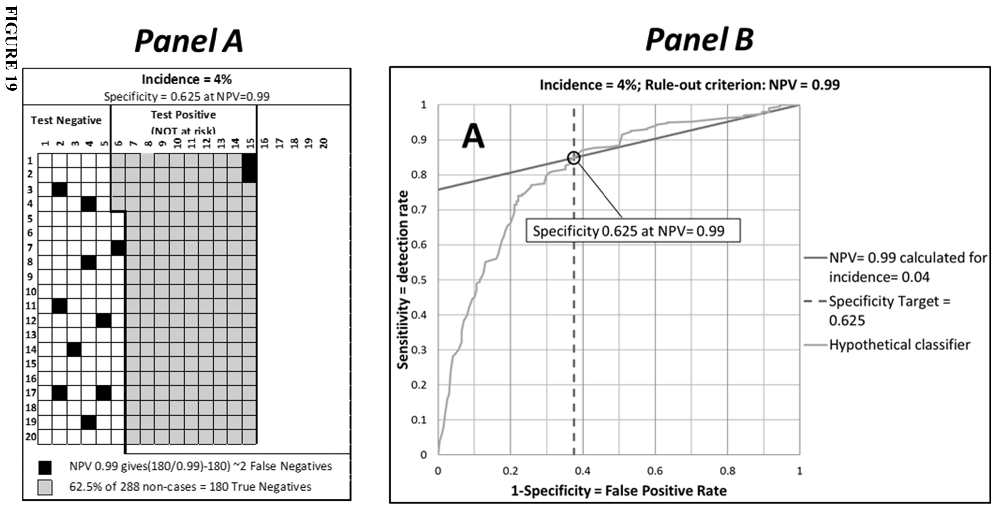

**Fig. 19****:** Establishing Rule-out classification by means of a sequential approach; Step 2. Application of a rule-out classification resulting in the final Low-risk group (Test Positive). Panel A: Rule-out classifier with Specificity = 0.625 (62.5 % detection Rate for future non-cases) at NPV= 0.99 threshold: Panel B Hypothetical rule-out classifier delivering Specificity = 0.625 at NPV= 0.99 represented in the ROC space. Any classifier with a ROC curve with points in Area "A" would meet the minimal criterion.

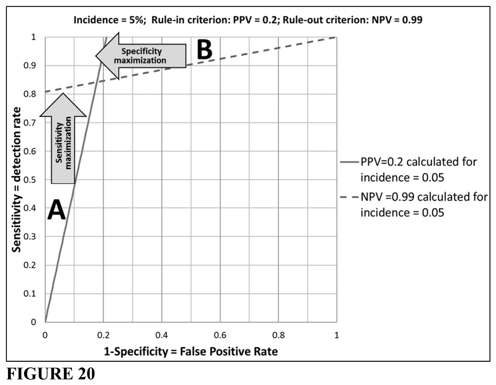

**Fig. 20****:** Illustration of the minimal requirements for a clinically meaningful prognostic test (hypothetical) for an outcome incidence (or prevalence) of 5% (the hypothetical "base-case" population). Representation of the requirements in the ROC space; For any classifier, risk scores which agree with a point in Area "A" would meet the minimal rule-in criterion, risk scores which agree with a point in Area "B" would meet the minimal criterion rule-out criterion. For a classifier to meet both the rule-in and rule-out criteria at the same time, it's associated ROC curve will have points in the intersect area (A ∩ B).

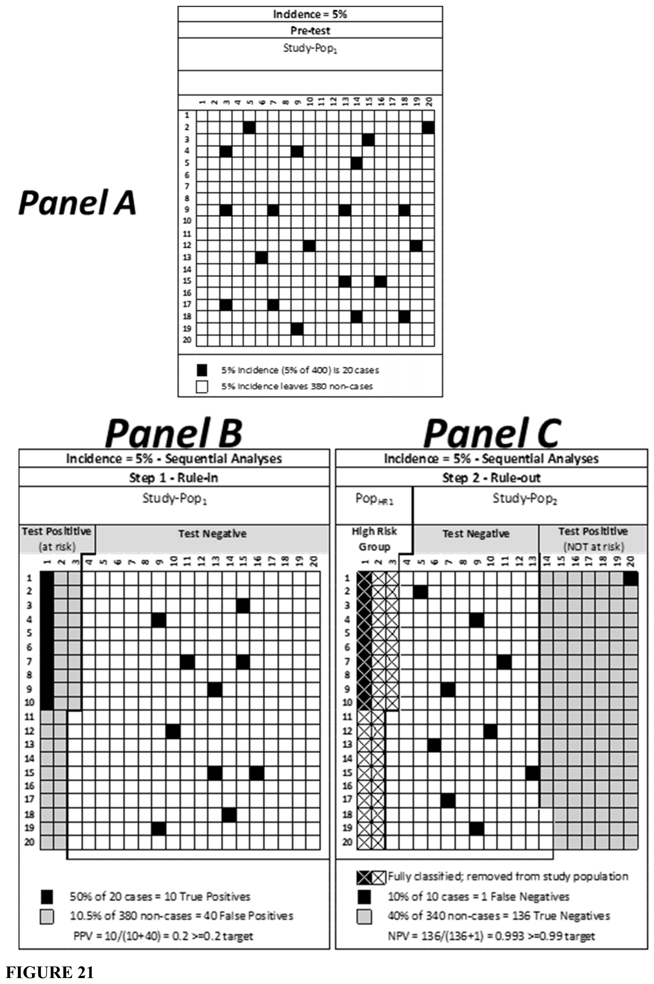

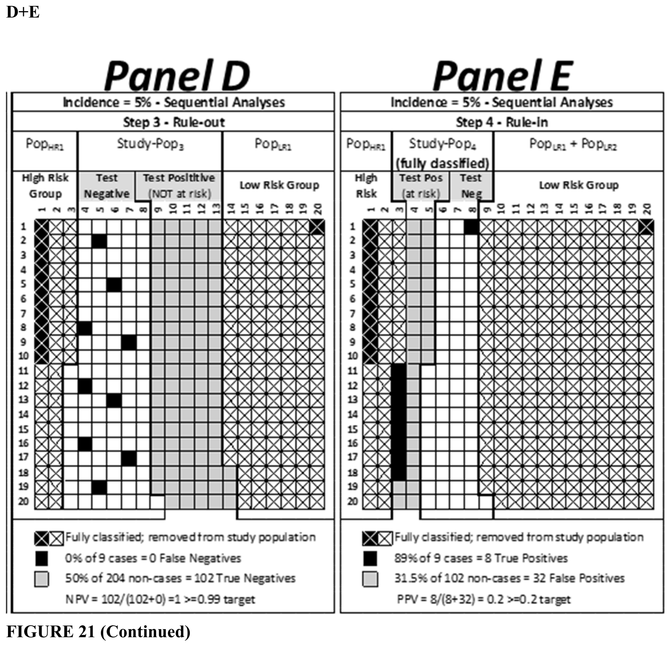

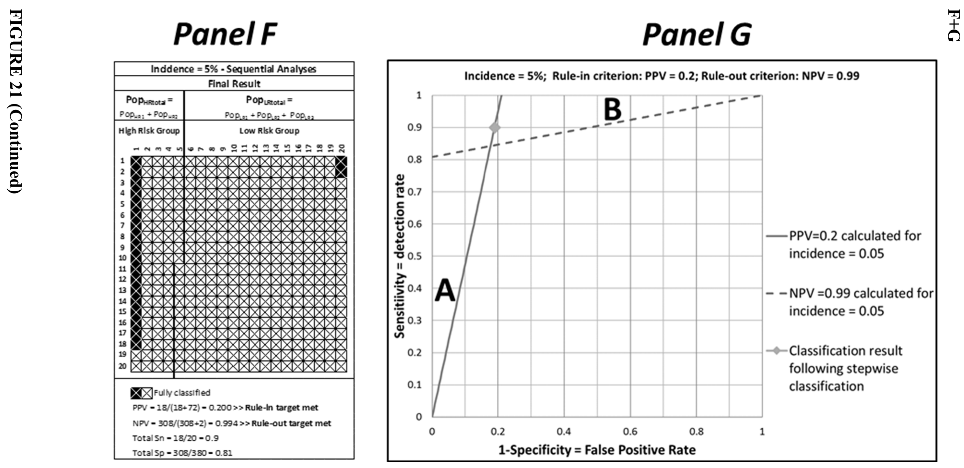

**Fig. 21****:** Establishing extraordinary prognostic classification (hypothetical) which complies with both a pre-set rule-in criterion PPV>= 0.2 and NPV>= 0.99, for an outcome incidence (or prevalence) of 5% (the hypothetical "base-case" population). Total classification follows the application of a step-wise classification approach augmented with a decision criterion [fully classified yes/no] at each step. Panel A Pre-test study-population (Study-Pop1). Panel B: 1st step: Rule-in classifier resulting in high-risk group PopHR1 (fully classified) and a novel Study-Pop2. Panel C: 2nd step: Rule-out classifier applied to Study-Pop2 resulting in low-risk group PopLR1 (fully classified) and a novel Study-Pop3. Panel D: 3rd step: Rule-out classifier applied to Study-Pop3 resulting in low-risk group PopLR2 (fully classified) and a novel Study-Pop4. Panel E 4th step: Rule-in classifier resulting in high-risk group PopHR2 (fully classified) and a residual population, as yet unclassified. Panel F: Total classification result presented in "grid format" representing the outcome if the residual population from step 4 were deemed to be low risk. Panel G: Representation of the total classification result presented in the ROC space.

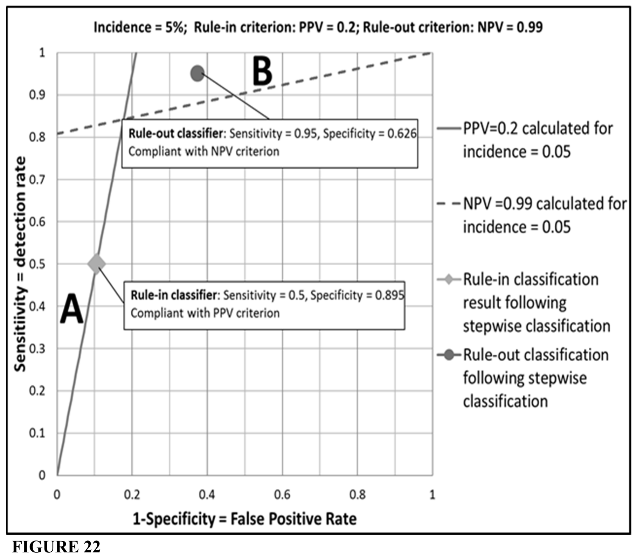

**Fig. 22****:** Illustration of the total prognostic classification (hypothetical) which complies with both a pre-set rule-in criterion PPV >= 0.2 and NPV >= 0.99, for an outcome incidence (or prevalence) of 5% (the hypothetical "base-case" population). Total classification follows the application of a step-wise classification approach augmented with a decision criterion [fully classified yes/no] at each step. Resulting Total classification produces 2 thresholds, i.e., specific [Sensitivity - Specificity] pairs in the ROC space, corresponding a rule-in classification threshold (diamond in area "A"; high-risk group) and a rule-out classification threshold (dot in area "B"; low-risk group). Individuals which are neither classified in the high-risk group nor the low-risk group, are considered un-classified.

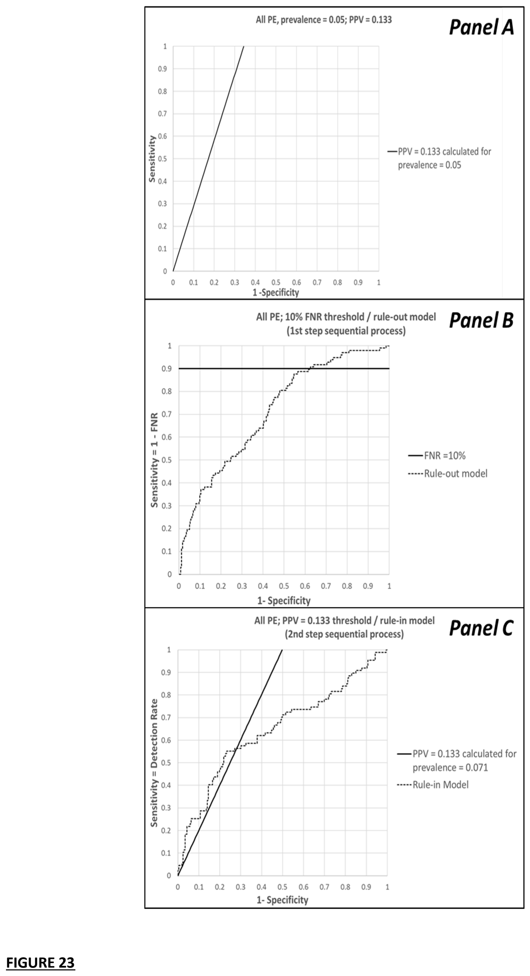

**Fig. 23** Example 4A; Sequential application of a Rule-out classifier followed by a rule-in classifier to achieve prognostic performance for predicting "All PE" in function of a preset PPV cut-off. Panel A: the PPV = 0.133 cut-off for a pretest prevalence of future PE of p = 0.05 plotted in the ROC space. Panel B: Step 1; ROC curve corresponding a selected rule-out classifier (bp + s-ENG + 1-HD) with the model M1: *0.292700587596098 log10[s-ENG (MoM)] + 0.0103090246336299 [2nd_sbp] - 0.335817558146904 log10 [1-HD].;* classification of the full test-population (P1) is done at a 10% FNR threshold. This corresponds to a rule-out threshold score of the model M1 being less than (<) 0.66643052785405. This results in 38.3% of the true negatives (future non-cases) being classified at low risk, together with 10% of the future PE cases (false Negatives); these individuals removed from the test population. Panel C: Step2; ROC curve corresponding an exemplary rule-in classifier (bp + PIGF + DC) with the model M2: *-0.195394942337404 log10 [PIGF (MoM)] + 0.00590836118884227 [map_1^{st}] +* 0.143670336856774 *log10 [DC]* and Model M2 rule-in threshold score of larger than (>) 0.581930006682247, as applied within the remainder of the test population (P2), a rule-in classifier for predicting "All PE" in function of the preset PPV cut-off. Following the step 1 rule-out classification, the prevalence of future PE is enriched to p=0.071, resulting in a more relaxed PPV = 0.133 criterion.

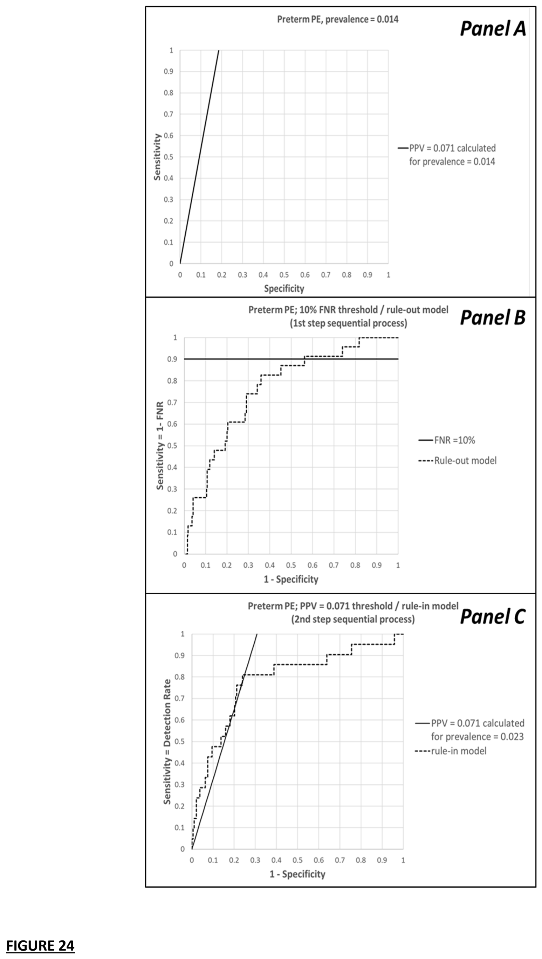

Fig. 24 Example 4B; Sequential application of a Rule-out classifier followed by a rule-in classifier to achieve prognostic performance for predicting "Preterm PE" in function of a preset PPV cut-off. Panel A: the PPV = 0.071 cut-off for a pretest prevalence of future preterm PE of p = 0.014, plotted in the ROC space. Panel B: Step 1; ROC curve corresponding a selected rule-out classifier (s-ENG + DLG) with the model M1: *0.22139876465602 log10 [s-ENG] + 0.0162829949120052 log10 [DLG];* classification of the full test-population (P1) is done at a 10% FNR threshold. This corresponds to
   a rule-out threshold score of the model M1 being less than (<) 0.710765699780132. This results in 43.7% of the true negatives (future non-cases) being classified at low risk, together with 10% of the future preterm PE cases (false Negatives); these individuals removed from the test population. Panel C: Step2; ROC curve corresponding an exemplary rule-in classifier (PIGF + s-ENG + DLG + 2-HBA) with the model M2; *0.20043337818718 log10 [s-ENG (MoM)] - 0.212088369466248 log10 [PIGF_(MoM)] + 0.112046727485729 log10[2-HBA] + 0.227265325783904 log10 [DLG]* and Model M2 rule-in threshold score of larger than (>) 0.668333882056883 as applied within the remainder of the test population (P2), a rule-in classifier for predicting "Preterm PE" in function of the preset PPV cut-off. Following the step 1 rule-out classification, the prevalence of future Preterm PE is enriched to p=0.023, resulting in a more relaxed PPV = 0.071 criterion.

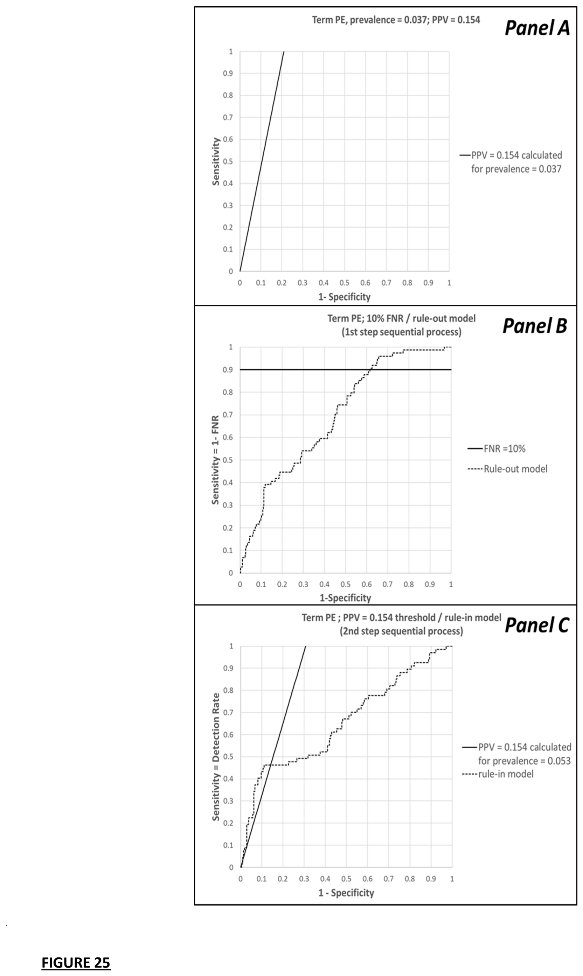

**Fig. 25** Example 4C; Sequential application of a Rule-out classifier followed by a rule-in classifier to achieve prognostic performance for predicting "Term PE" in function of a preset PPV cut-off. Panel A: the PPV = 0.154 cut-off for a pretest prevalence of future preterm PE of p = 0.037, plotted in the ROC space. Panel B: Step 1; ROC curve corresponding a selected rule-out classifier (bp + 1-HD) with the model M1: *0.0115467461789923 [map_1^{st}] - 0.324977743714534 log10 [1-HD];* classification of the full test-population (P1) is done at a 10% FNR threshold. This corresponds to
   a rule-out threshold score of the model M1 being less than (<) 0.680257687736226. This results in 38.2% of the true negatives (future non-cases) being classified at low risk, together with 10% of the future Term PE cases (false Negatives); these individuals removed from the test population. Panel C: Step2; ROC curve corresponding an exemplary rule-in classifier (bp + 1-HD + NGM) with the model M2; *0.0093936118486756 [2nd_sbp] + 0.560572544580583 log10 [NGM] - 0.302082838614281 log10 [1-HD]* and Model M2 rule-in threshold score of larger than (>) *0.581599411310977.* as applied within the remainder of the test population (P2), a rule-in classifier for predicting "Term PE" in function of the preset PPV cut-off. Following the step 1 rule-out classification, the prevalence of future Preterm PE is enriched to p=0.053, resulting in a more relaxed PPV = 0.154 criterion.
**Fig. 26** Example 4D1 Determination of the minimal prognostic criteria for predicting preterm PE. For any classifier, risk scores which agree with a point in Area "A" would meet the minimal criterion rule-in criterion, risk scores which agree with a point in Area "B" would meet the minimal criterion rule-out criterion. For a classifier to meet both the rule-in and rule-out criteria at the same time, it's associated ROC curve (or paired Sensitivity-specificity value(s)) will have point in the intersect area (A ∩ B).
**Fig. 27** Example 4D2 PIGF levels at time of sampling vs time of delivery. Star symbol: Preterm PE. "bar" symbol: Term PE. "circle" symbol: no PE. Area "A" contains future preterm PE cases which will be missed by application of a stand-anole PIGF threshold as indicated. PIGF threshold classifies population in "high risk" (below threshold) wherefore PPV >= 0.071. Above threshold will be considered for further classification (cf. text).
**Fig. 28** Example 4D3 Scatter plot displaying blood values at time of sampling of the variables PIGF and Dilinoleoyl-glycerol for the study subjects. Study samples labelled according to (future) pregnancy outcome; i.e., no PE or "preterm" PE. Area "A" indicates a large zone in the scatter plot without (future) preterm-PE.
**Fig. 29** Example 4D4: Segmentation of the Study-Pop1 using a PIGF level as a Rule-in classifier, so that the resulting "ruled-in" population is compliant with the pre-set PPV criterion.
**Fig. 30** Example 4D5 "Total Classification" as achieved by applying a 1 step PIGF based (rule-in) classification
**Fig. 31** Example 4D6 Segmentation of the Study-Pop2 using a DLG level as a Rule-out classifier, so that the resulting "ruled-out" population is compliant with the pre-set PPV criterion.
**Fig. 32** Example 4D7 "Total Classification" as achieved by applying a 2 step classification involving PIGF (rule-in) and DLG (rule-out), whereby the rule-in and the rule-out classifier are considered separately. The negative classification (not-rule-in, not ruled-out) is also plotted.
**Fig. 33** Example 4D8 Further Segmentation of the Study-Pop3 using a L-ERG level as a Rule-out classifier.
**Fig. 34** Example 4D9 "Total Classification" as achieved by applying a 3 step classification involving PIGF (rule-in), DLG (rule-out), and L-ERG (rule-out), whereby the rule-in and the rule-out classifiers are considered separately. The negative classification (not-rule-in, not ruled-out x 2) is also plotted.
**Fig. 35** Example 4D10 Further Segmentation of the Study-Pop4 using a s-ENG as a Rule-out classifier, creating a 3rd ruled-out population (Pop-LR3) as well as a Residual population. This population is considered a 2nd High-Risk population (Pop-HR2)
**Fig. 36** Example 4D11: Total Classification" as achieved by applying a 3 step classification involving PIGF (rule-in), DLG (rule-out), L-ERG (rule-out), and s-ENG (rule-out). A The rule-in and the rule-out classifiers are considered separately. The negative classification (not-rule-in, not ruled-out x 3) is also plotted. B. The total classifier is given as a single classifier classifying any pregnancy population or pregnant woman into a high risk group for developing preterm PE (with a PPV >= 0.071) or into a a low-risk group for developing preterm PE (with a NPV >= 0.9975)

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "outcome" refers to the result of an event where two or more results are possible. For example, in medical prognostics, the outcome may be the subject developing or not developing a specific health condition. The methods of the invention involve generating models using a population of subjects that may be applied on a test subject to predict the probability of the subject developing the health condition. Another example is in weather forecasting where the outcome may be a defined weather event, where the model is generated using a population of weather scenarios, and the model may be applied to a specific weather scenario to predict the probability of the defined weather event occurring. The methods of the invention may be used to predict other types of outcome. The term "sample" as used herein refers to the make-up of the test population used to generate the models. In the area of medical diagnostic or prognostics, the samples will generally be subjects. However, for other applications of the methods of the invention, the samples may be events (for example weather events), observations, scenarios, phenomena, or products.

As used herein, the term "health condition" or "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies. Examples of health conditions include inflammatory disease, metabolic disease, cardiovascular disease, autoimmune disease, neurological disease, degenerative disease, hepatic disease, and pulmonary disease. In one embodiment, the health condition is a syndromic disorder. In one embodiment, the health condition is a disorder of pregnancy, for example a hypertensive disorder of pregnancy or a metabolic disease associated with pregnancy. In one embodiment, the hypertensive disorder of pregnancy is preeclampsia, or a sub-type of preeclampsia selected from, f.i., but not limited to pre-term preeclampsia, term preeclampsia, early onset preeclampsia and HELLP syndrome. As used herein, the term "PE" refers to preeclampsia.

As used herein, the term "hypertensive disorder of pregnancy" refers to a complication of pregnancy characterised by hypertension and includes chronic hypertension (including mild and severe), gestational hypertension, preeclampsia and sub-types of preeclampsia.

The term "preeclampsia" includes pre-term preeclampsia, term preeclampsia, and early onset preeclamspia. Preeclampsia is defined as elevated blood pressure after 20 weeks of gestation (≥ 140 mm Hg systolic or ≥ 90 mm Hg diastolic) plus proteinuria (> 0.3 g/24 hours). The term "pre-term preeclampsia" refers to the occurrence of preeclampsia which results to the delivery of the infant before 37 weeks of gestation.

As used herein, the term "treatment" or "treating" refers to an intervention (e.g. the administration of an agent to a subject, or modification of diet or exercise, etc) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

As used herein, the term "health condition" refers to a disease or condition of mammals, for example a hypertensive disorder, cardiovascular disease, a proliferative disorder, an inflammatory disease, an autoimmune disease, a metabolic disorder, a neurological disease or a hepatic disorder. In one embodiment, the health condition is a syndromic disorder. In one embodiment, the health condition is a disorder of pregnancy, for example a hypertensive disorder of pregnancy, or a metabolic disorder of pregnancy. In one embodiment, the hypertensive disorder of pregnancy is preeclampsia, or a sub-type of preeclampsia selected from pre-term preeclampsia, term preeclampsia, early onset preeclampsia and HELLP syndrome, preeclampsia leading to adverse maternal outcomes. As used herein, the term "PE" refers to preeclampsia.

As used herein, the term "presence of a health condition" refers to risk of the subject having the health condition (i.e. diagnosis). When the methods of the invention are directed to generating diagnostic models, the test population of subjects will generally include subjects that are positive for the health condition and subjects that are negative for the health condition when measurement data is obtained from the subjects.

As used herein, the formula notation [variable] typically relates to the (relative) concentration in blood of this variable as determined with the assay as exemplified in this specification.

As used herein, the formula notation log10[variable] relates to the logarithm to the base 10 of the (typically relative) concentration in blood of this variable, whereby the variable is determined with the assay as exemplified in this specification.

As used herein, the formulation [variable (MoM)] relates to multiple-of-median (MoM) normalized concentration of the variable; When the variable shows a significant dependency (Mann-Whitney U test, Spearman correlation, Benjamini, Hochberg and Yekutieli, p<0.01); the methods applied in this specification allow for considered MoM normalisation of the variable. The variable is determined with the assay as exemplified in this specification.

As used herein, the term "predicting the risk of a health condition" should be understood to mean predicting increased risk or decreased risk of the health condition. In the case of detecting increased risk, the post-test probability is generally higher than the pre-test probability, for example 1.5, 2, 2.5, 3, 3.5, 4, 4.5 5, 5.5, 6, 6.5 7, 7.5 8, 8.5 9, 9.5 or 10 times the pre-test probability. in one embodiment, the method of the invention is configured to detect 40-60% of cases of the health condition (i.e. 40%-50% or 50-60%) with a false positive rate (FPR) of 5-25%, and preferably about 10-20% FPR. In the case of detecting decreased risk, the post-test probability is generally lower than the pre-test probability, for example 1.5, 2, 2.5, 3, 3.5, 4, 4.5 5, 5.5, 6, 6.5 7, 7.5 8, 8.5 9, 9.5 or 10 times lower than the pre-test probability. in one embodiment, the method of the invention is configured to detect 40-60% of non-cases of the health condition (i.e. 40%-50% or 50-60%) with a false negative rate (FNR) of 5-25%, and preferably about 10-20% FNR.

As used herein, the term "measurement data" as applied to a subject or population of test subjects refers to a plurality of variables selected from biometric, life-style and physiological characteristics. Examples of biometric characteristics include age, ethnicity, blood pressure (diastolic, systolic), body mass index, weight, waist measurement, family history of preeclampsia (mother or sister), nulliparous or multiparous, and heart rate. Examples of life-style characteristics include smoking status, alcohol consumption status. Examples of physiological characteristics include the presence/absence of or levels of biological molecules, for example proteins, nucleic acids, sugars, antibodies and metabolites. Thus, for example, the measurement data for a pregnant woman may include the levels of a number of metabolites and proteins, blood pressure, weight, BMI, smoking status and number of pregnancies. The measurement data may be obtained at one or more time points. For example, when the health condition is preeclampsia, measurement data may be obtained at an early stage of pregnancy (i.e. 10-20 weeks) and at time of delivery.

As used herein, the term "1 to n variables" refers to the variables employed in the models. For example, the first model may employ two variables (a protein and biometric variable) and the second model may employ a single variable (a metabolic variable). Alternatively, the first model may employ one variable (a protein) and the second model may employ one variable (a metabolic variable). For example, in a method that employs three or more segregation steps, the first model may employ one variable (a protein) the second model may employ one variable (a first metabolic variable), and the third model may employ two variables (a second protein and a second metabolite variable), and subsequent models may involve other combinations of one or more variables.

As used herein, the term " model M" refers to a model generated by the methods of the invention that may be employed with a test subject to determine the risk of the subject having, or subsequently developing, the health condition. The model M comprises at least two models, a first model M1 which is one of a rule-in or rule-out model and a second model M2 which is the other of a rule-in or rule-out model. As described above, the first or second (and in some cases, subsequent) models may each independently employ one or more variables. The two (or more) models will normally be applied to a subject in sequence, where depending on the outcome of applying the first model to the subject, the second model may or may not be applied to the patient. Alternatively, two models may be applied to the subject at the same time, or may be combined into a single model or algorithm.

An example of a theoretical model M where each step requires just 1 variable, is exemplified in Table 1:

**Table 1 - Model M**

| | | |
|---|---|---|
| **Model M1 (Rule-out):** | if the subject has a level of metabolite X1 higher than or equal to value Y1, then apply Model M2 | if the subject has a level of metabolite X1 lower than value Y1, then subject may be classified as **low risk** of having or developing a condition. |
| **Model M2 (Rule-in):** | if the subject has a level of protein X2 lower than or equal to value Y2, then apply Model M3 | if the subject has a level of protein X2 higher than value Y2, then subject may be classified as **high risk** of having or developing a condition. |
| **Model M2 (Rule-in):** | if the subject has a level of metabolite X2 higher than or equal to value Y3, then the subject may be classified as **high risk** of having or developing a condition. | if the subject has a level of metabolite X2 lower than or equal to value Y3, then the subject's risk of having or developing a condition, may be calculated based on the performance characteristics of the test. |

The models employed in the segregation steps (M1, M2, etc) are generally configured to segregate the population (or sub-population) to generate a population subset that is enriched in subjects that have or subsequently develop the health condition, i.e. a population subset exhibiting an increased prevalence of the (future) health condition (Rule-in model), or that is enriched in subjects that do not have or do not subsequently develop the health condition, i.e. a population subset exhibiting an decreased prevalence of the health condition (Rule-out model). The models are configured to segregate the population (or sub-population) according to a pre-set threshold or cut-off probability, i.e. rule-out cut-off probability or a rule-out cut-off probability. Examples of probability parameters that may be employed include Predictive Values (i.e. NPV or PPV), Sensitivity/Specificity (False Negative Rate or False Positive Rate), or Likelihood Ratio cut-off (i.e. Negative Likelihood Ratio Cut-off or Positive Likelihood Ratio Cut-off). Thus, for example, the first model may be configured to generate a population subset containing 50% of all positive cases at a false positive rate of 20% Generally, the final segregation step of the methods of the invention employs a model defined by pre-set predictive value, for example a PPV or a NPV. The preceding segregation steps may employ different probability parameters, for example false negative rate and false positive rate.

As used herein, the term "rule-out cut-off probability" as applied to a rule-out model refers to the cut-off threshold for the score calculated by the model, which ensures compliance with the probability parameter employed. The probability parameter may be selected from NPV (Negative Predictive Value), Sensitivity (1-False Negative Rate) or Negative Likelihood Ratio (NLR). The parameters employed in the models will depend on the circumstances of the case, for example the type and prevalence of the health condition, and maybe determined by a person skilled in the art.

Typically, the NPV value (or equivalent) is selected to provide a post-test probability that is at least 1.5, 2, 3, 4 or 5 times less than the pre-test probability for (future) disease. In one embodiment, the NPV value (or equivalent) is selected to provide a post-test probability that is at least 5-10 times less the pre-test probability for (future) disease. In one embodiment, the NPV value (or equivalent) is selected to provide a post-test probability that is at least 10 times less the pre-test probability for (future) disease. Examples of rule-out cut-off probabilities are provided and further explained in Figs 10 and 11 and the accompanying text below.

As used herein, the term "rule-in cut-off probability" as applied to a rule-in model refers to the cut-off threshold for the score calculated by the model, which ensures compliance with the probability parameter employed. The probability parameter may be selected from PPV (Positive Predictive Value), Specificity (1-False Positive Rate) or Positive Likelihood Ratio (PLR). The parameters employed in the models will depend on the circumstances of the case, for example the type and prevalence of the health condition, and may be determined by a person skilled in the art.

Typically, the PPV value (or equivalent) is selected to provide a post-test probability that is at least 1.5, 2, 3, 4 or 5 times the pre-test probability for (future) disease. In one embodiment, the PPV value (or equivalent) is selected to provide a post-test probability that is at least 5-10 times the pre-test probability for (future) disease. In one embodiment, the PPV value (or equivalent) is selected to provide a post-test probability that is at least 10 times the pre-test probability for (future) disease. Examples of rule-in cut-off probabilities are provided and further explained in Figs 1 to 9 and the accompanying text below.

As used herein, the term "NPV" or "Negative Predictive Value" refers to the probability of a subject not having or developing a condition if they have been categorised by the test as not having or being at low risk of developing that condition. For example, if one person out of every 100 identified as being at low risk of a condition, is subsequently found to have or develops the condition, then the test correctly predicted the outcome for the other 99, giving a NPV of 0.99. NPV is defined as True Negatives / (True Negatives + False Negatives). NPV can only be employed when the prevalence (or incidence) of the (predisposition for the) condition is known (or can be estimated) and factored in the establishment of the NPV criterion.

As used herein, the term "PPV" or "Positive Predictive Value" refers to the probability of a subject having or developing a condition if they have been categorised by the test as having or being at high risk of developing that condition. For example, if ten people out of every 100 identified as being at high risk of a condition, are subsequently found to not have or develop the condition, then the test correctly predicted the outcome for the other 90, giving a PPV of 0.90. PPV is defined as True Positives / (True Positives + False Positives). PPV can only be employed when the prevalence (or incidence) of the (predisposition for the) condition is known (or can be estimated) and factored in the establishment of the PPV criterion.

As used herein, the term *"Sensitivity"* (Sn, Sens) refers to the probability that a test result will be positive when *(the predisposition for)* the condition is present (true positive rate). Sensitivity is independent from the prevalence (or incidence) of the (predisposition for the) condition

As used herein, the term *"Specificity"* (Sp, Spec) probability that a test result will be negative when *(the predisposition for)* the condition is not present (true negative rate). Specificity is independent from the prevalence (or incidence) of the (predisposition for the) condition

As used herein, the term "False Negative Rate" or "FNR" refers to the portion of subject who have or will develop a condition, who are incorrectly categorise as no having or being at risk of developing the condition; it is also referred to as (1-Sensitivity). For example, if 10 people out of every 100 who have or go on to develop a condition, are incorrectly categorised as not having or being at risk of developing the condition, then the test has incorrectly categorised these 10, giving a FNR of 0.10. FNR is defined as False Negatives / (False Negatives + True Positives). FNR is independent from the prevalence (or incidence) of the (predisposition for the) condition; a FNR criterion does not require the prevalence (or incidence) of the (predisposition for the) condition to be known.

As used herein, the term "False Positive Rate" or "FPR" refers to the portion of subject who do not have and will not develop a condition, who are incorrectly categorise as having or being at risk of developing the condition; it is also referred to as (1-Specificity). For example, if 10 people out of every 100 who do not have or go on to develop a condition, are incorrectly categorised as having or being at risk of developing the condition, then the test has incorrectly categorised these 10, giving a FPR of 0.10. FPR is defined as False Positives / (False Positives + True Negatives). FPR is independent from the prevalence (or incidence) of the (predisposition for the) condition; a FPR criterion does not require the prevalence (or incidence) of the (predisposition for the) condition to be known.

As used herein, the term "Negative Likelihood Ratio" refers to a ratio designed to evaluate the rule-out performance of a test, it is ratio between the probability of a negative test result given the *presence* of *(the predisposition for)* the condition and the probability of a negative test result given the *absence* of *(the predisposition for)* the condition. It is calculated by dividing the False Negative Rate (or 1- Sensitivity) by the Specificity, (the detection rate of "(future) non-cases").

As used herein, the term "Positive Likelihood Ratio" refers to a ratio designed to evaluate the rule-in performance of a test, ratio between the probability of a positive test result given the *presence* of *(the predisposition for)* and the probability of a positive test result given the of *(the predisposition for)* the condition. It is calculated by dividing the Sensitivity (or True Positive Rate or detection rate of "(future) cases") by the False Positive Rate (1-Specificity).

As used herein the term "(future) case" refers to a subject which has (the predisposition for) an outcome.

As used herein the term "(future) non-case" refers to a subject which doesn't have (the predisposition for) an outcome.

In one embodiment, the methods of the invention are applied to low prevalence outcomes, in particular low prevalence health conditions. In another embodiment, the methods of the invention are applied to moderate to high prevalence outcomes, in particular moderate to high prevalence health conditions. As used herein, the term "low prevalence" as applied to a health condition means a health condition with a prevalence (incidence) typically equal or lower than 7%, or equal or lower than 5%, or equal or lower than 3%, or equal or lower than 1%. As used herein, the term "moderate to high prevalence condition" means a health condition that a health condition with a prevalence (incidence) typically higher than 7%, or equal or higher than 10%, or equal or higher than 20%, or equal or higher than 30%.

The methods of the invention are especially applicable to low prevalence health conditions. In this context, the term "low prevalence" refers to a health condition that has a frequency of less than 10% of the relevant population. An example is preeclampsia which has a prevalence of about 2-8% (i.e. about 20 to 80 in 1000 pregnant women will develop preeclampsia during pregnancy).

As used herein, the term "sufficient number of subject" as applied to a population subset generated using one of the models (M1, M2, M3,..., M^{nth}) refers to a cohort large enough to allow model to be identified which is capable of dividing the cohort into two subgroups, one of which complies with either a rule-in or rule-out cut-off threshold

As used herein, the term "diagnostic/prognostic core" refers to a specific recurrent combination of variables with exceptional prognostic performance, as found within a comprehensive collection of diagnostic/prognostic models available.

As described in the Background the statistics AUROC, Sn, and Sp are prevalence-independent statistics, yet prevalence (or incidence; depending on the application) is important when assessing the clinical usefulness of a diagnostic/prognostic test. Therefore, when a diagnostic/prognostic test is assessed / applied in its clinically relevant context, metrics like positive and negative predictive value (PPV and NPV), which take the disease prevalence (or incidence) into account, are far more appropriate. Here, PPV corresponds the fraction of patients that will actually have/develop the condition (TP, True Positives) within the group of all patients that have a positive test result (True Positives + False Positives (FP)). NPV corresponds to the fraction of patients that will not have/develop the conditions (TN, True Negatives) within the group of all patients that have a negative test result (True Negatives + False Negatives (FN)).

To link the use of prevalence independent statistics with prevalence-dependent statistics, the Applicants have developed a methodology to seamlessly link these two views of prognostic test performance, by plotting the PPV or NPV criteria, which account for prevalence, in the Receiver Operating Characteristic (ROC) space [1].

By integrating the methodology herein described, the methods outlined in this application are specifically suited for the identification of diagnostic/prognostic models and/or diagnostic/prognostic cores with clinical utility. The method and system of the invention enable the identification of diagnostic/prognostic models and/or diagnostic/prognostic cores with exceptional (future) case detection rates at a pre-set PPV criterion, when the clinical application requires for a rule-in prognostic test which controls the proportion of false positives.

Similarly, the methods enable the identification of diagnostic/prognostic models and/or diagnostic/prognostic cores with exceptional detection rates for (future) non-cases at a pre-set NPV criterion when the clinical application requires for a rule-out prognostic test which controls the proportion of false positives. Significantly, this also supports the notion, as realised by the Applicants, that a specific combination of variables constituting a prognostic test optimised for a given PPV criterion, is not necessarily the same combination of variables constituting a prognostic test optimised for a given NPV criterion.

By extension, prognostic models and/or prognostic cores which are optimised for a given PPV criterion for a rule-in test do not necessarily constitute the same variables as prognostic models and/or prognostic cores for a rule-in test which is optimised for a given FPR criterion. The same holds true for rule-out tests (NPV criterion vs. FNR criterion).

Therefore, one can also use the methodology elaborated here-in to assist in the development/ selection of appropriate diagnostic/prognostic models which meets with a particular pre-set rule-in or rule-out criteria.

As a non-limiting example, the Applications applied methods which capitalize on the creation of all comprehensive prognostic models with 1 to 2, 1 to 3, 1 to 4, or 1 to n variables, using one or more multivariable modelling techniques, to obtain a so called "comprehensive model space". This comprehensive model space is then interrogated by the application of specific success criteria to identify these models or "prognostic cores" which deliver good diagnostic/prognostic performance with regards to the pre-set rule-in or/and rule-out success criteria. For example, within the model space one will then identify those diagnostic/prognostic models and/or diagnostic/prognostic cores which maximize detection rate at the given pre-set predictive value criterion rather than merely focusing on AUROC. Other methods to develop /select appropriate models or cores can be employed (see example 4D). An example elaborating the process constituting 1) analytical technology (to determine levels of blood-borne metabolites and proteins; i.e. Physiological variables), 2) methodology, 3) data mining methodology and 4) the application of the methods as invention to establish novel prognostic models for predicting the risk of preeclampsia early in pregnancy are elaborated in Example 4. Examples of actual, novel prognostic models for pre-eclampsia risk prediction are also given in Example 4.

In Example 1, typical but non-limiting illustrations of the differences and eventual advantages of the use of PPV and NPV criteria compared to FPR or FNR criteria in the identification and assessment of clinically relevant prognostic classifiers are given.

Whereas the above methodology for the discovery of specific rule-in (or rule-out) diagnostic/prognostic models and/or prognostic cores involves the study of populations of many individuals and multiple characteristics thereof (i.e., variables), the resulting prognostic test has applicability at the level of the single individual. In any individual which is like the individuals in the study population, one can determine the levels/values of specific variables as per the identified prognostic model/core, and calculate the individuals risk score using the identified rule-in (or rule-out) prognostic model/core. Then, one will assess whether this risk score is higher or lower than a pre-specified threshold, whereby this threshold delineates the classification in "test-positive" or "test-negative", in accordance with the rule-in (or rule-out) classification established using the collection of methods elaborated in this application. As a result, one will be able to determine for any such individual, if he/she will be at increased risk (high-risk) of the outcome existing or occurring in a specific future timeframe (rule-in), or if he/she will be at decreased risk (low-risk) of the outcome existing of occurring in a specific future timeframe (rule-out).

The hypothetical scenario as presented in the "background to the invention, together with the above paragraphs and Example 1, clearly show that methods which integrate the use of PPV and/or NPV criteria to find prognostic models, and/or prognostic cores, are particularly suited to identify prognostic tests with clinically meaningful prognostic performance. However, the illustrations as elaborated in example 1 highlight some pertinent challenges to successfully develop such classifiers.

When the clinical requirement for a prognostic test calls for a rule-in test with a specific PPV, the paired [Sensitivity and Specificity] requirements for such test to comply with said specific PPV target, become progressively more stringent with decreasing outcome prevalence (or incidence); to the point where the existence of such classifier becomes improbable. At the same time, it can be appreciated that in clinical practice prognostic rule-in tests have the most utility for health outcomes with low prevalence (incidence) in the population of interest. Conversely, when the clinical requirement for a prognostic test calls for a rule-out test with a specific NPV, the paired [Sensitivity and Specificity] requirements for such test to comply with said specific NPV target become progressively more stringent with increasing outcome prevalence (or incidence). At the same time, it can be appreciated that in clinical practice, prognostic rule-out tests have the most utility for outcomes which are quite common in the population of interest.

Confronted with this problem, which essentially impedes the development of clinically meaningful prognostic tests for many health outcomes, the methods of the invention provide a solution to this problem.

### Process applicable to rule-in prognostic tests

Given the clinical need for a prognostic rule-in test delivering a minimal detection rate for future cases (Snₜₑₛₜ >= Sn_{target}), for a pre-set PPV threshold (PPVₜₑₛₜ >= PPV_{threshold}) for a health outcome with low incidence (or prevalence), the process involves the following discrete steps to enable the establishment of such prognostic performance:
1) Creation of a first comprehensive model space (Model-S1) of possible prognostic models for a given study population (Study-Pop1) using the methods as described earlier.
2) Definition of a "permissible" rate (FNR_{permissible}) or proportion (NPV_{permissible}) of future cases which can be misclassified as low-risk.
3) Identification of prognostic rule-out models and/or prognostic cores, in Model-S1 which maximize the specificity Sp (or detection rate of future non-cases) compliant with the rule-out criterion ((FNR_{permissible}) or (NPV_{permissible})) as defined in the previous step.
4) Selection of an appropriate rule-out model (classifier_{Rule-out}) as identified in the previous step and apply it to the study population (Study-Pop1). This will result in the creation of a defined low-risk population (PopLR), complying with a pre-specified number (FNR_{permissible}) or proportion (NPV_{permissible}) of false negatives and a significant fraction of the true negatives, i.e., future non-cases.
5) Purge this population from the initial study population to generate a novel study population (Study-Pop2), where (Study-Pop1) - (PopLR) = (Study-Pop2). It is of note that, compared to the initial Study-Pop1, the new study Study-Pop2 will have a higher incidence (or prevalence) of future cases.
6) Creation of a second comprehensive model space (Model-S2) of possible prognostic models for the novel study population (Study-Pop2) using the methods as described earlier.
7) Identification of prognostic rule-in models and/or prognostic cores, in Model-S2 which maximize the sensitivity Sn (or detection rate of future cases) compliant with the threshold PPV criterion (PPV_{threshold}). As the incidence (or prevalence) in Study-Pop2 is enriched in future cases compared to the Study-Pop1, it is much easier to find a classifier which meets the paired [Sensitivity and Specificity] requirements required to comply with said specific PPV_{threshold}.
8) Selection of an appropriate rule-in model (classifier_{Rule-in}) as identified in the previous step which delivers a detection rate for future cases which, after correction for the permissible misclassifications (cf. steps 2 and 3), meets the target sensitivity, i.e., Snₜₑₛₜ >= Sn_{target}.

The outcome of this process is a specific pair of prognostic models (or prognostic cores), i.e., a specific rule-out model and a specific rule-in model which, when applied jointly and sequentially will deliver significantly better rule-in prognostic performance than a rule-in model not preceded by a rule-out step.

The outlined process to achieve clinically relevant prognostic rule-in performance forms an integral part to the methods regarding the identification of non-obvious prognostic combinations as elaborated within this application. Preferred combinations of rule-out / rule-in cores for pre-eclampsia are provided in Example 4.

### Process applicable to rule-out prognostic tests

Given the clinical need for a prognostic rule-out test delivering a maximum detection rate for future non-cases (Spₜₑₛₜ >= Sp_{target}), for a pre-set NPV threshold (NPVₜₑₛₜ >= NPV_{threshold}) for a health outcome with moderate to high incidence (or prevalence), the process involves the following discrete steps to enable the establishment of such prognostic performance:
1) Creation of a first comprehensive model space (Model-S1) of possible prognostic models for a given study population (Study-Pop1) using the methods as described earlier.
2) Definition of a "permissible" rate (FPR_{permissible}) or proportion (PPV_{permissible}) of future non-cases which can be misclassified as high-risk.
3) Identification of prognostic rule-in models and/or prognostic cores, in Model-S1 which maximize the specificity Sn (or detection rate of future cases) compliant with the rule-in criterion ((FPR_{permissible}) or (PPV_{permissible})) as defined in the previous step.
4) Selection of an appropriate rule-in model (classifier_{Rule-in}) as identified in the previous step and apply it to the study population (Study-Pop1); this will result in the creation of a defined high-risk population (PopHR), constituting a pre-specified number (FPR_{permissible}) or proportion (PPV_{permissible}) of false positives and a significant fraction of the true positives, i.e., future cases.
5) Purge this population from the initial study population to generate a novel study population (Study-Pop2), where (Study-Pop1) - (PopHR) = (Study-Pop2). It is of note that, compared to the initial Study-Pop1, the new study Study-Pop2 will have a higher incidence (or prevalence) of future non-cases.
6) Creation of a second comprehensive model space (Model-S2) of possible prognostic models for the novel study population (Study-Pop2) using the methods as described earlier.
7) Identification of prognostic rule-out models and/or prognostic cores, in Model-S2 which maximize the specificity Sp (or detection rate of future non-cases) compliant with the threshold NPV criterion (NPV_{threshold}). As the incidence (or prevalence) in Study-Pop2 is enriched in future non-cases compared to the Study-Pop1, it is much easier to find a classifier which meets the paired [Sensitivity and Specificity] requirements required to comply with said specific NPV_{threshold}.
8) Selection of an appropriate rule-out model (classifier_{Rule-out}) as identified in the previous step which delivers detection rate for future non-cases which, after correction for the permissible misclassifications (cf. steps 2 and 3), meets the target specificity, i.e., Spₜₑₛₜ >= Sp_{target}.

The outcome of this process is a specific pair of prognostic models (or prognostic cores), i.e., a specific rule-in model and a specific rule-out model which, when applied jointly and sequentially will deliver significantly better rule-out prognostic performance than a rule-out model not preceded by a rule-in step.

Significantly, these elaborated stepwise processes capitalize on the notion, as realised by the inventors, that a specific combination of variables constituting a prognostic test with exceptional rule-out merits, is not necessarily the same combination of variables constituting a prognostic test with exceptional rule-in merits. Moreover, the characteristics of a transient population (Study-Pop2), following removal of specific sets of individuals, will be different compared to the initial test-population (Study-Pop1). As a result, the prognostic models within the respective comprehensive model spaces, i.e., model-S1 and model-S2) may be different.

In Example 2 typical but non-limiting illustrations of this sequential process and its specific merits compared to the single step methods in the identification and assessment of clinically relevant prognostic classifiers are elaborated.

Whereas the above collection of methods for the discovery of specific combination of 1) a rule-in prognostic model and/or prognostic core and 2) a rule-out prognostic model and/or prognostic core (or vice versa), involves the study of populations of many individuals and multiple characteristics thereof (i.e., variables), the resulting prognostic test has applicability at the level of the single individual. In any individual which is like the individuals in the study population, one can determine the levels/values of specific variables as per the first identified prognostic model/core, and calculate the individuals risk score using the identified rule-out (or rule-in) prognostic model/core. Then, one will assess whether this risk score is higher or lower than a pre-specified threshold, whereby this threshold delineates the classification in "test-positive" or "test-negative", in accordance with the rule-in (or rule-out) classification established using the collection of methods elaborated in this application. In the event that the individual is classified as "test-negative", one can determine the levels/values of specific variables as per the second identified prognostic model/core, and calculate the individuals risk score using the identified rule-in (or rule-out) prognostic model/core. By executing these two consecutive steps, one can determine for any such individual, if he/she will be at increased risk (high-risk) of the outcome occurring in a specific future timeframe (rule-in), or if he/she will be at decreased risk (low-risk) of the outcome occurring in a specific future timeframe (rule-out).

Likewise, calculating the consecutive risk scores, "test-positive" / "test negative" delineations, and final risk classification, i.e., being at high-risk (rule-in) or being at low-risk (rule-out) can be executed in a single calculation process.

*Prognostic Performance - performance maximisation by process of sequential classifiers.*

In some instances, and in view of achieving targets as pertinent to meeting clinical utility requirements, the inventors found that a further expansion of the above concept can deliver exceptional prognostic performance.

Given the clinical need for a prognostic test delivering a maximum detection rate of future cases (Sn,test_maximal), for a pre-set PPV threshold (PPVₜₑₛₜ >= PPV_{threshold}) or (/and), a prognostic test delivering a maximum detection rate of future non-cases (Sp,test_maximal), for a pre-set NPV threshold (NPVₜₑₛₜ >= NPV_{threshold}). The process involves the following discrete steps to enable the establishment of such prognostic performance:
1)Create a first comprehensive model space (Model-S1) of possible prognostic models for a given study population (Study-Pop1) using the methods as described earlier.
2)
   a. Identify a classifier within Study-Pop1 which either identifies a sub-population at high-risk (PopHR1) compliant with the PPV-threshold (progress to 3.a)), or
   b. Identify a classifier within Study-Pop1 which identifies a sub-population at low-risk (PopLR1) compliant with the NPV-threshold (progress to 3.b))
3)
   a. Purge this population from the initial study population to generate a novel study population Study-Pop2, where (Study-Pop1) - (PopHR1) = (Study-Pop2). It is of note that, compared to the initial Study-Pop1, the new study Study-Pop2 is effectively enriched in future non-cases. In the event the latter Study-Pop2 population meets the pre-set NPV criterion also, the classification process is halted as the pre-set goals are met. Otherwise, progress to step 4.
   b. Purge this population from the initial study population to generate a novel study population (Study-Pop2), where (Study-Pop1) - (PopLR1) = (Study-Pop2). It is of note that, compared to the initial Study-Pop1, the new study Study-Pop2 is effectively enriched in future cases. In the event the latter Study-Pop2 population meets the pre-set PPV criterion also, the classification process is halted as the pre-set goals are met. Otherwise, progress to step 4.
   c. In the event that no useful prognostic models are found in 3a) or 3b), the risk remaining in Study-Pop2 should be calculated and reported and the process terminated.
4) Create a second comprehensive model space (Model-S2) of possible prognostic models for the novel study population (Study-Pop2) using the methods as described earlier
5) Identify a classifier within Study-Pop2 which either;
   a. Identifies a sub-population at high-risk (PopHR2) compliant with the PPV-threshold and add this novel sub-population to PopHR1 (if such exists), to create a PopHR_total (progress to 6.a), or
   b. Identifies a sub-population at low-risk (PopLR2) compliant with the NPV-threshold and add this novel sub-population to PopLR1 (is such exists), to create a PopLR_total (progress to 6.b)
   c. In the event that no useful prognostic models are found in 5a) or 5b), the risk remaining in Study-Pop2 should be calculated and reported and the process terminated.
6)
   a. Purge PopHR2 from the (Study-Pop2) population to generate a novel study population (Study-Pop3), where (Study-Pop2) - (PopHR2) = (Study-Pop3). It is of note that, compared to the Study-Pop2, the new study Study-Pop3 is effectively enriched in future non-cases. In the event that the latter Study-Pop3 population meets the pre-set NPV criterion also, the classification process is halted as the pre-set goals are met. Otherwise, progress to step 7.
   b. Purge PopLR2 from the initial study population to generate a novel study population (Study-Pop3), where (Study-Pop2) - (PopLR2) = (Study-Pop3). It is of note that, compared to Study-Pop2, the new study Study-Pop3 is effectively enriched in future cases. In the event that the latter Study-Pop3 population meets the pre-set PPV criterion also, the classification process is halted as the pre-set goals are met. Otherwise, progress to step 7.
   c. In the event that no useful prognostic models are found in 5a) or 5b), the risk remaining in Study-Pop2 should be calculated and reported and the process terminated.
7) Repeat the steps 4 to 6, till maximum detection rate of future cases (Sn,test_maximal), for a pre-set PPV threshold (PPVₜₑₛₜ >= PPV_{threshold}) or (/and), maximum detection rate of future non-cases (Sp,test_maximal), for a pre-set NPV threshold (NPVₜₑₛₜ >= NPV_{threshold}) as achievable using the variables available for the individuals under study.

The outcome of this process is a specific Total Classifier, which is made up of a set of prognostic models (or prognostic cores) which, when applied sequentially will deliver exceptional rule-in or/and rule-out prognostic performance, in accordance with pre-set clinical requirements for risk classification.

It is of note that, in applying this process, one can either select for a rule-in classifier or a rule-out classifier at any point. In other words, to achieve the desired prognostic classification performance, one shall always apply the best classifier and hence possibly but not necessarily, alternate between rule-in and rule-out classifiers.

Furthermore, to comply with the pre-set PPV and/or NPV requirements, it is sufficient that the respective final PopHR_total = PopHR1 + PopHR2+.... + PopHRn and /or PopLR_total = PopLR1 + PopLR2+.... + PopLRn comply with the pre-set criterions. The interim classifications can therefore deviate from the set thresholds.

The outlined process to achieve clinically relevant prognostic rule-in performance forms an integral part of the methods regarding the identification of non-obvious prognostic combinations as elaborated within this application.

Preferred combinations of multiple prognostic classifiers for preterm pre-eclampsia are described Example 4D

In Example 3, typical but non-limiting illustrations of this iterative sequential process and its specific merits compared to the single step methods in the identification and assessment of clinically relevant prognostic classifiers is elaborated.

Whereas the above collection of methods for the discovery of a specific combinations of prognostic models involves the study of populations of many individuals and multiple characteristics thereof (i.e., variables), the resulting total prognostic test has applicability at the level of the single individual. In any individual which is like the individuals in the study population, one can determine the levels/values of specific variables as per the first identified prognostic model/core, and calculate the individuals risk score using the identified prognostic model/core. Then, one will assess whether this risk score is higher or lower than a pre-specified threshold, whereby this threshold delineates the classification in "test-positive" or "test-negative", in accordance with the classification (rule-in or rule-out) established using the collection of methods elaborated in this application. When the individual is classified "test-positive", the corresponding result will be reported (either the individual is classified as high-risk or low-risk, depending on the classifier applied). In the event, the individual is classified as "test-negative", one can determine the levels/values of specific variables as per the second identified prognostic model/core, and calculate the individuals risk score using the identified rule-in (or rule-out) prognostic model/core. Then, one will assess whether this risk score is higher or lower than a pre-specified threshold, whereby this threshold delineates the classification in "test-positive" or "test-negative", in accordance with the classification (rule-in or rule-out) established using the collection of methods elaborated in this application. When the individual is classified "test-positive" in this 2nd step, the corresponding result will be reported (either the individual is classified as high-risk or low-risk, depending on the classifier applied). In the event, the individual is classified as "test-negative", one can determine the levels/values of specific variables as per the third identified prognostic model/core, and calculate the individuals risk score using the identified rule-in (or rule-out) prognostic model/core, etc. This will be repeated till such time the individual is classified in a "test-positive" group or till one has calculated for the individual a risk scores for each of the classifiers constituting the "total classifier". At that time, the individual will be either triaged as being high-risk or low-risk, or remain un-classified with regards to the pre-set PPV- or/and NPV- criteria.

In the event that the clinical requirements stipulate that a prognostic test should consider both the PPV and NPV criterions at the same time (cf. Example 3), individuals who remain unclassified with regards to the pre-set PPV- and NPV- criterions, are considered unclassified.

Calculating the consecutive risk scores, "test-positive" / "test negative" delineations, and final risk classification, i.e., being at high-risk (rule-in), being at low-risk (rule-out) or, unclassified (in the event of a combined [rule-in - rule-out] criterion) can be executed in a single calculation process.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLE 1

A non-limiting set of numerical examples is elaborated to illustrate the concepts of rule-in and rule-out tests, and variations thereof, as applied within the methods described.

For the sole purpose of exemplifying, the following "base-case" population is construed:

| | |
|---|---|
| - Population | n= 400 |
| - Incidence / prevalence | p= 0.05 (the condition will develop in 5% of the population in a time after the prognostic test is applied), or |
| o Future Cases: | n = 20 |
| o Future non-cases: | n =380 |

A graphical representation of this "base-case" population (pre-test) is shown in Figure 1.

In medicine, prognosis commonly relates to the probability or risk of an individual developing a particular state of health (an outcome) over a specific time, based on his or her clinical and non-clinical profile, i.e., a set of variables.

As prognostic models add the element of time, prognosis includes a stochastic element, one that is subject to chance. Prognostication and prediction involve estimating risk, or the probability of a future event or state. The outcome not only is unknown, but does not yet exist, distinguishing this task from diagnosis. In other words, in prognosis, a classifier will, per definition, not be perfect, whereas, theoretically a diagnostic classifier can be. Consequently, the perfect classifier example presented in Figure 2 Example is essentially impossible to find in prognostic research.

### Rule-in classifiers

A rule-in prognostic test is typically geared to identify those individuals who are at an increased risk of developing a future health condition. To this end, one will try to develop classifiers which are identifying a "high-risk" group, i.e., individuals at increased risk of developing the future health condition, as compared to the unstratified population

For the sole purpose of exemplifying, the following rule-in "base-case" is considered:
- a rule-in test which will deliver detection rate of "future cases" in the "high-risk group" of 50%, i.e., Sn= 0.5

Classically, one will apply a False Positive Rate (1-specificity) threshold, i.e., a fixed percentage of individuals who will not develop the condition will be falsely classified as being at risk.

For the sole purpose of exemplifying, the following fixed FPR rule-in "base-case" is considered:
- a rule-in test which will deliver detection rate of "future cases" in the "high risk group" of 50%, i.e., Sn= 0.5, for a given FPR= 20% (or [1-specificity] = 0.2). This is illustrated in Figure 3.

As elaborated elsewhere in this application, one would typically express the prognostic performance of a classifier by means of a ROC curve. For the given example, the 20% FPR threshold corresponds the vertical line in the ROC space; a hypothetical compliant classifier (exhibiting a 50% detection rate at 20% FPR is also shown in Figure 4.

The classifier as depicted in Figure 4 doesn't account for outcome incidence (prevalence). To exemplify this, consider varying the 5% incidence either to 2.5% (Figure 5 - Panel A) or to 10% (Figure 5 - Panel B), keeping the 50% Detection rate and the 20%FPR (as per the fixed FPR rule-in "base-case"). One can observe that the proportion of "False Positives" is inversely associated with the outcome incidence. Yet, the classifier as depicted in Figure 4 would be compliant with the three incidence (prevalence) scenarios, i.e., p= 0.025, p= 0.05 and p= 0.1.

As elaborated elsewhere in this application, the clinical utility of a prognostic test, and hence its relevance to clinical practice, is often determined by the cost (monetary and/or health-wise) associated with the classification. In this context, one will typically but not limiting, weigh the impact of the "rule-in" classification on the correctly classified future-cases (true positives) as well as on the incorrectly classified future non-cases (false positives). One can appreciate that such assessment of clinical utility is highly dependent on the true positive/false positive ratio. As exemplified in the above fixed FPR rule-in illustrations, the use of a fixed FPR criterion is invariant to disease incidence (prevalence), and is therefore not appropriate for assessing the clinical utility of a prognostic test, and hence its relevance to clinical practice. To overcome this limitation, the Applicants developed a methodology to enable the discovery of prognostic models / prognostic cores which comply with a pre-set positive predictive value. PPV is the fraction of patients that will actually develop the condition (TP) within the group of all patients that have a positive test result (TP+FP); PPV accounts for the incidence (or prevalence) of the health condition. Hence PPV criterions are found more relevant in the development (and evaluation) of novel prognostic classifiers.

For the sole purpose of exemplifying, the following fixed PPV rule-in "base-case" is considered:
- a rule-in test which will deliver detection rate of "future cases" in the "high-risk group" of 50%, i.e., Sn= 0.5, for a given PPV= 0.2. This is illustrated in Figure 6.

As per the inventor's methodology [1], one can also project this PPV criterion on the ROC space. For the given example, the PPV=0.2 threshold as calculated for a 5% incidence (or prevalence), corresponds the straight line in the ROC space in Figure 7. A hypothetical compliant classifier (exhibiting a 50% detection rate at PPV= 0.2) is also shown.

In view of developing "rule-in" prognostic classifiers with clinical relevance, the use of a fixed PPV criterion allows for a more meaningful assessment of the performance of prognostic classifiers. At the same time, as PPV-criteria do account for outcome incidence (or prevalence), the compliance of a classifier with a PPV criterion is dependent on said incidence (or prevalence).

To exemplify this, consider varying the 5% incidence in the previous example, to either to 2.5% (Figure 8 - Panel A) or to 10% (Figure 8 - Panel B) while retaining the same PPV= 0.2 criterion. One can observe that the proportion of "True Positives" to "False Positives" is constant irrespective of the outcome incidence.

Yet, in the ROC space, the PPV-criterion will vary with the outcome incidence (prevalence). For the sole purpose of exemplifying, the hypothetical classifier as presented in Figure 7, which delivered 50% detection rate at PPV= 0.2 for a 5% outcome incidence, is replicated in Figure 9 for the scenarios with 2.5% outcome incidence (Figure 9 Panel A) and 10% outcome incidence (Figure 9 Panel B).

From these illustrations, it is obvious that the prognostic requirements for a given PPV rule-in criterion (in terms of sensitivity and specificity) get more stringent the lower the incidence (prevalence) of disease. In view of the methods elaborated in this application, the reader can appreciate that it is more challenging to find suitable prognostic models / prognostic cores given a clinically relevant target PPV-criterion with decreasing outcome incidence (prevalence).

### Rule-out classifiers

A rule-out prognostic test is typically geared to identify these individuals at decreased risk of developing a future health condition. To this end, one will try to develop classifiers which are identifying a "low-risk" group, i.e., individuals at decreased risk of developing the future health condition, as compared to the unstratified population.

For the sole purpose of exemplifying, the following rule-out "base-case" is considered:
- a rule-out test which will deliver detection rate of "future non-cases" in the "low-risk group" of 50%, i.e., Specificity Sp= 0.5

Classically, one will apply a False Negative Rate (1-sensitivity) threshold, i.e., a fixed percentage of individuals who will develop the condition will be falsely classified as being at low risk.

For the sole purpose of exemplifying, the following fixed FNR rule-out "base-case 2" is considered:
- a rule-out test which will deliver detection rate of "future non-cases" in the "low-risk group" of 50%, i.e., Sp= 0.5, for a given FNR= 10% (or [1-sensitivity] = 0.1). This is illustrated in Figure 10.

In Panel A the example is presented in a grid. Panel B exemplifies the prognostic performance of such rule-out classifier by means of a ROC curve; a hypothetical compliant classifier, exhibiting a specificity Sp= 0.5 at 10% FNR, is also given.

Again, the use of fixed FNR criterion to assess the performance of a prognostic rule-out classifier, does not account for outcome incidence (prevalence). To exemplify this, consider varying the 5% incidence of the "base case" population either to 2.5% (Figure 11 - Panel A) or to 10% (Figure 11 - Panel B), keeping the Specificity =0.5 and the 10% FNR (as per the fixed FNR rule-out "base-case2"). One can observe that the proportion of "False Negatives" is directly associated with the outcome incidence.

Again, as the clinical utility of a prognostic test is often determined by the cost (monetary and/or health-wise) associated with the classification, the use of an NPV threshold, which accounts for outcome incidence (or prevalence), might be more appropriate to assess prognostic performance of a rule-out test. NPV is the fraction of patients that will not develop the condition (TN) within the group of all patients that have a negative test (TN+FN).

For the sole purpose of exemplifying, the following fixed NPV rule-out "base-case" is considered:
- a rule-out test which will deliver specificity = 0.5, i.e., 50% detection rate of "future non- cases", in the "low- risk group for a set NPV = 0.99. The latter corresponds to 1 false negative (i.e., a future case falsely classified to be at low-risk) per 99 true negatives. (This corresponds with the earlier Figure 10 - Panel A).

As per the Applicants' methodology [1], one can also project this NPV criterion on the ROC space. For the given example, the NPV=0.99 threshold as calculated for a 5% incidence (or prevalence), corresponds the straight line in the ROC space; a hypothetical compliant classifier (exhibiting a specificity = 0.5 at NPV =0.99) is also shown (Figure 12).

As with the PPV case above, the NPV-criterion will conserve a constant proportion of true negatives over false negatives [not shown]. In the ROC space, the NPV-criterion will vary with the outcome incidence (or prevalence). For the sole purpose of exemplifying, the hypothetical classifier as presented in Figure 12, is replicated below for the scenarios with 2.5% outcome incidence (Figure13 Panel A) and 10% outcome incidence (Figure13 Panel B).

From these illustrations, it is obvious that the prognostic requirements for a given NPV rule-out criterion (in terms of sensitivity and specificity) get more stringent the higher the incidence (prevalence) of disease. In view of the methods elaborated in this application, the reader can appreciate that it is more challenging to find suitable prognostic models / prognostic cores given a clinically relevant target NPV-criterion with increasing outcome incidence / prevalence.

### EXAMPLE 2

A non-limiting set of numerical examples is elaborated to illustrate the concepts of the sequential application of [rule-out and rule-in tests], to deliver exceptional rule-in classification, or the sequential application of [rule-in and rule-out tests], to deliver exceptional rule-out classification.

For the sole purpose of exemplifying, the same "base-case" population as per Example 16 is considered.

| | |
|---|---|
| - Population | n= 400 |
| - Incidence / prevalence | p= 0.05 (the condition will develop in 5% of the population in a time after the prognostic test is applied), or |
| o Future Cases: | n = 20 |
| o Future non-cases: | n =380 |

### Goal: Clinically Relevant Rule-in classification

A rule-in prognostic test is typically geared to identify those individuals who are at an increased risk of developing a future health condition. To this end, one will try to develop classifiers which are identifying a "high-risk" group, i.e., individuals at increased risk of developing the future health condition, as compared to the unstratified population.

For the sole purpose of exemplifying, the following rule-in classification target is considered:
- To be clinically meaningful a rule-in test should deliver a detection rate of "future cases" in the "high-risk group" of >= 70%, i.e., Sn >= 0.7, for a Positive Predictive Value =>0.2.

In Figure 14, these prognostic targets are exemplified in "grid" format (Panel A) as well as in the ROC space (Panel B). From the latter panel B, one can appreciate that a compliant classifier needs to have an ROC curve with points in the area designated A in the ROC space. At a minimum the classifier needs to meet the [Sensitivity = 0.7, 1-Specificity = 0.15] pair. It can also be appreciated that prognostic classifiers with these minimal paired [Sensitivity - Specificity] will be either hard to establish or possibly will not exist.

For those instances where one cannot establish classifiers, constituting combinations of variables and / or prognostic cores, delivering the prognostic rule-in performance as mandated by the clinical context, the Applicants established a novel process.

For the sole purpose of exemplifying, consider the following stepwise process:
1) Establish a "permissible" fraction of future-cases which will be certainly miss-classified; establishing this fraction will typically but not limiting, be determined by the health care context and can vary accordingly. For the example here, it is assumed that it is acceptable that 10% of the future-cases will be miss-classified; this corresponds a 10% FNR threshold (FNR_{permissible}) for a rule-out test as exemplified in Example 1.
2) Applying the methods elaborated earlier, one can establish combinations of variables and/or prognostic cores which can classify a test population (in this example: Study-Pop1; n= 400) in accordance with this criterion. For the sole purpose of exemplifying, assume one can develop a rule-out classifier, which correctly classifies 50% of the future non-cases, together with 10% of the future cases (i.e., false negatives). The paired [Sensitivity-Specificity] requirements for such classifier are far more modest, and hence the likelihood of identifying such classifiers is higher.
3) Apply this classifier to the Study-Pop1 and create a "low-risk" population PopLR, and a novel Study-Pop2. The individuals in the PopLR are considered "fully classified" and not considered further. This is exemplified in Figure 15. The individuals in Study-Pop2 remain in the process and will be considered for another classification step.
The composition of Study-Pop2 is now as follows

| | |
|---|---|
| - Population | n= 208 |
| o Future Cases: | n = 18 |
| o Future non-cases: | n =190 |
| - In the Study-Pop2 population, the outcome incidence / prevalence has increased from p=0.05 to p= 0.0865 (the condition will develop in 8.65% of the population Study-Pop2). | |

4) The second step of the process involves a de-novo application of the earlier elaborated methods to generate a new model-Space (model-S2) and identify all robust prognostic models therein. Then, one will look for prognostic models and/or prognostic cores which comply with the initial rule-in criterion, i.e., PPV= 0.2 and maximize the detection rate. To meet the earlier stipulated overall detection rate of 70%, this step needs to detect 70% of the 20 cases in StudyPop1, even though 2 of these future cases were misclassified in the first step of this process. It is therefore necessary to detect 14 cases out of 18 cases in StudyPop2. The detection rate for the second step rule-in classifier will therefore need to be 14/18 (Sn= TP/(TP+FN)), i.e., Sn>= 0.77. For the sole purpose of exemplifying, a rule-in classifier, which correctly classifies 77% of the future cases, given the pre-set PPV= 0.2 criterion, is illustrated in Figure 16.

While the detection rate (i.e., Sensitivity) of the second step needs to be higher to comply with the overall detection rate target, the enrichment of future-cases in the transient Study-Pop2 lowers the requirements for the paired [Sensitivity - Specificity] values of a classifier's ROC curve to meet the pre-set PPV-criterion. The latter "relaxation" of the PPV criterion outweighs the increased detection rate requirements. This is also apparent from comparing the size of the compliant Areas "A" in the ROC space (e.g. the area "A" in Figure 16 is significantly larger than the area "A" in Figure 14).

Based on this exemplification, it can be appreciated by the reader that the likelihood of identifying a rule-in classifier which complies with the PPV-criterion as relevant to the second step in this novel process is significantly higher compared to the likelihood of identifying a compliant single- step rule-in classifier. It can also be appreciated that the degree of "relaxation" of the PPV-criterion in the second step will depend on the prognostic performance achieved in the preceding rule-out step. The better the 1st step rule-out classification, the lower the requirements for the paired [Sensitivity - Specificity] values to meet the PPV-criterion in the 2nd step of classification, and vice versa.

In summary, the methods as exemplified in the above 4 steps, support the discovery of prognostic rule-in classifiers with exceptional prognostic performance by means of the identification of specific pairs of prognostic models and/or prognostic cores, involving a specific rule-out classifier and a specific rule-in classifier, applied jointly and consecutively. The given example considered the application of a FNR_{permissible} (rule-out) criterion and a PPV_{threshold} (rule-in) criterion. Alternative methods can involve, for example but not limiting, NPV_{permissible} (rule-out) and PPV_{threshold} (rule-in); or NLR_{permissible} (rule-out).

### Goal: Clinically Relevant Rule-out classification.

A rule-out prognostic test is typically geared to identify those individuals who are at a decreased risk of developing a future health condition. To this end, one will try to develop classifiers which are identifying a "low-risk" group, i.e., individuals at decreased risk of developing the future health condition, as compared to the unstratified population.

As highlighted in Example 1, achieving high rule-out prognostic performance has typically more clinical utility when the incidence (or prevalence) of the health outcome is more common. For this reason, a different "base-case2" population as compared to Example 16 is considered for the sole purpose of exemplifying.

| | |
|---|---|
| - Population | n= 400 |
| - Incidence / prevalence | p= 0.10 (the condition will develop in 10% of the population in a time after the prognostic test is applied), or |
| o Future Cases: | n = 40 |
| o Future non-cases: | n =360 |

For the sole purpose of exemplifying, the following rule-out classification target is considered:
- To be clinically meaningful a rule-in test should deliver a detection rate of "future non-cases" in the "low-risk group" of >= 50%, i.e., Sp>= 0.50, for a Negative Predictive Value=> 0.99.

In Figure 17, the pre-test "base-case" population (Panel A) is illustrated in grid-format, together with the prognostic targets which are exemplified in "grid" format (Panel B) as well as in the ROC space (Panel C). From the latter panel C, one can appreciate that a compliant classifier needs to have a ROC curve with points in the area designated A in the ROC space. At a minimum the classifier needs to meet the [Sensitivity = 0.955, 1-Specificity = 0.5] pair. It can also be appreciated that prognostic classifiers with these minimal paired [Sensitivity - Specificity] will be either hard to establish or possibly will not exist.

For those instances where one cannot establish classifiers, constituting combinations of variables and / or prognostic cores, delivering the prognostic rule-out performance as mandated by the clinical context, a stepwise process alike the one elaborated in the previous section [refer to Clinically Relevant Rule-in classification], is established by the inventors.

For the sole purpose of exemplifying, consider the following stepwise process:
1) Establish a "permissible" fraction of future non-cases which will be certainly miss-classified as being at risk. For the example here, it is assumed that it is deemed acceptable that 20% of the future non-cases will be miss-classified; this corresponds a 20% FPR threshold (FPR_{permissible}) for a rule-in test as per Example 1.
2) Applying the methods elaborated earlier, one can establish combinations of variables and/or prognostic cores which can classify a test population (in this example: Study-Pop1; n= 400) in accordance with this criterion. For the sole purpose of exemplifying, assume one can develop a rule-in classifier, which correctly classifies 70% of the future cases, together with 20% of the future non-cases (i.e., false positives). The paired [Sensitivity-Specificity] requirements for such classifier are far more modest, and hence the likelihood of identifying such classifiers is higher.
3) Apply this classifier to the Study-Pop1 and create a "high-risk" population PopHR, and a novel Study-Pop2. The individuals in the PopHR are considered "fully classified" and not considered further. This is exemplified in Figure 18. The individuals in Study-Pop2 remain in the process and will be considered for another classification step.
The composition of Study-Pop2 is now as follows

| | |
|---|---|
| - Population | n= 300 |
| o Future Cases: | n = 12 |
| o Future non-cases: | n =288 |
| - In the Study-Pop2 population the outcome incidence / prevalence is now decreased from p=0.1 to p= 0.04 (the condition will develop in 4% of the population Study-Pop2). | |

4) The second step of the process involves a de-novo application of the earlier elaborated methods to generate a new model-Space (model-S2) and identify all robust prognostic models therein. Then, one will look for prognostic models and/or prognostic cores which comply with the initial rule-out criterion, i.e., NPV =0.99 and maximize the specificity (i.e., detection rate for future non-cases).

To meet the earlier stipulated overall non-case detection rate of 50% (Sp=0.5), this step needs to detect 50% of the 360 non-cases in StudyPop1, even though 72 of these were misclassified in the first step of this process. It is therefore necessary to detect the 180 non-cases from the 288 non-cases in the StudyPop2. The specificity for the second step rule-out classifier will therefore need to be 180/288 (Sp= TN/(TN+FP)), i.e., Sp >= 0.625. For the sole purpose of exemplifying, a rule-out classifier, which correctly classifies 62.5% of the future non-cases, given the pre-set NPV =0.99 criterion is illustrated in Figure 19.

While the specificity of the second step needs to be higher to comply with the overall non-case detection rate target, the depletion of future cases (equivalent to an enrichment of future non-cases) in the transient Study-Pop2 lowers the requirements for the paired [Sensitivity - Specificity] values of a classifier's ROC curve to meet the pre-set NPV-criterion. The latter "relaxation" of the NPV criterion outweighs the increased specificity requirements. This is also apparent from comparing the size of the compliant Areas "A" in the ROC space (e.g. the area "A" in Figure 19 is significantly larger than the area "A" in Figure 17.

Based on this exemplification, it can be appreciated by the reader that the likelihood of identifying a rule-out classifier which complies with the NPV-criterion as relevant to the second step in this novel process is significantly higher compared to the likelihood of identifying a compliant single- step rule-out classifier. It can also be appreciated that the degree of "relaxation" of the NPV-criterion in the second step will depend on the prognostic performance achieved in the preceding rule-in step. The better the 1st step rule-in classification, the lower the requirements for the paired [Sensitivity - Specificity] values to meet the NPV-criterion in the 2nd step of classification, and vice versa.

In summary, the methods as exemplified in the above 4 steps, support the workings of prognostic rule-out classifiers with exceptional prognostic performance by means of the identification of specific pairs of prognostic models and/or prognostic cores, involving a specific rule-in classifier and a specific rule-out classifier, applied jointly and consecutively. The given example considered the application of a FPR_{permissible} (rule-in) criterion and a NPV_{threshold} (rule-out) criterion. Alternative methods can involve, for example but not limiting, PPV_{permissible} (rule-in) and NPV_{threshold} (rule-out); or PLR_{permissible} (rule-in) and NPV_{threshold} (rule-out).

### EXAMPLE 3

A non-limiting numerical example is elaborated to illustrate the concepts of the sequential application of multiple classifiers in conjunction with a decision criterion [fully classified yes/no] at each classification step, to deliver exceptional rule-in classification, or/and to deliver exceptional rule-out classification, as applied within the methods.

For the sole purpose of exemplifying, the same "base-case" population as per Example 1 is considered.

| | |
|---|---|
| - Population | n= 400 |
| - Incidence / prevalence | p= 0.05 (the condition will develop in 5% of the population in a time after the prognostic test is applied), or |
| o Future Cases: | n = 20 |
| o Future non-cases: | n =380 |

For the sole purpose of exemplifying, the following classification target is considered:
- To be clinically meaningful a prognostic test should maximize detection rate of future cases in a high-risk group with PPV >= 0.2 and, at the same time, maximize detection rate of future non-cases in a low risk-group wherefore the Negative Predictive Value >= 0.99.

In Figure 20, the classification aims for this exemplary prognostic test are illustrated. From Figure 20, it can be appreciated that classifiers compliant with the PPV >=0.2 high-risk criterion will need [Sensitivity-Specificity] pairs within the area designated "A", whereas classifiers compliant with the NPV >=0.99 high-risk criterion will need [Sensitivity-Specificity] pairs within the area designated "B".

For a prognostic test to comply with both the rule-in and rule-out criterion at the same time, it needs to have [Sensitivity-Specificity] pairs within the intersect of the areas "A" and "B". It can be appreciated that a single step prognostic classifier with [Sensitivity - Specificity] pairs in the intersect of the areas "A" and "B" will be hard to establish.

For a prognostic test which delivers [Sensitivity-Specificity] pairs within the "A" or "B" area, yet not in the intersect area "A" ∩ "B", an individual can be classified in any of the following 3 risk classes: High-risk, Low-Risk, or undetermined. A "undetermined" classification follows when an individual's calculated prognostic risk score is neither classifying it in the high-risk group nor in the low risk group. Their post-test probability for developing the outcome of interest is not in accordance with the pre-set clinical PPV or/and NPV criteria.

In accordance with the hypothetical clinical test requirements, the "rule-in" component of the classifier should maximize the Sensitivity (i.e., future case detection rate) for the given PPV criterion; whereas the "rule-out" component of the classifier should maximize the Specificity (i.e., future non-case detection rate).

For the sole purpose of exemplifying, consider the following hypothetical stepwise process:
1) Applying the methods elaborated earlier, one can establish combinations of variables and/or prognostic cores which can classify a test population (in this example: Study-Pop1; n= 400; cf. Figure 21 - Panel A).
2) Assume one can develop a rule-in classifier, which correctly classifies 50% of the future cases with a PPV = 0.2 (cf. Figure 21 - Panel B).
3) Apply this classifier to the Study-Pop1 and create a "high-risk" population PopHR1, and a novel Study-Pop2. The individuals in the PopHR1 are considered "fully classified" and not considered further. The individuals in Study-Pop2 remain in the process and will be considered for another classification step.
The composition of Study-Pop2 is now as follows

| | |
|---|---|
| - Population | n= 350 |
| o Future Cases: | n = 10 |
| o Future non-cases: | n = 340 |

4) Applying the methods elaborated earlier, one can establish combinations of variables and/or prognostic cores which can classify the Study-Pop2 population.
5) Assume one can develop a rule-out classifier, which correctly classifies ~40% of the future non-cases in Study-Pop2 with a NPV >= 0.99 (cf. Figure 21 - Panel C).
6) Apply this classifier to the Study-Pop2 and create a "low-risk" population PopLR1, and a novel Study-Pop3. The individuals in the PopLR1 are considered "fully classified" and not considered further. The individuals in Study-Pop3 remain in the process and will be considered for another classification step.
The composition of Study-Pop3 is now as follows

| | |
|---|---|
| - Population | n= 213 |
| o Future Cases: | n = 9 |
| o Future non-cases: | n = 204 |

7) Applying the methods elaborated earlier, one can establish combinations of variables and/or prognostic cores which can classify the Study-Pop3 population.
8) Assume one can develop another rule-out classifier, which correctly classifies 50% of the future non-cases in Study-Pop2 with a NPV >= 0.99 (cf. Figure 21 - Panel D).
9) Apply this classifier to the Study-Pop3 and create a "low-risk" population PopLR2, and a novel Study-Pop4. The individuals in the PopLR2 are considered "fully classified" and added to the total low-risk group (PopLRₜₒₜₐₗ = popLR1 + PopLR2). The individuals of PopLR2 are not considered further. The individuals in Study-Pop4 remain in the process and will be considered for another classification step.
The composition of Study-Pop4 is now as follows

| | |
|---|---|
| - Population | n= 111 |
| o Future Cases: | n = 9 |
| o Future non-cases: | n = 102 |

10) Assume one can develop a rule-in classifier, which correctly classifies 89% of the future cases with a PPV = 0.2 (cf. Figure 21 - Panel E).
11) Apply this classifier to the Study-Pop4 and create a "high-risk" population PopHR2.The latter are added to the total high-risk group (popHRₜₒₜₐₗ = popHR1 + PopHR2). Review of the remaining population shows its composition falls just short of the NPV >=0.99 criterion, i.e., NPV_{remainder} = 70/(70+1) = 0.986.

Since the decision criterion to further classify the remaining population is governed by whether the PPV or/and NPV criteria are met within the respective "total high-risk group" and the "total low-risk group", the impact of classifying the remaining population into the "total low-risk group" is assessed. It can be seen from Figure Example18/2 - Panel F, that the addition of the remaining population to the "total low-risk group" keeps the latter in compliance with pre-set NPV criterion. Therefore, the remaining population is also considered fully classified, and labelled accordingly as PopLR3. With no further classification required, the resulting total classification achieved follows the "summing" of the different intermediate high-risk groups into the total high-risk group, i.e., PopHRₜₒₜₐₗ = PopHR1 + PopHR2, and "summing" of the different intermediate low-risk groups into the total low-high risk group, i.e., PopLRₜₒₜₐₗ = PopLR1 + PopLR2 + PopLR3 (cf. Figure 21 - Panel F). It is important to note that both the total high-risk group as well as the total low-risk group comply with their respective pre-set criteria; i.e. a PPV >= 0.2 for the total high-risk group and a NPV >= 0.99 for the total low-risk group.

Moreover, for the illustration given, the "total classifier", constituting the specific set of 4 classifiers applied in a specific order, results in the classification of all individuals of the initial study-population in either a high-risk group or a low-risk group in accordance with the pre-set clinical requirements. In this instance there are no individuals which are classified as undetermined.

For the given hypothetical example, the here elaborated method results in a very specific "total classifier", and is associated with a single [sensitivity-specificity] pair in the ROC space. It can be seen in Figure 21 - Panel G that the "total classifier", constituting the specific set of 4 classifiers applied in a specific order, is simultaneously meeting both the rule-in PPV-criterion and the rule-out NPV-criterion, and thus lies in the intercept of the Areas "A" and "B".

In other instances, when the available variables and the classifiers derived thereof (with different classifiers being developed for each intermediate Study-PopN) do not support the discovery of a set of classifiers which achieves total classification of the study-population to meet pre-set PPV- and NPV- criteria simultaneously, one might still find a classifier, constituting a set of two [Sensitivity - Specificity] pairs, one in Area "A" and one in Area "B", yet neither point is in the intersect of the areas "A" and "B".

To illustrate this, consider the above exemplified stepwise process, but assume one cannot discover an appropriate classifier to further stratify Study-Pop4. So maximal classification was achieved at step 3 (cf. Figure 21 - Panel D). Within the "total classifier", constituting the specific set of 3 classifiers applied in a specific order, the rule-in classification is the result of the first classification step only (step-1, Rule-in; cf. Figure 21 - Panel B), whereas the rule-out classification follows the execution of 3 consecutive classification steps (step 1 to step 3, cf. Figure 21 - Panel B-D). The "total" classification agrees with the "crossed" zones in Figure 21 - Panel E. his translates into the ROC space as follows (cf. Figure 22):
- *Rule in [Sensitivity - Specificity] pair:*
   - Sn = 0.50, cf. PopHR1, or the fraction of future cases which are in PopHR1, i.e., 10/20 = 0.5
   - Sp = 0.895, which corresponds to the fraction of future non-cases which are not in PopHR1, i.e., 340/380 = 0.895
- *Rule out [Sensitivity - Specificity] pair:*
   - Sn = 0.95, (or FNR 1-Sn = 0.05) which corresponds to the fraction of future cases which are not in PopLR1 + PopLR2, i.e., 19/20 = 0.95
   - Sp = 0.895, or the fraction of future non-cases which are in are in PopLR1 + PopLR2, i.e., (136+102)/380 =0.626

Individuals of the initial Study-Pop1 which are neither classified in the high-risk PopHR1 nor in the low-risk PopLR1 + PopLR2, i.e., the individuals in the residual Study-Pop4, remain unclassified.

In summary, the methods as exemplified in this Example, support the discovery of prognostic classifiers with exceptional prognostic performance by means of the identification of specific sets of prognostic models and/or prognostic cores, which will then be applied jointly and consecutively. The number of consecutive steps as well as the order of rule-in and rule-out classifiers applied in the given examples are non-limiting. Any other permutation of number of steps or order of application of rule-in and rule-out classifiers are also covered by this application.

It will be appreciated that while the examples provided above are directed to the prediction of a health condition in a subject, the methods of the invention may also be applied to diagnosis of health conditions, in particular health conditions for which symptoms manifest at a late stage in the pathology of the health condition. The methods of the invention may also be applied to other health conditions, for example determining whether a subject will develop a neurodegenerative disease, a proliferative disorder, or a cardiovascular disease.

The methods of the invention may also be applied to predict health related outcomes, for example whether a primary cancer in a subject will metastasize, predicting whether a patient will develop sepsis in response to major surgery, and predicting whether a patient will reject a transplant. In addition, the methods of the invention may also be applied to predicting other outcomes in a subject, for example predicting the efficacy of a drug or therapy in a subject, predicting the outcome of a subject who has a health condition, and predicting whether a health condition will re-occur in the subject. The methods of the invention are also applicable to generating models for predicting non-medical outcomes, for example in the field of weather forecasting, predicting failure of electrical or mechanical components, machine learning and data mining research.

### EXAMPLE 4- Prognostic models for preeclampsia

The data analysis- methods elaborated here are focused on identifying non-obvious prognostic combinations of metabolites, and/or combinations of physiological variables, like metabolites, proteins and other variables, for a future health condition. In addition, the disclosed methods are construed as such that the non-obvious prognostic combinations of metabolites, and/or combinations of metabolites and other variables, will deliver clinically meaningful prognostic outcomes.

To exemplify these methods, the inventors applied the methods to identify specific non-obvious combinations of blood-borne metabolites, and/or combinations of blood-borne metabolites and other variables, to predict risk of preeclampsia in a pregnant woman prior to appearance of clinical symptoms of pre-eclampsia in the woman.

Preeclampsia (PE) is a disorder specific to pregnancy which occurs in 2-8% of all pregnancies. PE originates in the placenta and manifests as new-onset hypertension and proteinuria after 20 weeks' gestation. PE remains a leading cause of maternal and perinatal morbidity and mortality. Each year 70,000 mothers and 500,000 infants die from the direct consequences of PE. Maternal complications of PE include cerebrovascular accidents, liver rupture, pulmonary oedema or acute renal failure. For the fetus, placental insufficiency causes fetal growth restriction, which is associated with increased neonatal morbidity and mortality. To date, the only cure for PE is delivery of the placenta, and hence the baby. Consequently, iatrogenic prematurity adds to the burden of neonatal morbidity and mortality. The impact of PE on the health of patients is not restricted to the perinatal period: affected mothers have a lifelong increased risk of cardiovascular disease, stroke and type 2 diabetes mellitus. Children born prematurely as a result of PE may have neurocognitive development issues ranging from mild learning difficulties to severe disabilities. In the longer term young children and adolescents of pregnancies complicated by PE exhibit increased blood pressure and BMI compared to their peers, with increased incidences of diabetes, obesity, hypertension and cardiac disease.

Whilst there are currently no ready available treatments to cure preeclampsia when it manifests, there are some drug treatments, i.e, aspirin and metformin (and others), which have the potential to prevent some of the preeclampsia cases developing. However, for these prophylactic interventions with therapeutics to impact on the incidence of preeclampsia at the population level to be effective, health care providers need to have a risk stratification tool, or test, which combines the following two attributes: identification of these pregnancies at increased risk for the disease early in pregnancy and triage the pregnancies to the appropriate treatment. These requirements follow the precautionary principle that one should not do harm to the pregnant woman and her unborn child. A blanket administration of drugs to all pregnancies in order to prevent preeclampsia in some, might incur unnecessary health risks (e.g., due to treatment side effects) in these who are not at risk in the first place. Contemporary hypotheses regarding the etiological causes of preeclampsia suggest that PE is syndromic in nature, and that preeclampsia is possibly more than one disease. In this hypothesis framework, the ability for accurately assessing early in pregnancy (several months prior to the manifestation of any clinical symptoms) which pregnant women are at increased (or high) risk of developing preeclampsia, and which women are a decreased (or low) risk of developing preeclampsia, will hinge on this assumption of preeclampsia being a multi-disease. However, currently there are no means to unambiguously delineate the different sub-types of preeclampsia or/and to delineate specific sub-populations at risk for preeclampsia (or any of its subtypes).

To overcome this, the Applicants, and others, use readily available maternal characteristics or/and pregnancy outcome data to create "proxy" sub-groups of pregnant women or "proxy" sub-types of pre-eclampsia, which are assumed to be enriched in either truly different patient risk profiles or truly different preeclampsia subtypes. Likewise, and as elaborated herein, next to considering preeclampsia a single disease entity, the Applicants delineated preeclampsia in 2 subtypes using a preeclampsia diagnosis in conjunction with the gestational age of delivery, i.e., preterm preeclampsia and term preeclampsia. Whereby preterm corresponds to a delivery before 37 weeks of gestation, and term to a delivery at or later than 37 weeks of gestation.

When these artificial "proxies" indeed enrich for a true sub-population risk profile or/and a true sub-type of disease, this should be reflected in the variables, or/and the prognostic models constituting combinations of variables, relevant to predicting preeclampsia in these proxy groups. One would expect different combinations of variables to be prognostic for different population risk-groups or/and preeclampsia subtypes. At the same time, the Applicants reasoned that by applying each "proxy-specific" prognostic model to solely determine the "proxy-specific risk", their prognostic performance will be higher as the background noise arising from the biological variability in the target population is decreased. For instance, a prognostic model for preterm preeclampsia should only be used to stratify pregnant women to their preterm preeclampsia risk and not for their "total preeclampsia" risk, as the prognostic performance of the preterm preeclampsia prognostic model will not adequately predict term PE risk, and thus deliver poor prediction of "total preeclampsia" also. While this concept appears trivial, it is often overlooked.

In addition, the Applicants also expanded this concept by considering the prognostic question of finding pregnant women at low risk of developing preeclampsia a different one than the prognostic question of finding pregnant women at high risk of developing preeclampsia. Whereas prognostic models for the first question may constitute variables indicative for maternal and fetal "good" health, prognostic models for preeclampsia may constitute variables indicative for maternal and fetal "ill" health, whereby the combinations of variables for "good" health and "ill health" are not necessarily the same.

Furthermore, the Applicants recognized that the prediction of preeclampsia, and its subtypes, corresponds to the development of prognostic and hence the need for non-trivial combinations of variables will be required to achieve clinical relevant prognostic performance for preeclampsia and its subtypes as considered in this application.

It is evident that application of the methods requires for the availability of an appropriate collection of blood samples (or other biospecimens), as obtained from pregnant women at a time point early in their pregnancy, more specifically a time point when none of the women shows any symptoms of pre-eclampsia. In addition, it is also evident that, in addition to the samples, information on the pregnancy outcomes, i.e., whether the pregnant woman developed pre-eclampsia during her pregnancy or whether she did not, is available. Preferentially, but not a conditio sine qua non, is the availability of additional variables, like relevant (clinical) risk factors as collected at time of sampling or as available in (medical) records, or the results of relevant, well-established clinical tests (e.g., glucose measurements) or quantification data of other types of relevant putative biomarker molecules, as available for the same sample / originator individual. For the avoidance of doubt, the diagnosis of pre-eclampsia (or absence thereof), is at a time point in the pregnancy which is distinctively later (typically, but not restrictive, 20 or more weeks later) in the pregnancy compared to the timepoint when the biospecimen is taken which is used for establishing the future risk of pre-eclampsia occurring.

Details on the study participants and samples used to find prognostic combinations of variables, selected from metabolites, proteins and clinical risks factors of interest are described in the section *"Participants and Specimens", and "Sample set used"*;

Details on the variables of interest, i.e., metabolites, proteins and clinical risks factors of interest are described in the section *"Participants and Specimens"*;

With the aim of the collection of methods as disclosed herein to develop clinically meaningful tests for pre-eclampsia prognosis, a non-limiting example of clinically meaningful test performance targets as adopted in this application is also presented in the section *"Exemplary Prognostic targets for pre-eclampsia risk stratification tests":* The Variables of interest and the methods to determine is outlined in the section "Collection of the analytical methods"

For the pre-eclampsia example elaborated here-in*,* the methods and data-analysis steps undertaken are presented in the section *"Collection of data and data analysis methods used".* Real examples of prognostic models of prediction of low and/or risk of preeclampsia in pregnant women, early in pregnancy, prior to development of clinical symptoms of preeclampsia are given in the section *"results".*

### Participants and Specimens:

Prospective clinical samples were collected from pregnant women with a singleton pregnancy at 15+/-1 and 20 +/-1 weeks' gestation and which were either diagnosed with preeclampsia (cases) or not diagnosed with preeclampsia (controls) in the further course of their pregnancy. All samples were obtained from participants in the SCOPE (Screening for Pregnancy Endpoints) prospective screening study of nulliparous women [8,9].

Written consent was obtained from each participant. The inclusion criteria applied for the study were nulliparity, singleton pregnancy, gestation age between 14 weeks 0 days and 16 weeks 6 days gestation and informed consent to participate. The exclusion criteria applied were: Unsure of last menstrual period (LMP) and unwilling to have ultrasound scan at <= 20 weeks, >=3 miscarriages, >=3 terminations, major fetal anomaly/abnormal karyotype, essential hypertension treated pre-pregnancy, moderate-severe hypertension at booking >=160/100 mmHg, diabetes, renal disease, systemic lupus erythematosus, anti-phospholipid syndrome, sickle cell disease, HIV positive, major uterine anomaly, cervical suture, knife cone biopsy, ruptured membranes now, long term steroids, treatment low-dose aspirin, treatment calcium (>1 g/24h), treatment eicosopentanoic acid (fish oil), treatment vitamin C >=1000mg & Vit E >=400iu, treatment heparin/low molecular weight heparin.

Preeclampsia defined as gestational hypertension (systolic blood pressure (BP) >= 140 mmHg and/or diastolic BP >= 90mmHg (Korotkoff V) on at least 2 occasions 4 hours apart after 20 weeks gestation but before the onset of labour) or postpartum systolic BP >= 140 mmHg and/or diastolic BP >= 90mmHg postpartum on at least 2 occasions 4 hours apart with proteinuria >= 300 mg/24h or spot urine protein: creatinine ratio >=30 mg/mmol creatinine or urine dipstick protein >= 2 or any multi-system complication of preeclampsia. Multisystem complications include any of the following: 1) Acute renal insufficiency defined as a new increase in serum creatinine >=100 umol/L antepartum or >130 umol/L postpartum 2) Liver disease defined as raised aspartate transaminase and/or alanine transaminase >45 IU/L and/or severe right upper quadrant or epigastric pain or liver rupture 3) Neurological problems defined as eclampsia or imminent eclampsia (severe headache with hyperreflexia and persistent visual disturbance) or cerebral haemorrhage 4) Haematological including thrombocytopenia (platelets <100 x 109/L), disseminated intravascular coagulation or haemolysis, diagnosed by features on blood film (e.g., fragmented cells, helmet cells) and reduced haptoglobin. Preeclampsia could be diagnosed at any stage during pregnancy after recruitment until delivery or in the first 2 weeks after delivery.

Clinical data on known risk factors for preeclampsia [4, 5] was collected at 15+/-1 and 20 +/-1 weeks' gestation by interview and examination of the women. Ultrasound data were obtained at 20 weeks on fetal measurements, anatomy, uterine and umbilical artery Doppler and cervical length. Fetal growth, uterine and umbilical Dopplers are measured at 24 weeks. Pregnancy outcome was tracked and the woman seen within 48 hours of delivery. Baby measurements are obtained within 48 hours of delivery.

### Sample set used:

A nested case-control study was conducted within the European branch of SCOPE, using blood samples taken at 15 +/-1 weeks of gestation; the cohort constituted a case:control ratio of ~1:3.5. Cases are defined as these pregnant women who develop preeclampsia (as defined earlier) in the course of their pregnancy: within the study 97 cases were considered, this corresponds all cases within the European branch of SCOPE for which samples were available. Controls were randomly selected amongst all other pregnancies. To avoid artefacts due to selection bias, the demographic and clinical characteristics of the control population selected to the study was compared and the lack of bias was verified using the appropriate tests; the Chi-squared, Spearman Correlation, Mann Whitney U and Kruskal-Wallis tests were used as appropriate. Samples of 335 controls pregnancies were selected to the study.

In **Table 2,** the baseline characteristics of this study cohort are presented.

**Table 2: Characteristics of the study population**

| **Characteristics** | **Controls** | **PE** | **Preterm PE** | **Non-Preterm PE** |
|---|---|---|---|---|
| **Number of samples** | 335 | 97 | 23 | 74 |
| **Gestation at sampling, wk** | 15.6 (0.726) | 15.6 (0.698) | 15.6 (0.491) | 15.6 (0.754) |

| **Population characteristics at time of sampling (15 weeks)** | | | | |
|---|---|---|---|---|
| **Maternal age, y** | 30 (27 - 33) | 30 (27 - 34) | 31 (28.0 - 33.5) | 29.5 (26.0 - 33.8) |
| **White ethnicity** | 315 (94%) | 92 (94.8%) | 22 (95.7%) | 70 (94.6%) |
| **Body mass index at 15 wk, kg/m2** | 23.5 (21.9 - 26.9) | 26.1 (23.1 - 29.4) ‡ | 25.8 (24.0 - 28.3) † | 26.2 (22.9 - 29.7) ‡ |
| **Waist circumference at 15 wk, cm** | 80 (74 - 86) | 84 (78 - 91) ‡ | 84 (78.0 - 89.5) | 84 (77.2 - 93.0) † |
| **Smoker at 15 wk** | 117 (34.9%) | 28 (28.9%) | 6 (26.1%) | 22 (29.7%) |
| **Smoker during first trimester** | 106 (31.6%) | 28 (28.9%) | 6 (26.1%) | 22 (29.7%) |
| **Blood pressure at 15 wk, mm Hg - Diastolic, 1st** | 64.8 (7.15) | 68.1 (7.98) ‡ | 66.1 (8.05) | 68.7 (7.92) ‡ |
| **Blood pressure at 15 wk, mm Hg - Mean arterial pressure, 1st** | 77.8 (7.68) | 82.1 (8.51) ‡ | 79.5 (8.34) | 82.9 (8.44) ‡ |
| **Blood pressure at 15 wk, mm Hg - Mean arterial pressure, 2nd** | 78.6 (7.45) | 83.1 (7.84) ‡ | 80.7 (7.52) | 83.9 (7.84) ‡ |
| **Blood pressure at 15 wk, mm Hg - Systolic,** 1st | 104 (10.7) | 110 (11.2) ‡ | 106 (9.7) | 111 (11.5) ‡ |
| **Random glucose measured by glucometer** at **15 wk, mmol/L** | 5.1 (4.6 - 5.6) | 5.2 (4.5 - 5.7) | 5.3 (4.65 - 6.15) | 5.1 (4.45 - 5.70) |

| **Population characteristics at time of delivery** | | | | |
|---|---|---|---|---|
| **Maximum blood pressure - Diastolic, mm Hg** | 80 (74 - 85) | 100 (95 - 107) ‡ | 107 (100 - 116) ‡ | 98 (95 - 105) ‡ |
| **Maximum blood pressure - Systolic, mm Hg** | 125 (118 - 134) | 154 (147 - 170) ‡ | 167 (154 - 180) ‡ | 151 (145 - 162) ‡ |
| **Proteinuria*** | 7 (2.09%) | 91 (93.8%) ‡ | 21 (91.3%) ‡ | 70 (94.6%) ‡ |
| **Multiorgan complications** | 0 (0%) | 23 (23.7%) ‡ | 9 (39.1%) ‡ | 14 (18.9%) † |
| **Pregnancy outcome - Gestation age at delivery, wk** | 40.4 (39.6 - 41.3) | 38.9 (37.1 - 40.3) ‡ | 34.3 (33.0 - 35.8) ‡ | 39.5 (38.6 - 40.4) ‡ |

| | | | | |
|---|---|---|---|---|
| *Results are expressed* as *mean (SD), median (interquartile range), or n* (%). **Urine dipstick* ≥2+ *or 24-h urine protein excretion* ≥*300 mg or spot urine protein:creatinine ratio ≥30 mg*/*mmol.* *†P<0.05; ‡P<0.001 cases vs controls, Chi-squared, T or Mann Whitney U test.* | | | | |

### Exemplary Prognostic targets for pre-eclampsia risk stratification tests:

As elaborated elsewhere in this application, the methods as disclosed herein enable for the discovery of combinations of variables for total pre-eclampsia, but also for clinically relevant subtypes of pre-eclampsia or/and for different patient populations with different risk profiles.

Within this application, the focus is on establishing prognostic combinations for different sub-types of pre-eclampsia within a specific patient population, i.e., 1^{st} time pregnant women without overt clinical risk factors.

The pre-eclampsia sub-types targeted here are
- total pre-eclampsia ("all PE")
- preterm pre-eclampsia (PT-PE): this is defined as pre-eclampsia which results in a (iatrogenic) delivery before 37 weeks of gestation, or preterm.
- term pre-eclampsia (T-PE): this is defined as pre-eclampsia which is associated with at delivery at or later than 37 weeks of gestation, or term.

### Rule-in targets, based on a False Positive Rate (Specificity) threshold:

Within this application, the discovery of prognostic models / cores which maximize the detection rate (Sensitivity) of future cases of pre-eclampsia, for a given False Positive Rate (FPR or (1-Specificity)) of future non-cases, is also considered. These prognostic models focus on identifying individuals who will develop pre-eclampsia. The following FPRs are considered: 20% FPR (Specificity =0.8) and 10% FPR (Specificity =0.9). In view of delivering a clinical meaningful rule-in test, the following minimal detection rates are put:
- 20% FPR: >= 50% detection rate of future PE cases (Sensitivity >= 0.5)
- 10% FPR: >= 40% detection rate of future PE cases (Sensitivity >= 0.4)

### Rule-out targets, based on a False Negative Rate (Sensitivity) threshold:

Within this application, the discovery of prognostic models / cores which maximize the detection rate (Specificity) of future non-cases of pre-eclampsia, for a given False Negative Rate (FNR or (1-Sensitivity)) of future cases, is also considered. These prognostic models focus on identifying individuals who will not develop pre-eclampsia. The following FNRs are considered: 20% FNR (Sensitivity =0.8) and 10% FNR (Sensitivity =0.9). In view of delivering a clinical meaningful rule-out test, the following minimal detection rates are put:
- 20% FNR: >= 40% detection rate of future non-PE cases (Specificity >= 0.40)
- 10% FPR: >= 30% detection rate of future non-PE cases (Specificity >= 0.30)

### Positive -and Negative predictive value thresholds

All PE: First time pregnant women have a risk of ~1/20 to develop pre-eclampsia, or a relative risk of approximately 2, compared to non-nulliparous.

In efforts to develop a clinically meaningful screening test, the inventors recently published the following rationale.[1] The prenatal management of a multiparous woman with regards to pre-eclampsia is largely guided by her previous pregnancy history. Epidemiological studies have shown that previous pre-eclampsia is associated with an increased risk of recurrence. For a second pregnancy, recurrence risks of about 1 in 8.6 to 1 in 6.8 (or PPV of 0.116 to 0.147) are reported, whereas a woman without prior pre-eclampsia, will have a lower risk of 1 in 77 to 1 in 100 (or NPV of 0.987 to 0.99). In line with this, if a woman has experienced pre-eclampsia in a previous pregnancy, she will be managed more vigilantly in most healthcare systems in high resource settings, with more prenatal visits compared to a woman who did not develop pre-eclampsia in any earlier pregnancy.

Based on the above, we proposed that a pre-eclampsia risk stratification test for nulliparous should ideally mimic the pre-eclampsia risk information as available for a second-time pregnant woman. Therefore, the test should either stratify nulliparous women to a high-risk group with a post-test pre-eclampsia probability of at least 1 in 7.5 (equivalent to a PPV = 0.133; rule-in) or stratify them to a low-risk group with a post-test probability of at least 1 in 90 (equivalent to a NPV = 0.988; rule-out) and ideally both. Based on this rationale, and taking into account the prevalence of PE as reported in the SCOPE cohort, the "All PE" PPV and NPV thresholds were established; cf. Table 3.

Preterm PE: For preterm PE, the PPV and NPV thresholds were adopted from a benchmark preterm PE test, which has been deployed already. [6, 7] Cf. Table 3.

Term PE: For term PE, the thresholds were determined in association with clinicians, and grossly correspond with an 5 time enrichment compared to the pre-test prevalence in either direction; i.e., the high risk threshold corresponds a ~5x pre-test probability for being a future PE case; the low risk threshold corresponds a ~5x pre-test probability for being a future non-PE case. Within this application only prognostic models for term PE are elaborated on. Cf. Table 3.

Whereas the above paragraphs set the minimum PPV- (rule-in) for each PE sub-type and/or patient sub-population, the following minimum (future) PE case detection rate is pursued for any of the given PPV-thresholds, i.e., the (future) case detection rate of at least 40% (Sensitivity >=0.4). Similarly, for any of the pre-set NPV- (rule-out) criterions, the following minimum future non-PE cases (or "controls") detection rate is pursued for any of the given NPV-thresholds, i.e., the future non-case detection rate of at least 30% (Specificity >=0.3).

The specific methods as relevant to achieving exceptional rule-in or/and rule-out prognostic performances are elaborated elsewhere in this application, as well as in Examples 16-18.

**Table 3 PPV-and NPV- based performance targets for prognostic tests for predicting the risk of Pre-eclampsia in pregnant women prior to appearance of clinical symptoms of PE.**

| **Outcome sub-type Outcome in sub-group** | **Prevalence in SCOPE** | **Rule-in tests** | | **Rule-out tests** | |
|---|---|---|---|---|---|
| | | PPV cut-off | Sensitivity target | NPV cut-off | Specificity target |
| All PE [1] | 0.05[9] | 1./7.5= 0.133 | Sₙ >=0.4 | 1-1/90= 0.988 | Sₚ >=0.3 |
| Preterm PE (<37 wks) [10] | 0.014[9] | 1/14= 0.0714 | Sₙ >=0.4 | 1-1/400= 0.9975 | Sₚ >=0.3 |
| Term PE (>=37) | 0.037[9] | 1/6.5= 0.154 | Sₙ >=0.4 | 1-1/160= 0.99375 | Sₚ >=0.3 |

For the avoidance of doubt, albeit the above performance targets are based on clinical relevance, they are not limiting; different healthcare settings may require for different targets. Furthermore, when novel prophylactic treatment options become available, different targets may be required based on e.g., cost of treatment or/and side-effects.

For the avoidance of doubt, the prognostic combinations of variables, as disclosed in this application, will also be relevant to the prognosis of pre-eclampsia in women in their 2^{nd} or higher pregnancy. As these multiparous women will have a "pregnancy history", which will impact on their risk for pre-eclampsia, it is easily understood that this information, when combined with the findings as disclosed within this application, will enhance the prognostic performances for predicting the risk of pre-eclampsia occurring in their pregnancies.

### Variables of interest:

**Table 4.** tabulates a non-limiting list of variables of interest which are considered in this application. The metabolites, proteins and clinical risk factors, are deemed relevant by the Applicants in view of identifying non-obvious prognostic combinations of variables, to predict risk of preeclampsia in a pregnant woman prior to appearance of clinical symptoms of pre-eclampsia in the woman pre-eclampsia. Where possible the metabolites of interest are identified by their CAS number, or/and their HMDB identifier; the molecular weights are also given (na: not available); Proteins are identified by their Gene identifier.

**Table Example 4. Variables of interest**

| **METABOLITES** | | | | | |
|---|---|---|---|---|---|
| **Metabolite** | **Code** | **Metabolite Class** | **HMDB** | **CAS** | **MW** |
| **25-Hydroxyvitamin D3** | HVD3 | Vitamin D and derivatives | 3550 | CAS 63283-36-3 | 400.6371 |
| **2-Hydroxybutanoic acid** | 2-HBA | (Keto- or Hydroxy) Fatty acids | 8 | CAS 5094-24-6 | 104.1045 |
| **2-methylglutaric acid** | 2-MGA | Dicarboxylic acids | 0000422 | CAS 617-62-9 | 146.1412 |
| **3-Hydroxybutanoic acid** | 3-HBA | (Keto- or Hydroxy) Fatty acids | 357 | CAS 300-85-6 | 104.1045 |
| **Adipic acid** | ADA | Dicarboxylic acids | 448 | CAS 124-04-9 | 146.1412 |
| **L-Alanine** | L-ALA | Amino acids | 161 | CAS 56-41-7 | 89.0932 |
| **Arachidonic acid** | ARA | (Unsaturated) Fatty acids | 1043 | CAS 506-32-1 | 304.4669 |
| **L-Arginine** | L-ARG | Amino acids | 517 | CAS 74-79-3 | 174.201 |
| **L-Leucine** | L-LEU | Amino acids | 687 | CAS 61-90-5 | 131.1729 |
| **8,11,14 Eicosatrienoic acid** | DGLA | (Unsaturated) Fatty acids | 2925 | CAS 1783-84-2 | 306.4828 |
| **Citrulline** | CR | Amino acids | 904 | CAS 372-75-8 | 175.1857 |
| **Decanoylcarnitine** | DC | Carnitines | 651 | CAS 1492-27-9 | 315.4482 |
| **Dodecanoyl-I-carnitine (C12)** | 12CAR | Carnitines | 2250 | CAS 25518-54-1 | 343.5014 |
| **Docosahexaenoic acid** | DHA | (Unsaturated) Fatty acids | 2183 | CAS 6217-54-5 | 328.4883 |
| **Dilinoleoyl-glycerol [isomer mixture of 1,3-Dilinoleoyl-glycerol 1,2-rac-Dilinoleoyl-glycerol]** | DLG | Diacylglycerols | 7248 | CAS 15818-46-9 | 616.9542 |
| | | | | CAS 30606-27-0 | |
| **Choline** | CL | Cholines | 97 | CAS 62-49-7 | 104.1708 |
| **Glycyl-glycine** | GG | Dipeptides | 11733 | CAS 556-50-3 | 132.1179 |
| **Homo-L-arginine** | H-L-ARG | Amino acids | 670 | CAS 156-86-5 | 188.2275 |
| **Hexadecanoic acid (palmitic acid)** | PALMA | (Saturated) Fatty acids | 220 | CAS 57-10-3 | 256.4241 |
| **L-Isoleucine** | L-ISO | Amino acids | 172 | CAS 73-32-5 | 131.1729 |
| **Linoleic acid** | LINA | (Unsaturated) Fatty acids | 673 | CAS 60-33-3 | 280.4455 |
| **L-methionine** | L-MET | Amino acids | 696 | CAS 63-68-3 | 149.211 |
| **NG-Monomethyl-L-arginine** | NGM | Amino acids | 29416 | CAS 17035-90-4 | 188.2275 |
| **Oleic acid** | OLA | (Unsaturated) Fatty acids | 207 | CAS 112-80-1 | 282.4614 |
| **L-Palmitoylcarnitine** | 16CAR | Acyl carnitines | 222 | CAS 6865-14-1 | 399.6077 |
| **Asymmetric dimethylarginine** | ADMA | Amino acids | 1539 | CAS 30315-93-6 | 202.2541 |
| **Sphingosine-1-phosphate** | S-1-P | Phosphosphingolipids | 277 | CAS 26993-30-6 | 379.4718 |
| **Sphinganine-1-phosphate (C18 base)** | Sa-1-P | Phosphosphingolipids | 1383 | CAS 19794-97-9 | 381.4877 |
| **Symmetric dimethylarginine** | sDMA | Amino acids | 3334 | CAS 30344-00-4 | 202.2541 |
| **Taurine** | TR | Amino acids | 251 | CAS 107-35-7 | 125.147 |
| **Isobutyrylglycine** | IBG | N-acyl-alpha amino acids | 730 | CAS 15926-18-8 | 145.1564 |
| **Urea** | UR | Amino ketones | 294 | CAS 57-13-6 | 60.0553 |
| **Stearoylcarnitine** | SC | Acyl carnitines | 848 | CAS 1976-27-8 | 427.6609 |
| **Eicosapentaenoic acid** | EPA | (Unsaturated) Fatty acids | 1999 | CAS 10417-94-4 | 302.451 |
| **Ricinoleic acid** | RIA | (Unsaturated) Fatty acids; (Keto-or Hydroxy) Fatty acids | 34297 | CAS 141-22-0 | 298.4608 |
| **13-Oxooctadecanoic acid** | O-STERA | (Keto- or Hydroxy) Fatty acids | na | Not available | 298.4608 |
| **3-Hydroxytetradecanoic acid** | 3H-MYRA | (Keto- or Hydroxy) Fatty acids | 61656 | CAS 3422-31-9 | 244.3703 |
| **1-heptadecanoyl-2-hydroxy-sn-glycero-3-phosphocholine** | 1-HD | Glycerophospholipids | | CAS 50930-23-9 | 509.6566 |
| **Bilirubin** | BR | (Tetrapyrroles and derivatives) Bilirubins | 54 | CAS 635-65-4 | 584.6621 |
| **Biliverdin** | BV | (Tetrapyrroles and derivatives) Bilirubins | 1008 | CAS 114-25-0 | 582.6463 |
| **Etiocholanolone glucuronide** | ECG | Testosterones (androgens /steroids) | 4484 | CAS 3602-09-3 | 466.5644 |
| **Cotinine** | COT | Pyridines | 1046 | CAS 486-56-6 | |
| **Myristic acid** | MYRA | (Saturated) Fatty acids | 806 | CAS 544-63-8 | 228.3709 |
| **Stearic acid** | STERA | (Saturated) Fatty acids | 827 | CAS 57-11-4 | 284.4772 |
| **1-oleoyl-2-hydroxy-sn-glycero-3-phospho-L-serine** | OL-GPS | Glycerophospholipids | Na | CAS 326589-90-6 | 522.596 |
| **L-(+)-Ergothioneine** | L-ERG | Amino acids | 3045 | CAS 497-30-3 | 229.299 |
| **20-Carboxy-leukotriene B4** | 20-CL | Eicosanoids | 6059 | CAS 80434-82-8 | 366.4486 |
| **2-Hydroxytetradecanoic acid** | 2H-MYRA | (Keto- or Hydroxy) fatty acids | 2261 | CAS 2507-55-3 | 244.3703 |
| **1-Palmitoyl-2-hydroxy-sn-glycero-3-phosphocholine (LysoPC(16:0))** | PA-GPC | Glycerophospholipids | 10382 | CAS 17364-16-8 | 495.6301 |
| **L-Acetylcarnitine** | AcCAR | Carnitines | 201 | CAS 3040-38-8 | 203.2356 |
| **6-Hydroxysphingosine** | 6H-Sa | Sphingolipids | Na | Not available | 315.498 |
| **L-Lysine** | L-LYS | Amino acids | 182 | CAS 56-87-1 | 146.1876 |
| **L-Glutamine** | L_GLU | Amino acids | 0000641 | CAS 56-85-9 | 146.1445 |
| **Sphinganine-1-phosphate** (C17 **base)** | Sa-1-P(17) | Phosphosphingolipids | Na | CAS 474923-29-0 | |

| **PROTEINS** | | | | | |
|---|---|---|---|---|---|
| **Protein name** | **Abbreviation** | | **Gene Code** | | |
| **Placental Growth Factor** | PIGF | | Gene: PGF | | |
| **Soluble fms-Like Tyrosine Kinase 1** | sFlt1 | | Gene: FLT1 | | |
| **Soluble Endoglin** | s-ENG | | Gene: ENG | | |

| **CLINICAL RISK FACTORS** | | | | | |
|---|---|---|---|---|---|
| **Risk Factor Abbreviation** | | | | | |
| **1st MAP (mean arterial pressure) BP at blood sampling visit** | map_1st | | | | |
| **2nd systolic BP at blood sampling visit (mm Hg, sphygmomanometer)** | 2nd_sbp | | | | |
| **2nd diastolic BP at blood sampling visit (mm Hg, sphygmomanometer)** | 2nd_dbp | | | | |
| **2nd MAP (mean arterial pressure) at blood sampling visit** | map_2nd | | | | |
| **Age of participants** | Age | | | | |
| **Family history of pre-eclamspsia (PE), i.e. participant's mother or sister had had PE** | fh_pet | | | | |
| **weight participant at blood sampling visit (kg)** | wgt | | | | |
| **BMI (Body Mass Index) at blood sampling visit** | bmi | | | | |
| **Waist circumference participant at blood sampling visit (cm)** | waist | | | | |
| **number of cigarettes per day in the 1st trimester (categories) 1-5 cigs / 6-10 cigs / >10 cigs** | cig_1st_trim_gp | | | | |
| **gestation age at blood sampling visit (in weeks)** | gest | | | | |
| **Random (non-fasting) glucose measured by glucometer at blood sampling visit (mmol/L)** | random_glucose | | | | |

### "Collection of the analytical methods"

To assay the proteins, ELISA was employed; These protein biomarkers were analysed as part of a large scale assessment of putative protein biomarkers within the SCOPE study using ELISA assays.[9]

To assay the metabolites an LC-MSMS assay was employed; which is summarized here:
1. The use of an extraction solvent / protein precipitation solvent that enables the extraction of the different types (classes) of metabolites. This extraction solvent composition, being a mixture of Methanol, Isopropanol and 200 mM Ammonium Acetate (aqueous) in a 10:9:1 ratio, which in turn is fortified with 0.05% 3,5-Di-tert-4-butyl-hydroxytoluene; in the remainder of this example this solvent is referred to as "crash".
2. The use of a dual (High Pressure) Liquid Chromatography (LC) system to enable the identification and quantification of the different classes of metabolites in a short analytical run. The chromatographic systems were developed so that these could be directly hyphenated to a mass spectrometric detection system. This dual chromatography system allows the separation of different metabolite types / classes and at the same time generate a detectable signal at the level of the mass spectrometer; a single chromatographic system, with short turn-around time, is not effective in robustly generating a detectable signal across all classes. The ability to 1) comprehensively analyse metabolites across different classes of metabolites, as relevant to a prognostic question, in 2) short turn-around time is important to generate data on sufficiently large sample sets (necessary to enable ly robust multivariable models) in economically viable time-and cost-frames.

Typical, but non-limiting examples of LC methods, are detailed below:

### Materials and reagents used in the dual separations.

Instrument: The LC-MS/MS platform used consisted of a 1260 Infinity LC system (Agilent Technologies, Waldbronn, Germany). The latter was coupled to an Agilent Triple Quadrupole 6460 mass spectrometer (QqQ-MS) equipped with a JetStream Electrospray lonisation source (Agilent Technologies, Santa Clara, CA, USA) (Cf. Example 6).

### RPLC:

The RPLC method is defined by the following settings /parameters:
- Injection volume: 7 µL
- Column oven temperature: 60°C
- Gradient RPLC was performed to resolve the hydrophobic metabolites using a binary solvent system:
   ∘ mobile phase A: Water:MeOH:NH₄OAc buffer 200mM at pH 4.5, (92:3:5)
   ∘ mobile phase B: MeOH:Acetonitrile:IPA: NH₄OAc 200mM at pH 4.5 (35:35:25:5)

A linear gradient program was applied: from 10% mobile phase B to 100 %mobile phase B in 10 minutes. using the following gradient - flow rate program:

**Table 5**

| Time (min) | %Mobile phase A | %Mobile phase B | Flow rate (ml/min) |
|---|---|---|---|
| 0.00 | 100% | 0% | 0.350 |
| 6.00 | 0% | 100% | 0.5 |
| 8.00 | 0% | 100% | 0.5 |
| 8.10 | 100% | 0% | 0.5 |
| 9.00 | 100% | 0% | 0.5 |
| 10.00 | 100% | 0% | 0.350 |

The efflux of the RPLC column was led directly to the QqQ-MS for mass spectrometric determination of the hydrophobic compounds of interest (see below).

### HILIC:

The HILIC method is defined by the following settings /parameters:
- Injection volume: 3 µL, whereby the injection plug was bracketed by 3µL ACN solvent plugs; a specific injector program was devised for this.
- Column oven temperature: 30°C
- Gradient HILIC was performed to resolve the hydrophobic metabolites using a binary solvent system:
o mobile phase A: 50 mM Ammonium formate (aqueous)
∘ mobile phase B: ACN
- A linear step gradient program was applied: from 10% mobile phase B to 100 %mobile phase B in 10 minutes. using the following gradient - flow rate program:
-

**Table 6**

| Time (min) | %Mobile phase A | %Mobile phase B | Flow rate |
|---|---|---|---|
| 0.00 | 12% | 88% | 0.45 mL/min |
| 1.10 | 20% | 80% | 0.45 mL/min |
| 2.0 | 20% | 80% | 0.45 mL/min |
| 2.10 | 30% | 70% | 0.45 mL/min |
| 3.00 | 30% | 70% | 0.45 mL/min |
| 3.10 | 40% | 60% | 0.45 mL/min |
| 4.00 | 40% | 60% | 0.45 mL/min |
| 6.00 | 50% | 50% | 0.45 mL/min |
| 7.20 | 50% | 50% | 0.45 mL/min |
| 7.21 | 12% | 88% | 0.45 mL/min |
| 10.00 | 12% | 88% | 0.45 mL/min |

The efflux of the RPLC column was led directly to the QqQ-MS for mass spectrometric determination of the hydrophobic compounds of interest (see below).

3. The use of a form of quantitative mass spectrometry, i.e., a tandem mass spectrometry system (MS/MS) operated in the Multiple Reaction Monitoring modus to allow for sensitive and specific analysis of metabolites. Hereto the samples are subjected to ionization under conditions to produce ionized forms of the metabolites of interest. Then the ionized metabolites are fragmented into metabolite derived fragment ions. The amounts of two specific fragments per metabolite are determined to identify and quantify the amounts of the originator metabolites in the sample (for further detail see below). Tandem mass spectroscopy was carried out under both positive and negative electrospray ionization and multiple reaction monitoring (MRM) mode. For each metabolite of interest, as relevant to preeclampsia, the following parameters were specifically established and optimized for each and every metabolite of interest and each SIL-IS available:
- appropriate precursor ion m/z, inclusive its preferred ionization mode (positive or negative),
- Product ion spectra under various collision voltage conditions (cf. induction of ion-molecule collisions under different energy regimens, leading to specific product ions) and selection of the most appropriate Quantifier and Qualifier product ions to be used for mass spectrometric identification and quantifications.
- Establishment of the reference Quantifier ion / Qualification ion ratios which to serve for specificity assessment.
- In addition, a number of assay specific instrument parameters were also optimized per compound of interest: quadrupole resolutions, dwell time, Fragmentor Voltage, Collision Energy and Cell Accelerator Voltage.

At the same time, instrument-specific parameters were optimised to maximally maintain compound integrity in the electrospray source and achieve sensitive and specific metabolite analysis; source temperature, sheath gas flow, drying gas flow and capillary voltage. The mass spectrometer used was an Agilent Triple Quadrupole 6460 mass spectrometer (QqQ-MS) equipped with an JetStream Electrospray lonisation source (Agilent Technologies, Santa Clara, CA, USA).

### RPLC-ESI-MS/MS

For the mass spectrometric method used for analyzing the hydrophobic metabolites of interest, the optimized electrospray ionization source parameters were as follows:

**Table 7**

| **Source Parameters** | **Positive mode** | **Negative mode** |
|---|---|---|
| Gas Temperature, °C | 200 | 200 |
| Gas flow, I/min | 13 | 13 |
| Nebuliser, psi | 40 | 40 |
| Sheath Gas Heater | 400 | 400 |
| Sheath Gas Flow | 11 | 11 |
| Capillary, V | 5000 | 3000 |
| V Charging | 300 | 300 |

### HILIC-MS/MS:

For the mass spectrometric method used for analyzing the hydrophilic metabolites of interest, the optimized electrospray ionization source parameters were as follows:

**Table 8**

| **Parameters** | **Positive mode** | **Negative mode** |
|---|---|---|
| Gas Temperature, °C | 200 | 200 |
| Gas flow, I/min | 13 | 13 |
| Nebuliser, psi | 40 | 40 |
| Sheath Gas Heater | 400 | 400 |
| Sheath Gas Flow | 12 | 12 |
| Capillary, V | 2500 | 3000 |
| V Charging | 300 | 300 |

4. For each metabolite a specific LC-MS/MS assay was developed for each of the targets of interest as well as for each of the SIL-IS; a particular LC-MS/MS assay entails a combination of above points 2&3.

5. The use of Stable Isotope Labelled Internal Standards (SIL-IS) to enable Stable Isotope dilution mass spectrometry, to achieve accurate and precise and accurate mass spectrometry-bases compound quantifications [11][12]. In brief, Stable Isotope Dilution Mass spectrometry is based on the principle that one fortifies all study samples with the same volume of a well-defined mixture of Stable Isotope Labelled Internal Standards at the start of the analytical process. These SIL-IS are typically identical to the endogenous compounds of interest, in this case metabolites, but have a number of specific atoms (typically Hydrogen 1H, Nitrogen 14N or Carbon 12C) within their molecular structure replaced by a stable, heavy isotope of the same element (typically Deuterium 2H, Nitrogen 15N, Carbon 13C). The SIL-IS are therefore chemically identical, but have a different "heavier" mass than their endogenous counterparts. Since they are chemically identical they will "experience" all experimental variability alike the endogenous metabolites of interest. F.i., any differential extraction yield between study samples during sample preparation will equally affect the metabolite of interest and its corresponding SIL-IS. Equally, the metabolite of interest and its corresponding SIL-IS will undergo the same chromatography and are typically equally sensitive to variability during mass spectrometric analysis. As a result, the ratio of any target metabolite signal and its according SIL-IS signal are largely invariant to experimental variability, hence the ratio "metabolite signal / corresponding SIL-IS signal" is directly related to the original concentration of the target in the blood sample. So, in the here disclosed methods, the preferred way to precisely quantify the amount of a metabolite of interest in a sample is by means of establishing the ratio of "the amount of the target metabolite quantifier ion / the amount of the quantifier ion of the corresponding SIL-IS". Whereby the here disclosed methods allow one to quantify a multitude of different target metabolites in a single analysis of the sample. Moreover, as all study samples are fortified with the same volume of a well-defined mixture of SIL-IS, one can readily compare the levels of the metabolites of interest across all study samples. The SIL-IS are exogenous compounds and thus not to be found in the native biological samples, so their spiked levels act as a common reference for all study samples.

6. The use of specific sample processing protocols for the simultaneous processing of large batches of biospecimens with high reproducibility and low technical variability. The details of a non-limiting example of a fit-for-purpose processing protocol is elaborated below.

As part of the methods, a dedicated biospecimen preparation methodology has been established, involving the fortification of the samples with a relevant SIL-IS mixture, and the use of the "crash", to extract the metabolites of interest. In terms of sample handling, minimizing any potential sources of error is critical to ensure reliable and precise results. The critical source of error in this methodology relates to the control of volumes; with the most critical volumes being the actual specimen volume being available for analysis, and, the volume of the SIL-IS added. Whereas experienced lab analysts will be able to prepare samples precisely, the use robot liquid handlers, is preferred when processing large numbers of biospecimens is warranted to eliminate human induced technical variability.

Here, as a non-limiting example, we elaborate a dedicated blood processing process, as relevant to methods in this application, using a liquid handling robot.

The robot was configured to enable 96 blood specimens in parallel, using the well-established 96 well format; this is also the analytical batch format adopted for the collection of methods herein.

### Instrument:

Agilent Bravo Automated Liquid Handling Platform (BRAVO, Model 16050-102, Agilent Technologies, Santa Clara, CA, USA), equipped with, a 96 LT disposable Tip Head, an orbital shaker station and a Peltier Thermal Station (Agilent Technologies). The Robot deck has 9 predefined stations, which can be used for 96 well-plates (specimens, reagents, pipette tip boxes) or functional stations (e.g. Peltier Station, etc)

### Experimental Protocol:

In brief the following steps were performed for each batch of 96 40µl aliquots; partial batches (n<96) are processed identically:
a) A 96-position plate, constituting an analytical batch, are retrieved from -80 °C storage, and put on BRAVO deck (orbital shaker) and vortexed for 20 minutes to assist thawing. When thawed, the vials are decapped.
b) In the meantime,
   a. a pre-prepared SIL-IS aliquot is retrieved from -20°C storage for thermal conditioning, the SIL-IS is then vortexed (1 minute) and-sonicated (5 minutes), and the appropriate volumes are then placed in one column (8 wells) of a PolyPropylene (PP) 96 well plate. The SIL-IS plate is then placed on the BRAVO deck (Peltier at 4°C).
   b. the pre-prepared proprietary [protein precipitation-metabolite extraction] formulation "crash" stock was taken from -20°C storage, stirred, and a PP 96 well plate filled with the appropriate volumes, the "crash" plate is then put on the robot deck.
c) The Bravo protocol is then initiated, the critical steps of this process are:
d) Draw up 140 µl of SIL-IS from the filled column of the SIL-IS plate and sequentially dispense 10µl in each of the specimen vials.
e) Fortified specimens will then be vortexed, on deck, for 5min at 1200rpm
f) Addition of the "crash" solution; this part of the sample preparation is performed in two separate steps
   a. First step: addition of 200µl "crash" solution, followed by on deck vortexing for 1 minute at 1200 rpm,
   b. Second step: addition of 140 µl "crash" solution followed by vortexing for 4 minutes at 1000 rpm
g) The specimen plate is then removed from the BRAVO robot and vortexed at 4°C for 10min followed by 2min sonication
h) Transfer of the specimen plate to the freezer, where they are kept at -20°C for 20 minutes to maximize protein precipitation.
i) After precipitation, the specimen vials are centrifuged at 4°C for 20min at a speed of 8000 rpm, then they are returned to the BRAVO robot; the specimen plate is put on the Peltier station at 4°C.
j) Splitting of the supernatant (i.e., the metabolite extract) in two different aliquots to enable the separate analysis of the Hydrophobic and Hydrophilic compounds. Hereto, 240µl of supernatant is aspirated and 120µl dispensed is twice, into separate PP 96-well plates (duplicate "specimen extract" plates).
k) The specimen extract plates are then dried by means of vacuum evaporation at 40°C for 60 minutes. Typically, 1 dried specimen extract plate is transferred to -80°C until further analysis, the other specimen extract plate is returned to the BRAVO robot for reconstitution, readying the extracted specimens for LC-MS/MS analysis

Whereas the above exemplified method was applied in the analysis of metabolites of interest relevant to preeclampsia; variations of the above methods are also employed as appropriate for the health outcome under consideration, and associated metabolites of interest. Non-limiting variations include
- Pre-treatment of the sample and further extraction of metabolites using solid phase extraction instead of precipitation method; robot protocols are in place.

7. The use of specific Quality Assurance procedures to avoid the introduction of experimental bias and to assure quality of the quantification of the metabolites of interest. These procedures define for instance: Analytical Batch Size and batch composition, Number and type of Quality Controls Samples, Criteria for acceptance of data read-outs, Operator blinding, designing sufficiently powered studies, selection of the appropriate study samples. To avoid experimental bias, specific methods are used to randomize the study samples; the lack of bias in sample order is then confirmed using the appropriate tests. Upon signal processing of the mass spectrometric data, specific post-analysis Quality methods are applied to assess per metabolite of interest, the data missing-ness rate across a clinical study, the presence of any (unwarranted) experimental bias, eventual signal drift, and the appropriateness of the chosen quantitative read-out (i.e., "metabolite quantifier ion / selected SIL-IS quantifier ion ratio"). Where necessary, alternative quantitative read-outs can be selected. Review of the analyte quantitation is routinely performed to quantify the stability and robustness. In the event, there are some inter-day batch drift observed, an appropriate correction can be applied. The appropriate quantification metric is established for each metabolite of interest. Following quality control and the selection of the most robust quantification metric, the imprecision of each metabolite quantification will be gauged, by calculating coefficients of Variance (%CV), using the available QC samples and/or replicate measurements.

8. The application of a set of selection criteria ("Quality stage-gate criteria") to determine which metabolites of interest can be progressed to biomarker performance analysis. Typically, but non-limiting, precision, specificity and missingness criteria are considered; alternatively imputation of missing values can also be considered [13]. Examples of typical precision limits are e.g., %CV <=15%, or <=20%CV or <=25%. The appropriate Quality Stage-Gate criteria are specifically established for each study of biospecimens,and can vary per metabolite of interest. This step will define which metabolites of interest can be progressed to the next steps and be used in multi-component prognostic / diagnostic test discovery; and will vary per study of biospecimens.

9. The use of methods to pre-process the quantification data of the metabolites of interest in view of performing biomarker analysis. Typically, these methods involve testing for the need for, and when warranted, application of data transformations (e.g., logarithmic transformation to obtain a normal distribution). Additional methods will test for the need for, and when warranted, the application of corrections of metabolite biomarker read-outs for e.g., patient- or sampling characteristics which modulate the metabolite read-outs independent from the prognostic or diagnostic question under investigation. Typically, but not limiting, the methods involve testing for-, and when warranted, establishing Multiples of the Median of the metabolite quantifications. Correction for such factors seeks to reduce the between-sample/-patient variance. In some instances, it might be relevant to dichotomize or categorize metabolite quantifications. The appropriate data transformations and appropriate corrections are specifically established for each study of biospecimens, and can vary per metabolite of interest.

### a) Log -Transformations:

For the selected metabolites of interest (Example 10), the quantitation read-outs were log-transformed before modelling; with exception for the data as presented in Results 4D.

### b) Multiple of the means - Transformations:

The dependencies of each analyte quantitation on common patient characteristics such as clinical center, overweight or gestational age at sampling. The analytes that do show a significant dependency (Mann-Whitney U test, Spearman correlation, Benjamini, Hochberg and Yekutieli, p<0.01) on these factors are normalised using a multiple-of-median (MoM) methodology. Multiple of the mean corrections were applied for the following metabolites of interest:

**Table 9**

| **Variable** | **Clinical variable** |
|---|---|
| TR | sample collection center |
| S-1-P | sample collection center |
| Sa-1-P | sample collection center |
| s-ENG | bmi at time of sampling (log) |
| 1-HD | bmi at time of sampling (log) |
| L-GLU | bmi at time of sampling (log) |
| 2-MGA_GLU | bmi at time of sampling (log) |
| L-ERG | maternal age (log) |
| DHA | maternal age (log) |
| PIGF | gestational age at time of sampling (log) |

All selected metabolites of interest quantified with the mass spectrometry platform were used as predictors for the computation of predictive models for the disease; for MoM-normalised variables both non-normalised and normalized were considered as predictors for the computation of predictive models for the disease.

### c) Dichotomizing of data: cotinine

Analytes that are exogenous such as cotinine, are not quantifiable in many patients. This lack of quantitation is usually associated with the lack of exposure. Therefore, the detectability of the molecule may be a better biomarker than the actual concentration of the molecule in blood. This is the case for cotinine whose presence in blood indicates the inhalation of cigarette smoke. The (relative) quantitation for cotinine was therefore binarized, samples without quantifiable cotinine and samples with low cotinine value were given a score of 0. Samples with high cotinine concentration were given a score of 1. The accuracy to predict whether a patient is reporting smoking was used to define an optimal cotinine relative concentration cut-off. This cut-off corresponds to a low density in the cotinine distribution indicating a robustness in the score.

10. The selection of a specific set of measurements which will be considered as input variables (or putative predictors) in multi-component prognostic / diagnostic test discovery. This set of variables will constitute the pre-processed metabolite quantification data as generated in the previous step, and can be augmented with relevant non-metabolite variables as available for the biospecimens under study. For instance, when one aims to create a multi-component risk stratification test (or prognostic test) to establish the probability an individual will get (or not get) a medical condition, these non-metabolite variable might constitute, for instance, but not limiting, relevant (clinical) risk factors as collected at time of sampling or as available in (medical) records, or the results of relevant, well-established clinical tests (e.g., glucose measurements) or quantification data of other types of relevant putative biomarkers molecules, e.g., proteins, DNA, RNA, etc as available for the same sample / originator individual. The selection of the appropriate set of non-metabolite variables are specifically established per study and per specific aim of the multi-component prognostic / diagnostic test discovery. Since the Applicants specifically set out to find prognostic tests for preeclampsia which can be easily administered by first line care providers and/or in healthcare systems with limited resources and are robust, only these clinical risk factors which are well established and easy to obtain were selected. For the same reason, the following types of variables were explicitly and deliberately excluded: patient data which is error prone, for instance but not limited to, detailed life-style variables or detailed medical history data, as well as variables which require for specialized staff to collect them, for instance but not limited to: Uteroplacental blood flow assessment by means of Doppler ultrasound (uterine artery doppler) and its derived parameters like pulsatility index or resistance index. Albeit these uteroplacental blood flow metrics associate with preeclampsia risk, they require for an expert sonographer and advanced ultrasound apparatus to be available. Since the metabolites of interest will typically be determined in a clinical laboratory setting, exemplary variables which can also be assessed in a clinical laboratory environment and which are widely reported to associate with preeclampsia risk were also considered: measurements on 3 specific proteins were selected as additional input variables; see earlier section "Variables of interest".

### Collection of data and data analysis methods used

### 1. Development of multivariable models:

The Applicants realised that the relevance of prognostic classifiers to predict the risk (or probability) an individual will develop a future health condition is largely determined by the extent the prognostic merits of such classifiers meets the clinical requirements as identified by health care providers and /or healthcare systems.

Yet, different clinical contexts might mandate for different requirements for a classifier. For instance, some clinical contexts will primarily focus on finding individuals at increased risk for a future health outcome. For these individuals with higher risk, care could be escalated, and /or prophylactic treatment could be prescribed. In other contexts, identifying individuals at decreased risk for the future outcome is more appropriate, e.g., to rationalize the use of certain care pathways. In some instances, both classifier questions will be of interest.

In addition, or alternatively, there might exist different sub-types (or grades) of the future health condition, for instance in terms of outcome severity. The requirements for classifiers might vary in function of outcome sub-type. In addition, or alternatively, there might exist sub-groups of individuals, which exhibit a different a-priori risk profile, and/or are more prone to the outcome or any of its subtypes. Once again, the clinical requirements for classifiers might vary for sub-groups of individuals. For this reason, the Applicants adopted, as part of the methods, the best subset regression method to create the space of all possible multivariable prediction models using one or more multivariable modelling techniques as relevant for the set of input of variables and outcomes (e.g., continuous or categorical) under study. By doing so, one has the possibility to address multiple classifier questions and/or classifier requirement questions at the same time; provisional to the study being sufficiently large and representative for the populations of interest and provisional to the prognostic information as carried by the input variables, and their combinations, do support the discovery of such classifiers. For instance, for a binary classifier for risk, PLS-DA, logistic regression, fractional polynomials could be applied. Depending on the question at hand and the study size available, the number of variables per model allowed can be varied; typically, but not limiting, the model space is construed of all combinations of e.g., 1 to 3, 1 to 4, 1 to 5 or 1 to 6 variables. In addition, methods are applied to ensure that only robust multivariate classifiers are considered, for instance, by applying cross-validation. A description of the methods used to create the comprehensive model space for the preeclampsia study, are elaborated below. Non-limiting example with description of the creation of a comprehensive prognostic model space as applied to identify multi-component risk stratification tests (or prognostic tests) to *predict risk of (all*/*preterm*/*term) preeclampsia in a pregnant woman prior to appearance of clinical symptoms of preeclampsia in the woman.*

For each possible combination of one to four predictor variables, a model is trained on complete cases using either logistic regression or partial least squares discriminant analysis (PLS-DA) to predict the outcome. For each of three outcomes models were computed, these are preeclampsia, term preeclampsia and preterm preeclampsia. For the outcomes term preeclampsia, the models were trained and tested on patients that did not develop preeclampsia (controls) versus the patients that developed preeclampsia and delivered at gestation age 37 weeks or higher. For the outcomes preterm preeclampsia, the models were trained and tested on patients that did not develop preeclampsia (controls) versus the patients that developed preeclampsia and delivered at gestation age below 37 weeks. This selection of patients was done to take into account the low prevalence of preeclampsia and the strong over-representation of preeclampsia patients in the dataset studied.

For each model, a range of statistics are derived to estimate its discriminative performance and its clinical relevance. These statistics are:
- AUC and (95% CI)
- sensitivity at PPV (95% CI)
- specificity at NPV (95% CI)
- sensitivity at 80% specificity (95% CI)
- sensitivity at 90% specificity (95% CI)
- specificity at 80% sensitivity (95% CI)
- specificity at 90% sensitivity (95% CI)
- number of controls (full cases)
- number of cases (full cases)
where CI stands for confidence interval.

### 2. Selection of robust prognostic combinations of variables / prognostic cores:

These statistics are computed for the test set over three iterations of a three-fold cross validation. The mean of each statistics over the three iterations was generated and used for model selection using an improvement.

Additionally, all the above statistics are also generated as well as for the complete sample sets. In this later case models were trained and evaluated on all controls and cases. To control for over-fitting only models wherefore the difference of the respective AUC metrics for the "Mean" (cf. 3-fold cross validation) and the "complete" are <=0.1 are retained within the model space.

Furthermore, models with a lower limit of the 95% confidence (ICI) for the AUC statistic lower than <=0.495 in either the "mean" or "complete" were also purged.

To achieve ly robust results, the selection of prognostic models / prognostic cores is typically based on an assessment of the lower limit of the 95% confidence (ICI) as calculated using the 3-fold cross validation derived "mean" statistic. Further to ensure that sparse models are selected, the improvement as calculated using the 3-fold cross validation derived "mean" statistic is also used as selection criteria.

For reporting purposes, the statistics as calculated for the complete data sets are used. Indeed, due to the conservative modelling and selection methods used, little to no over-fitting is observed.

### Notes:

- Whereas the comprehensive model space was established with either logistic regression or partial least squares discriminant analysis (PLS-DA), only the prognostic models / cores following the PLS-DA were considered for this application (for reporting simplicity). Whereas the statistics for individual prognostic models for logistic regression and PLS-DA may differ, both methods lead to approximately the selection of similar predictor combinations and underlying prognostic predictor cores are found to be largely the same.
- For the preeclampsia study considered in this application, the limitation to 4 variables / model is driven by 1) the desire to identify sparse prognostic cores 2) the restricted power for preterm PE, 3) the observation that within the preeclampsia data set exemplified here-in little additional "improvement" is achieved when considering more than 4 variables.

To identify relevant prognostic cores within the comprehensive prognostic model space created, the inventors established a logical rule to estimate the relevance of a model. It is important to evaluate whether each of its constituting input variables is contributing to the model discriminative performance. To estimate this, the minimum difference in performance between the model in question and its parent models is computed for each statistic under consideration. Parent models are all models 1) with fewer variables than the model in question and 2) whose variables are all variables of the model in question. The calculated differences are termed "improvement". For prognostic core selection purposes, only models with "improvement" above a given positive threshold are considered of relevance.

### 3. Model Space:

For the preeclampsia study considered in this application, models were computed for each possible combination of one to four predictor variables, for each of the 3 outcomes under investigation (see higher). Within the generated PLS-DA model space >256.000 models complied with the basic performance requirements as mentioned earlier.

The inventors then set out to discover the non-trivial core combinations of variables, with predictive merits for each of the performance targets as outlined in *Prognostic targets for pre-eclampsia risk stratification tests.* To do so, the model space was filtered using the lower limits of the 95% confidence intervals (ICI) as calculated using the 3-fold cross validation derived "mean" of the relevant statistic and the improvement as calculated using the 3-fold cross validation derived "mean" for the same statistic, for each performance target (AUC, Rule-in, Rule-out) for each of the PE-subtypes (All PE, Preterm PE and Term PE). Filtering thresholds were manually adjusted with a view to yielding a limited set (typically between 20 to 60) of core combinations of 2 to 4 variables (models). This was found sufficient to identify these variables which consistently contribute to performant models. These variables and/or specific combinations thereof, constitute prognostic cores with relevance to the prediction of preeclampsia risk. As elaborated elsewhere in this application, prognostic cores of variables may differ depending on the PE-subtype considered and/or whether generic prognostic performance (AUC), prediction of high-risk ("Rule-in"; Sensitivity at FPR- or PPV-thresholds) or prediction of low-risk ("Rule-out"; Specificity at FNR- or NPV-thresholds) are considered. For reporting, the data of the relevant statistic as achieved within the "complete data set", which from here on is abbreviated as "complete" when considering filtering thresholds.

### 4. Final prognostic models

Upon identification of the prognostic cores as relevant to a specific prognostic question, e.g., prognostic core relevant to "Rule-out" of "Preterm PE", (subsets of) the prognostic core variable were used to create the "final" prognostic models.

### Results

### 1. Introduction

As elaborated in this specification, the applicants conceptualized a process which has the potential to improve the detection rate (Sensitivity) at a pre-set PPV rule-in cut-off, by means of firstly establishing a rule-out model (using the entirety of methods as elaborated elsewhere in this application), secondly applying this model to identify these individuals at (a defined) low probability of developing the preeclampsia, and thirdly establishing a rule-in model (using the entirety of methods as elaborated elsewhere in this application), which maximizes the detection rate for future cases at a pre-set PPV threshold.

Here we demonstrate the validity of this concept using the following inputs:
- pre-test preeclampsia prevalence values as per the section" *Prognostic targets for pre-eclampsia risk stratification tests",* i.e.
   - prevalence all PE = 0.05
   - prevalence Preterm PE = 0.014
   - prevalence Term PE = 0.037
   - post-test PPV cut-offs as per Table 3
   - PPV _{All PE} = 0.133
   - PPV _{Preterm PE} = 0.071
   - PPV_{term PE} = 0.154
- Application of a (sparse) rule-out model (as developed by the), whereby the specificity (detection rate of future non-cases) at 10% FNR cut-off (10% of true future cases will be lost for rule-in classification) is used to rule-out a fraction of the test population.

### 2. Example 4A: All PE

In accordance with Example 1, the PPV criterion can be plotted in the ROC-space, whereby the criterion is dependent on the pre-test preeclampsia prevalence. This is illustrated in Figure 23 panel A. Within the above exemplary framework to find prognostic cores, the most performant (single step) rule-in multivariable model (bp + HVD3 + CR + ADMA) delivers a detection rate of 48%.

In view of applying a sequence of a rule-out model followed by a rule-in model, an exemplary (sparse) rule-out model was considered, which is exemplified in Figure 23 panel B.
- All PE Rule-out model: bp + sENG + 1-HD

Following the application of this rule-out model, 38.3 % of the future non-cases as well as 10% of future cases (cf. 10% FNR) are removed from the test population. As a result, the prevalence of pre-eclampsia in the remaining test population (P2) has gone up to 0.071 (from 0.05). This has the effect of changing the PPV cut-off line as seen in Figure 23 panel C. Within the residual population (P2), a novel set of predictive models was generated and assessed for their rule-in performance against the PPV criterion. The ROC curve as obtained with an exemplary (sparse) model is plotted in Figure D1 panel3.
- All PE Rule-in model: bp + PIGF + DC

Within test population P2, the latter model will deliver a sensitivity of 0.56 (56% detection rate). When correcting for the 10% future cases which were disregarded during the rule-out step, the overall detection rate is 51% (56% x 0.9).

This overall detection rate, following the sequential application of a rule-out model and a rule-in model, is better than the 48% obtained with the application of a single rule-in model as per Example B1.

Therefore, as elaborated in Example 2, combinations of rule-out prognostic cores and rule-in prognostic cores featuring multivariable combinations of specific sets of the variables considered in this application will deliver exceptional rule-in prognostic performance for all preeclampsia.

### 3. Example 4B: Preterm PE

In accordance with Example 1, the PPV criterion can be plotted in the ROC-space, whereby the criterion is dependent on the pre-test preeclampsia prevalence. This is illustrated in Figure 24 panel A. Within the above exemplary framework to find prognostic cores, the most performant (single step) rule-in multivariable model (PIGF + sENG + DLG + L-ERG) delivers a detection rate of 65%.

In view of applying a sequence of a rule-out model followed by a rule-in model, an exemplary (sparse) rule-out model was considered, which is exemplified in Figure 24 panel B.
- Preterm PE Rule-out model: sENG + DLG

Following the application of this rule-out model, 43.7 % of the future non-cases as well as 10% of future cases (cf. 10% FNR) are removed from the test population. As a result, the prevalence of preterm pre-eclampsia in the remaining test population (P2) has gone up to 0.023 (from 0.014). This has the effect of changing the PPV cut-off line as seen in Figure 24 panel C. Within the population (P2), a set of predictive models was generated and assessed for their rule-in performance against the PPV criterion. The ROC curve as obtained with an exemplary model is plotted in Figure 24 panel C.
- Preterm PE Rule-in model: PIGF + sENG + DLG + 2-HBA

Within test population P2, the latter model will deliver a sensitivity of 0.81 (81% detection rate). When correcting for the 10% future cases which were disregarded during the rule-out step, the overall detection rate is 73% (81% x 0.9).

This overall detection rate, following the sequential application of a rule-out model and a rule-in model, is better than the 65% obtained with the application of a single rule-in model as per Example B2.

Therefore, as elaborated in Example 2, combinations of rule-out prognostic cores and rule-in prognostic cores featuring multivariable combinations of specific sets of the variables considered in this application will deliver exceptional rule-in prognostic performance for preterm preeclampsia.

### 4. Example 4C: Term PE

In accordance with Example 1, the PPV criterion can be plotted in the ROC-space, whereby the criterion is dependent on the pre-test preeclampsia prevalence. This is illustrated in Figure 25 panel A. Within the above exemplary framework to find prognostic cores, the most performant (single step) rule-in multivariable model (bp + HVD3 + TR) delivers a detection rate of 35%, which is not meeting the pre-set minimum detection rate as set in Table 3.

In view of applying a sequence of a rule-out model followed by a rule-in model, an exemplary (sparse) rule-out model was considered, as per Example C3, which is exemplified in Figure 25 panel B.
- Term PE Rule-out model: bp + 1-HD

Following the application of this rule-out model, 38.2 % of the future non-cases as well as 10% of future cases (cf. 10% FNR) are removed from the test population. As a result, the prevalence of term pre-eclampsia in the remaining test population (P2) has gone up to 0.053 (from 0.037). This has the effect of changing the PPV cut-off line as seen in Figure 25 panel C. Within the population (P2), a novel set of predictive models was generated and assessed for their rule-in performance against the PPV criterion. The ROC curve as obtained with an exemplary model is plotted in Figure 25 panel C.
- Term PE Rule-in model: bp + 1-HD + NGM

Within test population P2, the latter model will deliver a sensitivity of 0.465 (46.5% detection rate). When correcting for the 10% future cases which were disregarded during the rule-out step, the overall detection rate is 42% (46.5% x 0.9).

This overall detection rate, following the sequential application of a rule-out model and a rule-in model, is better than the 35% obtained with the application of a single rule-in model as per Example B3, and does meet the preset minimal detection rate as put forward in Table 3. Therefore, as elaborated in Example 2, combinations of rule-out prognostic cores and rule-in prognostic cores featuring multivariable combinations of specific sets of the variables considered in this application will deliver exceptional rule-in prognostic performance for Term preeclampsia.

### 5. Example 4D: Prognostic performance maxisimation by a process of sequential classifiers

### Introduction

In Examples 4A, 4B, 4C the primary aim for the prognostic test, the test being was the achievement of exceptional rule-in performance.

With specific combinations of variables, as considered throughout this application, consistently showing exceptional prognostic relevance for preterm PE, the inventors explored whether it is conceivable to develop prognostic test that delivers simultaneously exceptional rule-in and rule out performance as expressed by the clinically relevant metrics PPV (rule-in) and NPV (rule-out).

As per Table 3, the following clinically relevant cut-offs were considered:
- Rule-in: the test shall classify a population into a high risk group, wherein the probability of developing preterm preeclampsia is >=1/14 or PPV >= 0.071
- Rule-out: the test shall classify a population into a low risk group, wherein the probability of developing preterm preeclampsia is =< 1/400 or NPV >= 0.9975.

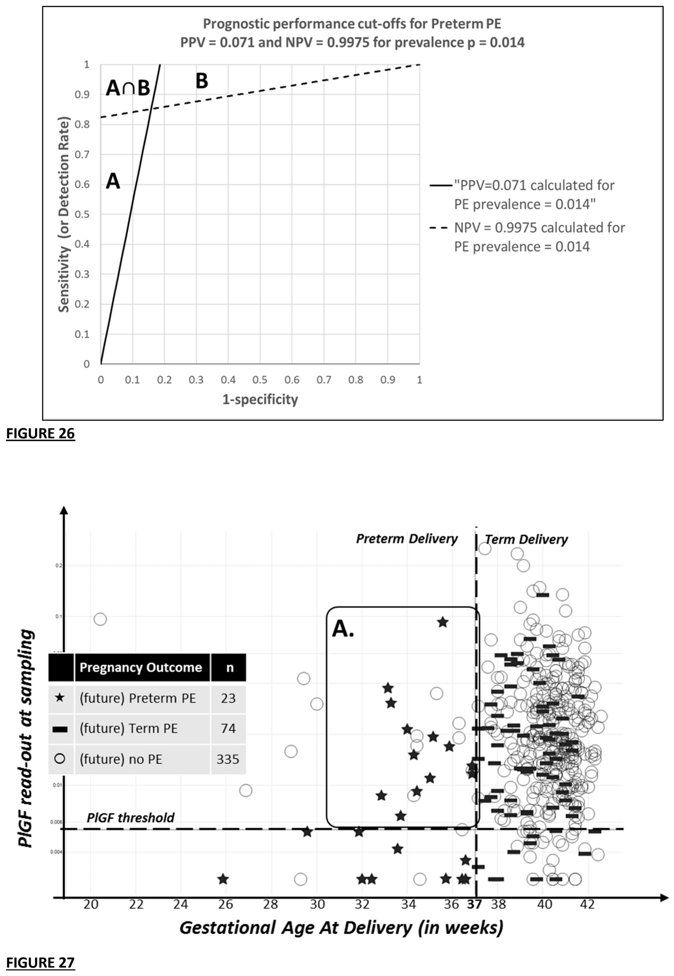

By accounting for the prevalence of (future) pre-eclampsia, i.e., p = 0.014 (cf. Table 3), the prognostic requirements for such test can be represented in the ROC space; The clinically relevant PPV- and NPV- thresholds as relevant to Preterm PE are illustrated in Figure 26.

### Preterm PE: sequential classifiers

The Applicants found that by application of specific sequences of "rule-in" and "rule-out" classifications, as elaborated theoretically in Example 3, using the variables of interest as considered within this application, prognostic tests with exceptional Rule-in AND rule-out performance metrics can be established.

In a first step, the inventors utilized the well-known predictive merits of PIGF to predict preterm pre-eclampsia, as published for the SCOPE study in Kenny et al [9]. In Figure 27, the PIGF levels as determined in maternal blood samples at ca. 15 weeks of pregnancy vs. the gestational age at delivery is given for all subjects of the study considered in this application. Please note, that at blood sampling all the women are considered healthy, and exhibited no ***clinical symptoms of pre-eclampsia, nor any clinical risk factors for preeclampsia.*** Women who delivered preterm, i.e., before 37 weeks of gestation, due to pre-eclampsia ("preterm preeclampsia") are represented by "star" symbols, women who experienced pre-eclampsia, but delivered at term, i.e., at or later than 37 weeks of gestation, are represented by "bar" symbols, women who delivered without experiencing preeclampsia are represented by "circle" symbols.

As can be seen from Figure 27, using PIGF levels at time of sampling will allow to classify the test population in 2 groups. The women with PIGF below the shown threshold will have a PPV > 0.071 to develop preterm PE, and are considered high risk. It is also clear that the group with PIGF levels higher (or equal) to the threshold constitutes >50% of the future preterm PE cases (Area "A").

For the remainder of this exemplification, the (future) term PE cases are not considered any further.

This results in the following Study-population Study-pop1, constituting

| | |
|---|---|
| - (Future) Preterm PE; | n =23 |
| - (Future) no PE; | n = 335 |

To appreciate the prognostic performance of the classifiers as established within this example E, one needs to correct the Study data as the Study is based on a Case-Controls study design and thus has an over-representation of (future) preterm PE cases compared to the natural disease prevalence p=0.014. Hereto any classification data is recalculated for a hypothetical population of 10,000 pregnancies, constituting

| | |
|---|---|
| - (Future) Preterm PE; | n = 140 |
| - (Future) no PE; | n = 9860 |

By applying the following scaling factors
- (future) Preterm PE: 140/23 = **6.087**
- (Future) no PE; 9860 / 335 = **29.43**

Study results can be interpreted for a population of 10,000 pregnancies, whilst accounting for the natural disease prevalence.

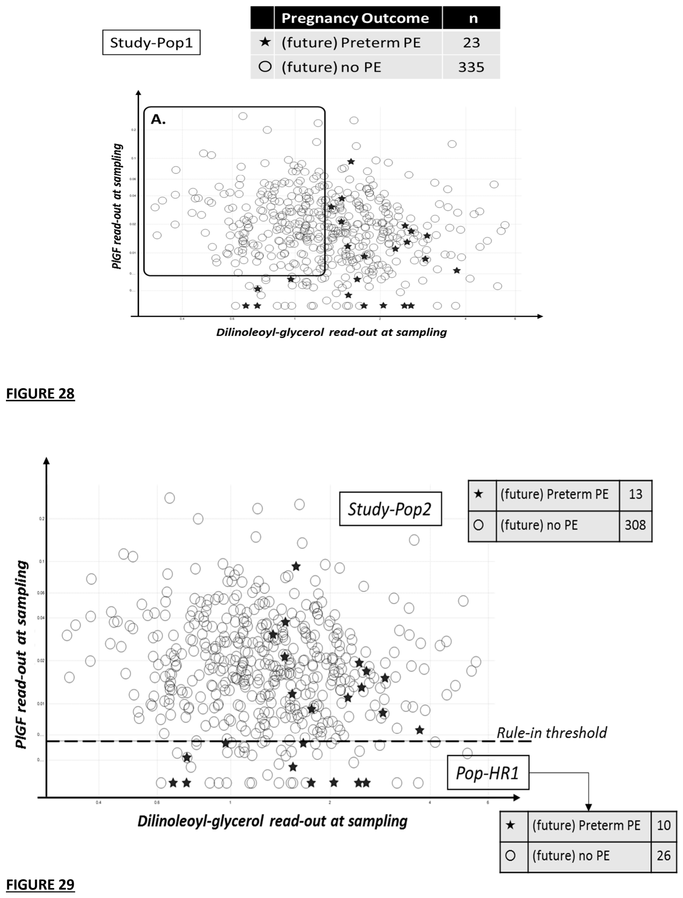

The inventors found that PIGF and DLG exhibit complementary classification potential, which becomes apparent when plotting both, as illustrated in Figure 28.

In view of the clinical relevant Rule-in / Rule Out classification targets under consideration, this prognostic complementarity can be utilized as follows:

### Step 1

Application of a Rule-in classifier using the PIGF cut-off, as exemplified in Figure 27. This classifier will segment the Study-Pop1 in a "Ruled-in" or High Risk population (Pop-HR1), as per Figure 29, and a new study population Study-Pop2.

Using the rule PIGF < 0.005445, the following classification is achieved:

**Table 10A**

| **Study - Pop 1** | | | |
|---|---|---|---|
| **Rule-in (Pop-HR1)** | | **Study - Pop2** | |
| Cases | controls | cases | controls |
| 10 | 26 | 13 | 309 |

**Table 10B**

| **population of 10000** | | | | | |
|---|---|---|---|---|---|
| **Rule in (Pop-HR1)** | | | **Study - Pop2** | | |
| Cases | controls | total | cases | controls | total |
| 60.87 | 765.254 | 826.12 | 79.1 | 9094.75 | 9173.9 |

When expressed as part of a total classifier, this 1^{st} step results following:

**Table 10C**

| **Classifier classes** | **Prognostic performance metrics - Total classifier** | | | |
|---|---|---|---|---|
| | Sn | Sp | PPV | NPV |
| **Rule-in (Pop-HR1)** | 0.43 | 0.92 | **0.074** | 0.926 |
| **Rule-out** | na | na | na | na |
| **Residual (Study-Pop2)** | 0.57 | 0.08 | 0.009 | 0.991 |

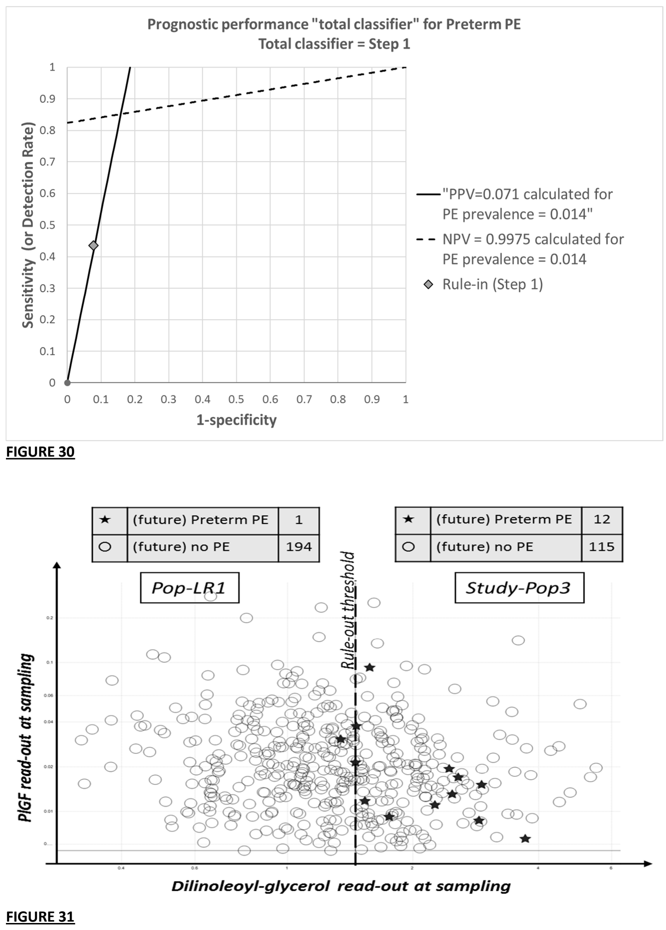

The PPV within the established Rule-in group, Pop-HR1, is compliant with the preset-PPV criterion (PPV >= 0.071). The overall detection rate of this single step classifier is 43% (Sn = 0.43), i.e., one will find 43% of all future preterm PE cases when applying just this PIGF based cut-off. It is of note that the non-ruled-in group (Study-Pop2) is not compliant with either the PPV or NPV criterion. This single step classifier can also be plotted in the ROC space, as illustrated in Figure 30; confirming compliance with the PPV-criterion (cf. Figure 27)

Because population Pop-HR1 is fully compliant with the pre-set PPV criterion, PoP-HR1 is considered fully classified and not considered further (removed from the Study). This means that the next step in classification will only consider Study-Pop2.

### Step 2

Following the removal of Pop-HR1 from the study-Pop1, the additive prognostic value of DLG becomes very apparent. A Rule-out classifier using the DLG cut-off, is exemplified in Figure 31 This classifier will segment the Study-Pop2 in a "Ruled-out" or Low Risk population (Pop-LR1), and a new study population Study-Pop3.

Using the rule DLG < 0.1454243, the following classification is achieved:

**Table 11A**

| **Study - Pop 2** | | | |
|---|---|---|---|
| **Rule out (Pop-LR1)** | | **Study-Pop3** | |
| Cases | controls | cases | controls |
| 1 | 194 | 12 | 115 |

**Table 11B**

| **population of (10000 - 826.12)** | | | | | |
|---|---|---|---|---|---|
| **Rule out (Pop-LR1)** | | | **Study-Pop3** | | |
| Cases | controls | total | cases | controls | total |
| 6.09 | 5709.97 | 5716.06 | 73 | 3384.78 | 3457.8 |

When added to the total classifier, this 2^{nd} step results in the following:

**Table 11C**

| **Classifier classes** | **Prognostic performance metrics - Total classifier** | | | |
|---|---|---|---|---|
| | Sn | Sp | PPV | NPV |
| **Rule-in (Pop-HR1)** | 0.43 | 0.92 | **0.074** | 0.926 |
| **Rule-out (Pop-LR1)** | 0.96 | 0.58 | 0.001 | **0.999** |
| **Residual (Study-Pop3)** | 0.52 | 0.66 | 0.021 | 0.979 |

Whereby the NPV within the established Rule-out group, Pop-LR1, is compliant with the preset-NPV criterion (NPV >= 0.9975).

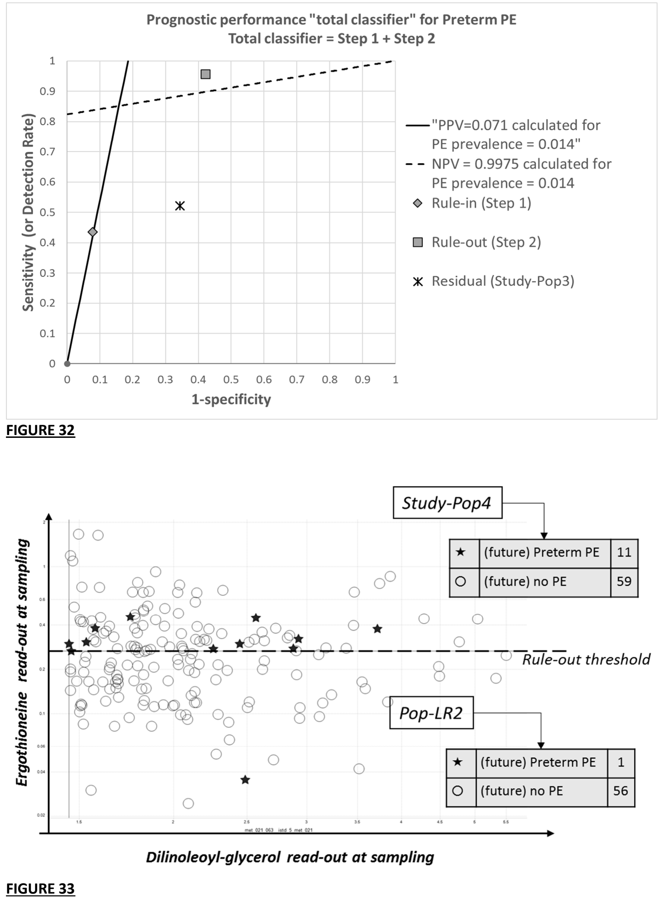

The overall detection rate of this dual step classifier is 43% (Sn = 0.43), i.e., one will find 43% of all future preterm PE cases when applying the PIGF based cut-off (step1; lower than). For any subject which has a PIGF higher than (or equal to) the PIGF cut-off, one will determine whether the subject has a value lower (ruled-out) than the DLG based cut-off, or higher than the DLG cut-off (becoming part of Study-pop3). 58% (Sp = 0.58) of the (future) non-PE cases will be stratified into the Pop-LR1 and will be considered low-risk. It is of note that the composition of Study-Pop3 is not compliant with either the PPV or NPV criterion. This two step classifier can also be plotted in the ROC space, as illustrated in Figure 32. As the total classifier considers the Rule-in classification and Rule-out classification separately, the total test classifier corresponds to 2 separate (Sn-Sp) pairs. One can see that the resulting Rule-in classification and Rule-out classification are compliant with either the pre-set PPV- or NPV- cut off. These subjects which are not classified to either be at high-risk or at low risk, constitute Study-Pop3. One can also plot the metrics of Study-pop3 in the ROC space; it is clear that this group (residual) is not compliant.

Because population Pop-LR1 is fully compliant with the pre-set NPV criterion, Pop-LR1 is also considered fully classified and not considered further (removed from the Study). This means that the next step in classification will only consider Study-Pop3.

### Step 3

Following the removal of Pop-LR1 from the study-Pop2, the inventors found that L-ERG can be used to stratify Study-Pop3 once more, to rule-out an additional group of subjects, and classify them as low-risk as well. Application of a Rule-out classifier using a L-ERG cut-off, is exemplified in Figure 33. This classifier will segment the Study-Pop3 into a "Ruled-out" or Low Risk population (Pop-LR2), and a new study population Study-Pop4.

Using the rule L-ERG< 0.266432, the following classification is achieved:

**Table 12A**

| **Study** - **Pop 3** | | | |
|---|---|---|---|
| Rule-out (Pop-LR2) | | Study-Pop4 | |
| Cases | controls | cases | controls |
| 1 | 56 | 11 | 59 |

**Table 12B**

| **population of (10000 - 826.12-5716.06)** | | | | | |
|---|---|---|---|---|---|
| Rule out (Pop-LR2) | | | Study-Pop4 | | |
| Cases | controls | total | cases | controls | total |
| 6.09 | 1648.24 | 1654.33 | 67 | 1736.54 | 1803.5 |

Within the new Ruled-out group, i.e., Pop-LR2, the preset NPV criterion is just missed (NPV= 0.996), yet when the 3^{rd} step is considered in combination with Pop-LR1, the cumulative rule out criterion is met.

In combination with the previous steps, the 3^{rd} step gives rise to the following combined Rule-in and Rule-out classification:

**Table 12C**

| **Classifier classes** | **Prognostic performance metrics - Total classifier** | | | |
|---|---|---|---|---|
| | Sn | Sp | PPV | NPV |
| **Rule-in (Pop-HR1)** | 0.43 | 0.92 | **0.074** | 0.926 |
| **Rule-out (Pop LR1 + LR2)** | 0.91 | 0.75 | 0.002 | **0.998** |
| **Residual (Study-Pop4)** | 0.48 | 0.82 | 0.037 | 0.963 |

The overall detection rate of this dual step classifier is 43% (Sn = 0.43), i.e., one will find 43% of all future preterm PE cases when applying the PIGF based cut-off (step1; lower than; Pop-HR1).

For any subject which has a PIGF higher than (or equal to) the PIGF cut-off, it is then determined whether the subject has a value lower than the DLG based cut-off; if yes, these subjects are considered low risk (step 2; Pop-LR1).

For any subject which has a PIGF blood value >= PIGF cut-off AND a DLG blood value >= Met_021_062 cut-off, one will determine whether the subject has a value lower than the L-ERG based cut-off (ruled-out; PopLR2), or higher than the L-ERG cut-off (becoming part of Study-pop4). When combining these two consecutive rule-out segmentation steps 75% (Sp = 0.75) of the (future) non-preterm PE cases will be stratified into the total Low risk Group. It is of note that the composition of Study-Pop4 is not compliant with either the PPV or NPV criterion.

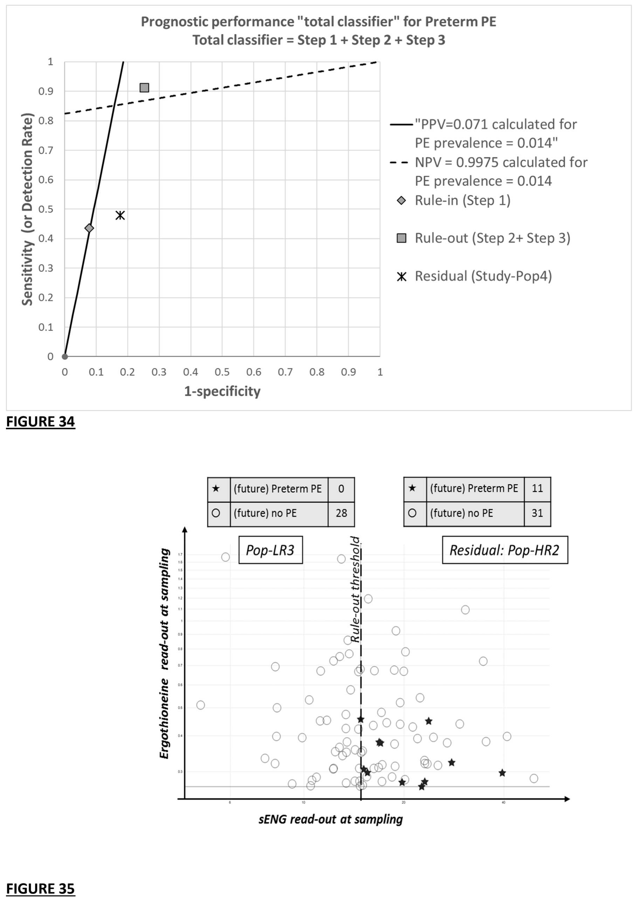

This three step classifier can also be plotted in the ROC space, as illustrated in Figure 34. As the total classifier considers the Rule-in classification and Rule-out classification separately, the total test classifier corresponds to two separate (Sn-Sp) pairs. One can see that the resulting Rule-in classification and Rule-out classification are compliant with either the pre-set PPV- or NPV- cut off. These subjects which are not classified to either be at high-risk or at low risk, constitute Study-Pop4. One can also plot the metrics of this "negative" test, corresponding to Study-pop4 in the ROC space. It is clear that this group (residual) is not compliant.

### Step 4

The inventors found that sENG can be used to stratify Study-Pop4 once more, to rule-out an additional group of subjects, and classify them as low-risk as well. Application of a Rule-out classifier using a sENG cut-off, is exemplified in Figure 35. This classifier will segment the Study-Pop4 into a "Ruled-out" or Low Risk population (Pop-LR2), and a residual study population.

Using the rule sENG< 14.8293, the following classification of Study-Pop 4 is achieved:

**Table 13A**

| **Study** - **Pop 3** | | | |
|---|---|---|---|
| Rule-out | | Residual = Rule-in | |
| Cases | controls | cases | controls |
| 0 | 28 | 11 | 31 |

**Table 13B**

| **population of (10000 - 826.12-5716.06-1648.24)** | | | | | |
|---|---|---|---|---|---|
| Rule out | | | Residual = rule -in | | |
| Cases | controls | total | cases | controls | total |
| 0.00 | 824.12 | 824.12 | 67 | 912.42 | 979.37 |

Within the new Ruled-out group, i.e., Pop-LR3, the preset NPV criterion is met once more (NPV=1), which will ensure that when the 4^{th} step is considered as part of the total classifier, the cumulative rule-out criterion is met.

When added to the total classifier, the 4^{th} step gives rise to the following combined Rule-in and Rule-out classification:

**Table 13C**

| **Classifier classes** | **Prognostic performance metrics - Total classifier** | | | |
|---|---|---|---|---|
| | Sn | Sp | PPV | NPV |
| **Rule-in (Pop-HR1)** | 0.43 | 0.92 | **0.074** | 0.926 |
| **Rule-out (Pop LR1** + **LR2+LR3)** | 0.91 | 0.83 | 0.001 | **0.999** |
| **Residual** | 0.48 | 0.91 | 0.068 | 0.932 |

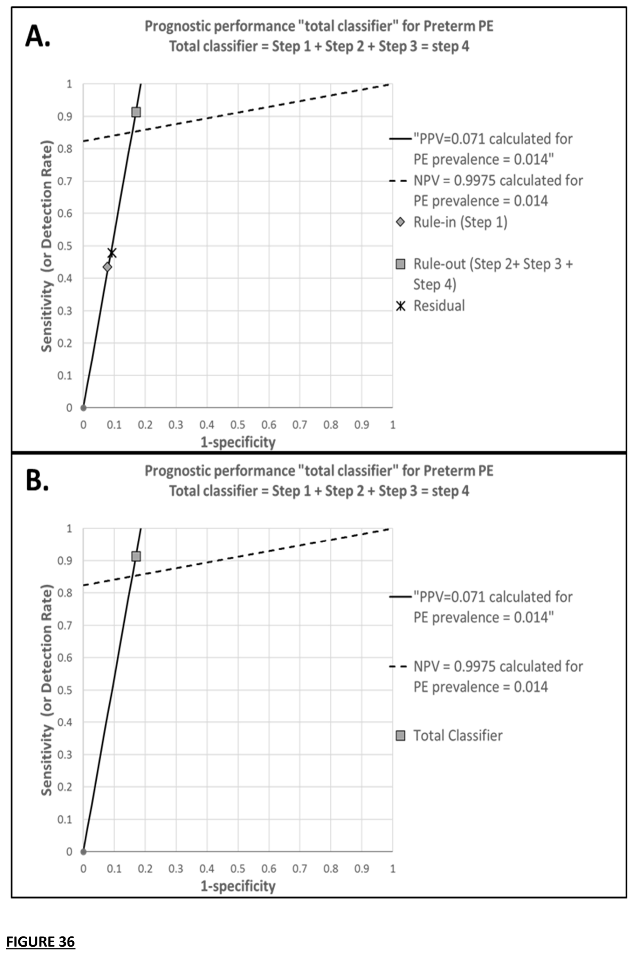

Interestingly, it can be observed that within the Residual population the risk to get preterm PE later in pregnancy is virtually compliant with the PPV threshold. This is also apparent when plotting the Total Classifier in the ROC space as illustrated in Figure 36 Panel A.

From Figure 36-panel A, one can appreciate that, following the application of the last Rule-out classification, the totality of "Rule-out" classifications is compliant with the NPV rule-out criterion (as intended), but it is also compliant with the pre-set PPV "Rule-in" criterion. In other words, as a result of this specific sequential application of individual classifiers, any subject which is not classified as Low-risk (as per the previous rule-out classifiers), is High-risk. This is also clear from the "residual" population, which also complies with the PPV criterion (similar to the first Rule-in group Pop-HR1). The iterative removal (or "ruling-out") of "low risk" subjects lead to a residual population highly enriched in (future) preterm PE cases. Therefore, one can consider a total High Risk group which constitutes: Pop-HR1 + Residual (or Pop-HR2).

By doing so, this "total classifier", as illustrated in Figure 36-panel B, will segment the original study Study-pop1 population in 2 groups; i.e.
- a high risk group which contains 91% of (future) preterm PE cases, and wherein any subject has a risk of >=1/14 (i.e., PPV >= 0.071) of effectively developing the disease later in pregnancy
- a low risk group which contains 83 % of (future) non-PE cases, and wherein any subject has a risk of =<1/400 or (NPV >= 0.9975) of effectively developing preeclampsia.

In addition to the fully exemplified Total classifier (Classifier A; in Table 14), additional Total classifiers constituting the ordered application of a set of 4 variables, were also found. Their key prognostic performance statistics are presented in Table 14.

**Table 14: Prognostic models for Preterm PE: Variables, in order of application in the Sequential classifier, and exemplary prognostic performance metrics for the preterm PE example elaborated in this application.**

| **Classifier** | **Variables - and order of their application to achieve** | **Prognostic Metrics Total Classifier** | | | |
|---|---|---|---|---|---|
| | | **Sn** | **Sp** | **PPV** | **NPV** |
| Classifier A | **PIGF, DLG, L-ERG, sENG** | **0.913** | **0.830** | **0.071** | **0.999** |
| Classifier B | PIGF, DLG, sENG, 1-HD | 0.957 | 0.76 | 0.054 | 0.999 |
| classifier C | PIGF, DLG, L-LEU, sENG | 0.87 | 0.81 | 0.061 | 0.998 |
| Classifier D | PIGF, DLG, sENG, L-LEU | 0.913 | 0.799 | 0.061 | 0.998 |
| Classifier E | PIGF, DLG, L-ISO, sENG | 0.957 | 0.742 | 0.050 | 0.999 |
| classifier F* | PIGF, DLG, (L-LEU +L-ISO)*, sENG | 0.957 | 0.748 | 0.051 | 0.999 |
| Classifier G | PIGF, DLG, L-ERG, L-LEU | 0.87 | 0.829 | 0.067 | 0.998 |
| Classifier H | PIGF, DLG, L-ERG, L-ISO | 0.87 | 0.817 | 0.063 | 0.998 |
| Classifier I* | PIGF, DLG, L-ERG, (L-LEU + L-ISO)* | 0.87 | 0.829 | 0.067 | 0.998 |

| | | | | | |
|---|---|---|---|---|---|
| * With L-LEU and L-ISO strongly correlating, and being closely related compounds, it was investigated whether the summed signal of L-LEU and L-ISO delivers similar prognostic performance compared to classifiers wherein L-LEU and L-ISO were used individually. The tabulated data confirmed that the summed signal of L-LEU and DC can be considered. | | | | | |

It will be apparent to reader that the following Total Classifiers constituting the ordered application of any, 2, 3 or 4 variable classifiers will deliver highly prognostic stratification for preterm PE in pregnant women early in pregnancy prior to showing any clinical symptoms of pre-eclampsia:
- PIGF, DLG, L-ERG and sENG
- PIGF, DLG, sENG, 1-HD
- PIGF, DLG, L-LEU, sENG
- PIGF, DLG, sENG, L-LEU
- PIGF, DLG, L-ISO, sENG
- PIGF, DLG, (L-LEU +L-ISO), sENG
- PIGF, DLG, L-ERG, L-LEU
- PIGF, DLG, L-ERG, L-ISO
- PIGF, DLG, L-ERG, (L-LEU + L-ISO)

Therefore, as elaborated in Example 3&4, sequential combinations of rule-out prognostic cores and rule-in prognostic cores featuring (multivariable) combinations of specific sets of the variables considered in this application will deliver exceptional total (Rule-in + Rule-out) prognostic performance for preterm preeclampsia.

### Hardware Implementation

It will be appreciated that in the context of the present invention dedicated computer hardware implementations including, but not limited to, application specific integrated circuits, programmable logic arrays and other hardware devices can likewise be constructed to implement the methods described herein. Applications that may include the apparatus and systems of various embodiments broadly include a variety of electronic and computer systems. Some embodiments implement functions in two or more specific interconnected hardware modules or devices with related control and data signals communicated between and through the modules, or as portions of an application-specific integrated circuit. Various modules can be provided to implement the invention as defined by the claims. Thus, the example system is applicable to software, firmware, and hardware implementations.

In accordance with various embodiments of the present disclosure, the methods described herein are intended for operation as software programs running on a computer processor. Furthermore, software implementations can include, but not limited to, distributed processing or component/object distributed processing, parallel processing, or virtual machine processing can also be constructed to implement the methods described herein.

The present disclosure contemplates a machine readable medium containing instructions so that a device connected to the communications network, other network, or both, can send or receive voice, video or data, and to communicate over the communications network, other network, or both, using the instructions. The instructions may further be transmitted or received over the communications network, other network, or both, via the network interface device.

For example, the data obtained in the various examples described above relating to a particular subject can be uploaded to a computing apparatus 'on-site' and the information processed by a processor. The processor can then output a value to a screen indicative of a detection, or a prediction of risk, of the health condition in the subject based on said model M. It is envisaged that this could be implemented in the form of a standalone personal computer or a handheld device or a smart phone/communication device. Alternatively, the data can be uploaded to a cloud or virtual based server where the data is processed in accordance with the invention. The processed data can then be sent or used in any appropriate way.

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

### REFERENCES

1 Thomas G, Kenny LC, Baker PN, et al. A novel method for interrogating receiver operating characteristic curves for assessing prognostic tests. Diagnostic Progn Res 2017;1:17. doi:10.1186/s41512-017-0017-y
2 Moons KGM, Altman DG, Vergouwe Y, et al. Prognosis and prognostic research: application and impact of prognostic models in clinical practice. BMJ 2009;338:b606-b606. doi:10.1136/bmj.b606
3 Liao Y, McGee DL, Cooper RS, et al. How generalizable are coronary risk prediction models? Comparison of Framingham and two national cohorts. Am Heart J 1999;137:837-45. doi:10.1016/S0002-8703(99)70407-2
4 Lucas A, Armbruster B. The cost-effectiveness of expanded HIV screening in the United States. Aids 2013;27:795-801. doi: 10.1097/QAD.0b013e32835c549
5 Dondorp W, De Wert G, Bombard Y, et al. Non-invasive prenatal testing for aneuploidy and beyond: Challenges of responsible innovation in prenatal screening. Eur J Hum Genet 2015;23:1438-50. doi:10.1038/ejhg.2015.57
6 World Health Organisation. Prevention of cardiovascular disease: guidelines for assessment and management of cardiovascular risk. Geneva, Switzerland: : World Health Organisation 2007.
7 Royston P, Moons KGM, Altman DG, et al. Prognosis and prognostic research: Developing a prognostic model. BMJ 2009;338:b604. doi:10.1136/bmj.b604
8 North RA, Mccowan LME, Dekker GA, et al. Clinical risk prediction for pre-eclampsia in nulliparous women: development of model in international prospective cohort. BMJ 2011;342:d1875. doi:10.1136/bmj.d1875\rbmj.d1875 [pii]
9 Kenny LC, Black MA, Poston L, et al. Early Pregnancy Prediction of Preeclampsia in Nulliparous Women, Combining Clinical Risk and Biomarkers: The Screening for Pregnancy Endpoints (SCOPE) International Cohort Study. Hypertension 2014;64:644-52. doi:10.1161/HYPERTENSIONAHA.114.03578
10 Rolnik DL, Wright D, Poon LC, et al. ASPRE trial: performance of screening for preterm pre-eclampsia. Ultrasound Obstet Gynecol Published Online First: 25 July 2017. doi:10.1002/uog.18816
11 Pitt JJ. Principles and Applications of Liquid Chromatography- Mass Spectrometry in Clinical Biochemistry. Clin Biochem Rev 2009;30:19-34.
*12* CLSI. Liquid Chromatography - Mass Spectromtry Methods; Approved Guideline CLSI document 62-A. October 20. Wayne, PA: : Clinical and Laboratory Standards Institute 2014.
13 Hrydziuszko O, Viant MR. Missing values in mass spectrometry based metabolomics: an undervalued step in the data processing pipeline. Metabolomics 2012;8:161-74. doi:10.1007/s11306-011-0366-4

### Clauses/Statements

1. A computer implemented method of generating a model M for detecting or predicting risk of a health condition in a subject, the method comprising the steps of:
   providing a population of test subjects and measurement data for a plurality (n) of variables for each of the test subjects selected from biometric, life-style and/or physiological characteristics;
   providing a first model M1 configured to predict the presence or risk of the health condition in the population of test subjects comprising 1 to n variables, wherein the model M1 is configured to segregate the population of test subjects into first generation population subsets A and B in which the subjects in the first generation population subset A have a probability selected from one of: a probability to have/develop the health condition lower than or equal to a predefined rule-out cut-off probability; or a probability to have/develop the health condition higher than or equal to a predefined rule-in cut-off probability;
   segmenting the population of subjects based on the first model M1 into first generation population subsets A and B;
   when the first generation population subset B comprises a sufficient number of subjects, providing a second model M2 configured to predict the presence or risk of the health condition in the first generation population subset B, the model comprising 1 to n variables, wherein the model M2 is configured to segregate the first generation population subset B into second generation population subsets A and B in which the subjects in the second generation population subset A have a probability selected from the other of: a probability to have/develop the health condition lower than or equal to a predefined rule-out cut-off probability; or a probability to have/develop the health condition higher than or equal to a predefined rule-in cut-off probability;
   segmenting the first generation population subset B based on the second model M2 into second generation population subset A and B; and
   wherein the model M comprises the first model M1 and the second model M2; and
   outputting a value indicative of a detection, or a prediction of risk, of the health condition in the subject based on said model M.
2. A computer implemented method according to Clause 1 in which the model M2 is configured to segregate the first generation population subset B into second generation population subsets A and B in which the subjects in the second generation population subset A have a probability selected from the other of: a probability to have/develop the health condition lower than or equal to a predefined NPV cut-off; or a probability to have/develop the health condition higher than or equal to a predefined PPV cut-off.
3. A computer implemented method according to Clause 1 in which the model M1 is configured to segregate the population of test subjects into first generation population subsets A and B in which the subjects in the first generation population subset A have a probability to have/develop the health condition lower than or equal to a predefined rule-out cut-off probability; wherein the model M2 is configured to segregate the first generation population subset B into second generation population subsets A and B in which the subjects the second generation population subset A have a probability to have/develop the health condition higher than or equal to a predefined rule-in cut-off probability which is optionally a PPV cut-off.
4. A computer implemented method according to Clause 1 in which the model M1 is configured to segregate the population of test subjects into first generation population subsets A and B in which the subjects in the first generation population subset A have a probability to have/develop the health condition higher than or equal to a predefined rule-in cut-off probability; wherein the model M2 is configured to segregate the first generation population subset B into second generation population subsets A and B in which the subjects the second generation population subset A have a probability to have/develop the health condition lower than or equal to a predefined rule-out probability which is optionally a NPV cut-off.
5. A computer implemented method according to any preceding Clause, including an additional segmentation step comprising:
   when the second generation population subset B comprises a sufficient number of subjects, providing a third model M3 configured to predict the presence or risk of the health condition in the second generation population subset B, the model comprising 1 to n variables, wherein the model M3 is configured to segregate the second generation population subset B into third generation population subsets A and B such that the subjects in the third generation population subset A has a probability selected from one of: a probability to have/develop the health condition lower than or equal to a predefined rule-out NPV cut-off; or a probability to have/develop the health condition higher than or equal to a predefined rule-in PPV cut-off; and
   segmenting the other of the second generation population subsets based on the third model M3 into third generation population subsets A and B.
6. A computer implemented method according to Clause 5 in which:
   the first model M1 is configured to segment the population of test subjects such that the subjects the first generation population subset A have a probability to have/develop the health condition higher than a predefined rule-in cut-off probability;
   the second model M2 is configured to segment the first generation population subset B such that the subjects in the second generation population subset A have a probability to have/develop the (health) condition lower than a predefined rule-out cut-off probability; and
   the third model M3 is configured to segment the second generation population subset B such that the subjects in at least one of the third generation population subsets have a probability to have/develop the health condition higher than a predefined rule-in PPV cut-off.
7. A computer implemented method according to Clause 5 in which:
   the first model M1 is configured to segment the population of test subjects such that the subjects the first generation population subset A have a probability to have/develop the health condition lower than a predefined rule-out cut-off probability;
   the second model M2 is configured to segment the first generation population subset B such that the subjects in the second generation population subset A have a probability to have/develop the health condition higher than a predefined rule-in cut-off probability; and
   the third model M3 is configured to segment the second generation population subset B such that the subjects in at least one of the third generation population subsets have a probability to have/develop the health condition lower than a predefined rule-out NPV cut-off.
8. A computer implemented method according to Clause 6 in which:
   the first model M1 is configured to segment the population of test subjects such that the subjects the first generation population subset A have a probability to have/develop the health condition higher than a predefined PPV rule-in cut-off;
   the second model M2 is configured to segment the first generation population subset B such that the subjects in the second generation population subset A have a probability to have/develop the health condition lower than a predefined rule-out cut-off probability; and
   the third model M3 is configured to segment the second generation population subset B such that the subjects in at least one of the third generation population subsets have a probability to have/develop the health condition higher than a predefined rule-in PPV cut-off.
9. A computer implemented method according to Clause 4 in which:
   the first model M1 is configured to segment the population of test subjects such that the subjects the first generation population subset A have a probability to have/develop the health condition lower than a predefined rule-out NPV cut-off;
   the second model M2 is configured to segment the first generation population subset B such that the subjects in the second generation population subset A have a probability to have/develop the health condition higher than a predefined rule-in cut-off probability; and
   the third model M3 is configured to segment the second generation population subset B such that the subjects in at least one of the third generation population subsets have a probability to have/develop the health condition lower than a predefined rule-out NPV cut-off.
10. A computer implemented method according to Clause 1, including one or more additional segmentation steps in which each segmentation step comprises:
   when the n^{th} generation population subset B comprises a sufficient number of subjects, providing a (n+1)^{th} model Mⁿ⁺¹ configured to predict the presence or risk of the health condition in the n^{th} generation population subset B, the model comprising 1 to n variables, wherein the model Mⁿ⁺¹ is configured to segregate the n^{th} generation population subset B into (n+1)^{th} generation population subset A and B such that the subjects in the (n+1)^{th} generation population subset A have a probability selected from one of: a probability to have/develop the health condition lower than or equal to a predefined rule-out NPV cut-off; or a probability to have/develop the health condition higher than or equal to a predefined rule-in PPV cut-off; and; and
   segmenting the n^{th} generation population subset B based on the (n+1)^{th} multivariable model into (n+1)^{th} generation population subsets A and B.
11. A computer implemented method according to Clause 10, in which a final segregation step employs a final model configured to predict the presence or risk of the health condition in the penultimate generation population subset B, the model comprising 1 to n variables, wherein the final model is configured to segregate the penultimate generation population subset B into final generation population subset A and B such that the subjects in the final generation population subset A have a probability selected from one of: a probability to have/develop the health condition lower than or equal to a predefined rule-out NPV cut-off; or a probability to have/develop the health condition higher than or equal to a predefined rule-in PPV cut-off;
12. A computer implemented method according to Clause 1, including one or more additional segmentation steps in which each segmentation step comprises:
   (a) when the nth generation population subset B comprises a sufficient number of subjects, generating a (n+1)^{th} model Mⁿ⁺¹ comprising 1 to n variables, configured to predict the presence or risk of developing a health condition in the n^{th} generation population subset B, wherein the model Mⁿ⁺¹ is configured to segregate the nth generation population subset B into (n+1)^{th} generation population subset A and B whereby
      when the subjects in the (n+1)^{th} generation population subset A are added to the combined earlier generation population rule-in subsets A, the composite rule-in population has a probability higher than or equal to a predefined rule-in PPV cut-off to have/develop the condition; or
      when the subjects in the (n+1)^{th} generation population subset A are added to the combined earlier generation population rule- out subsets A, the composite rule-out population have a probability lower than or equal to a predefined rule-out NPV cut-off to have/develop the condition; and.
   (b) segmenting the nth generation population subset B based on the (n+1)^{th} multivariable model into (n+1)^{th} generation population subsets A and B.
13. A computer implemented method according to Clause 10, 11 or 12 in which successive segmentation steps are repeated until a population subset is generated that cannot be further segmented.
14. A computer implemented method according to any of clauses 1 to 13, whereby the rule-in cut-off probability is selected from, unless stated otherwise, a Positive Predictive Value PPV-, specificity- (False Positive Rate) or positive likelihood ratio- cut-off.
15. A computer implemented method according to clauses 1 to 13, whereby the rule-out cut-off probability is selected from, unless stated otherwise, NPV-, Sensitivity- (False Negative Rate) or negative likelihood ratio- cut-off.
16. A computer implemented method according to any preceding clause, wherein when one of the population subsets does not comprise a sufficient number of subjects, the first segregation step is repeated using an alternative model.
17. A computer implemented method of predicting the probability of the presence or risk of a health condition in a subject using the model M generated according to any of Clauses 1 to 16, the method comprising the steps of:
   using the first model M1 to determine whether the subject has a probability to have/develop the health condition selected from one of: a probability lower than or equal to a first predefined rule-out cut-off probability; and a probability higher than or equal to a first predefined rule-in cut-off probability; and
   optionally, using the second model M2 to determine whether the subject has a probability to have/develop the health condition selected from the other of: a probability lower than or equal to a second predefined rule-out cut-off probability; or a probability higher than or equal to a second predefined rule-in cut-off probability.
18. A computer implemented method according to Clause 17, wherein when the first model M1 determines that the subject has a probability to have/develop the health condition that is higher than a first predefined rule-out cut-off probability, the second model M2 is employed to determine whether the subject has probability to have/develop the health condition that is higher than or equal to a second predefined rule-in PPV cut-off; wherein when the second model M2 determines that the subject has a probability to have/develop the health condition that is higher than or equal to a second predefined rule-in PPV cut-off, the subject is determined to have a high probability to have/develop the health condition;
19. A computer implemented method according to Clause 17, wherein when the first model M1 determines that the subject has a probability to develop the health condition that is lower than a first predefined rule-in cut-off probability, the second model M2 is employed to determine whether the subject has probability to have/develop the health condition that is lower than or equal to a second predefined rule-out NPV cut-off; wherein when the second model M2 determines that the subject has a probability to have/develop the health condition that is lower than or equal to a second predefined rule-out NPV cut-off, the subject is determined to have a low probability to have/develop the health condition.
20. A computer implemented method according to Clause 17, wherein when the first model M1 determines that the subject has a probability to develop the health condition that is lower than or equal to a first predefined rule-out NPV cut-off, the subject is determined to have a low probability to have/develop the health condition;
21. A computer implemented method according to Clause 17, wherein when the first model M1 determines that the subject has a probability to have/develop the health condition that is higher than or equal a first predefined rule-in PPV cut-off, the subject is determined to have a high probability to have/develop the health condition;
22. A computer implemented method of predicting the probability of the presence or risk of a health condition in a subject using the model M generated according to any of Clauses 5 to 9, the method comprising the steps of:
   using the first model M1 to determine whether the subject has a probability to develop the health condition selected from one of: a probability lower than or equal to a first predefined rule-out cut-off probability; and a probability higher than or equal to a first predefined rule-in cut-off probability; and
   using the second model M2 to determine whether the subject has a probability to develop the health condition selected from the other of: a probability lower than or equal to a second predefined rule-out cut-off probability; or a probability higher than or equal to a second predefined rule-in cut-off probability; and
   using the third model M3 to determine whether the subject has a probability to develop the health condition selected from one of: a probability lower than or equal to a third predefined rule-out cut-off probability; or a probability higher than or equal to a third predefined rule-in cut-off probability.
23. A computer implemented method according to Clause 22, comprising the steps of:
   using the first model M1 to determine whether the subject has a probability to have/develop the health condition higher than or equal to a first predefined rule-in PPV cut-off;
   wherein when the first model M1 determines that the subject has a probability to develop the health condition that is lower than a first predefined rule-in PPV cut-off, the second model is employed to determine whether the subject has probability to have/develop the health condition that is lower than or equal to a second predefined rule-out NPV cut-off;
   wherein when the second model M2 determines that the subject has a probability to have/develop the health condition that is higher than the second predefined rule-out NPV cut-off, the third model M3 is employed to determine whether the subject has probability to have/develop the health condition that is lower than or equal to a third predefined rule-out NPV cut-off;
   wherein when the third model determines that the subject has a probability to develop the health condition that is higher than the third predefined rule-out NPV cut-off probability, the subject is determined to have a high probability to have/develop the health condition.
24. A computer implemented method according to any preceding Clause, in which the physiological characteristics include metabolite levels and protein levels.
25. A computer implemented method according to any preceding Clause, in which the health condition is preeclampsia.
26. A computer implemented system for generating a model M for detecting or predicting risk of a health condition in a subject, comprising:
   an input device;
   a processor;
   a memory;
   an output device;
   said processor operatively coupled to the input device, the memory and the output device, said processor configured with:
   a module or means for providing a population of test subjects and measurement data for a plurality (n) of variables for each of the test subjects selected from biometric, life-style and/or physiological characteristics;
   a module or means for providing a first model M1 configured to predict the presence or risk of the health condition in the population of test subjects comprising 1 to n variables, wherein the model M1 is configured to segregate the population of test subjects into first generation population subsets A and B in which the subjects in the first generation population subset A have a probability selected from one of: a probability to have/develop the health condition lower than or equal to a predefined rule-out cut-off probability; or a probability to have/develop the health condition higher than or equal to a predefined rule-in cut-off probability;
   a module or means for segmenting the population of subjects based on the first model M1 into first generation population subsets A and B;
   optionally, a module or means for determining when the first generation population subset B comprises a sufficient number of subjects;
   a module or means for providing a second model M2 configured to predict the presence or risk of the health condition in the first generation population subset B, the model comprising 1 to n variables, wherein the model M2 is configured to segregate the first generation population subset B into second generation population subsets A and B in which the subjects in the second generation population subset A have a probability selected from the other of: a probability to have/develop the health condition lower than or equal to a predefined rule-out cut-off probability; or a probability to have/develop the health condition higher than or equal to a predefined rule-in cut-off probability;
   a module or means for segmenting the first generation population subset B based on the second model M2 into second generation population subset A and B;
   wherein the model M comprises the first model M1 and the second model M2; and
   means for outputting a value indicative of a detection, or a prediction of risk, of the health condition in the subject based on said model M.
27. A computer program product stored in a non-transitory storage medium, said storage medium operatively coupled to a processor and said computer program product causing the processor to generate a model M for detecting or predicting risk of a health condition in a subject, the processor configured to implement the steps of:
   providing a population of test subjects and measurement data for a plurality (n) of variables for each of the test subjects selected from biometric, life-style and/or physiological characteristics;
   providing a first model M1 configured to predict the presence or risk of the health condition in the population of test subjects comprising 1 to n variables, wherein the model M1 is configured to segregate the population of test subjects into first generation population subsets A and B in which the subjects in the first generation population subset A have a probability selected from one of: a probability to have/develop the health condition lower than or equal to a predefined rule-out cut-off probability; or a probability to have/develop the health condition higher than or equal to a predefined rule-in cut-off probability;
   segmenting the population of subjects based on the first model M1 into first generation population subsets A and B;
   when the first generation population subset B comprises a sufficient number of subjects, providing a second model M2 configured to predict the presence or risk of the health condition in the first generation population subset B, the model comprising 1 to n variables, wherein the model M2 is configured to segregate the first generation population subset B into second generation population subsets A and B in which the subjects in the second generation population subset A have a probability selected from the other of: a probability to have/develop the health condition lower than or equal to a predefined rule-out cut-off probability; or a probability to have/develop the health condition higher than or equal to a predefined rule-in cut-off probability;
   segmenting the first generation population subset B based on the second model M2 into second generation population subset A and B; and
   wherein the model M comprises the first model M1 and the second model M2; and outputting a value indicative of a detection, or a prediction of risk, of the health condition in the subject based on said model M.

## Claims

1. A computer implemented method of generating a model M to detect or predict the risk of a health condition in a subject, the method comprising the steps of:
providing a population of test subjects and measurement data for a plurality (n) of variables for each of the test subjects selected from biometric, life-style and/or physiological characteristics;
providing a first model M1 configured to predict the presence or risk of the health condition in the population of test subjects comprising 1 to n variables, wherein the model M1 is configured to segregate the population of test subjects into first generation population subsets A and B in which the subjects in the first generation population subset A have a probability selected from one of: a probability to have/develop the health condition lower than or equal to a predefined rule-out cut-off probability; or a probability to have/develop the health condition higher than or equal to a predefined rule-in cut-off probability; **characterised by** the steps of:
segmenting the population of subjects based on the first model M1 into first generation population subsets A and B; when the first generation population subset B comprises a sufficient number of subjects,
providing a second model M2 configured to predict the presence or risk of the health condition in the first generation population subset B, the model comprising 1 to n variables, wherein the model M2 is configured to segregate the first generation population subset B into second generation population subsets A and B in which the subjects in the second generation population subset A have a probability selected from the other of: a probability to have/develop the health condition lower than or equal to a predefined rule-out cut-off probability; or a probability to have/develop the health condition higher than or equal to a predefined rule-in cut-off probability; wherein, when one of the population subsets does not comprise a sufficient number of subjects, the previous segregation step is repeated using an alternative model configured to generate a population subset with a sufficient number of subjects,
segmenting the first generation population subset B based on the second model M2 into second generation population subset A and B; and
and wherein the model M comprises the first model M1 and the second model M2; and outputting a value indicative of a detection, or a prediction of risk, of the health condition in the subject based on said model M and wherein the health condition is selected from: a hypertensive disorder, a cardiovascular disease, a proliferative disorder, an inflammatory disease, an autoimmune disease, a metabolic disorder, a neurological disease, a hepatic disorder, or a pulmonary disease, and excluding a disorder of pregnancy.

2. A computer implemented method according to Claim 1 in which the model M2 is configured to segregate the first generation population subset B into second generation population subsets A and B in which the subjects in the second generation population subset A have a probability selected from the other of: a probability to have/develop the health condition lower than or equal to a predefined NPV cut-off; or a probability to have/develop the health condition higher than or equal to a predefined PPV cut-off.

3. A computer implemented method according to Claim 1 in which the model M1 is configured to segregate the population of test subjects into first generation population subsets A and B in which the subjects in the first generation population subset A have a probability to have/develop the health condition lower than or equal to a predefined rule-out cut-off probability; wherein the model M2 is configured to segregate the first generation population subset B into second generation population subsets A and B in which the subjects the second generation population subset A have a probability to have/develop the health condition higher than or equal to a predefined rule-in cut-off probability which is optionally a PPV cut-off.

4. A computer implemented method according to Claim 1 in which the model M1 is configured to segregate the population of test subjects into first generation population subsets A and B in which the subjects in the first generation population subset A have a probability to have/develop the health condition higher than or equal to a predefined rule-in cut-off probability; wherein the model M2 is configured to segregate the first generation population subset B into second generation population subsets A and B in which the subjects the second generation population subset A have a probability to have/develop the health condition lower than or equal to a predefined rule-out probability which is optionally a NPV cut-off.

5. A computer implemented method according to any preceding Claim, including an additional segmentation step comprising:
when the second generation population subset B comprises a sufficient number of subjects, providing a third model M3 configured to predict the presence or risk of the health condition in the second generation population subset B, the model comprising 1 to n variables, wherein the model M3 is configured to segregate the second generation population subset B into third generation population subsets A and B such that the subjects in the third generation population subset A has a probability selected from one of: a probability to have/develop the health condition lower than or equal to a predefined rule-out NPV cut-off; or a probability to have/develop the health condition higher than or equal to a predefined rule-in PPV cut-off; and segmenting the other of the second generation population subsets based on the third model M3 into third generation population subsets A and B.

6. A computer implemented method according to Claim 5 in which:
the first model M1 is configured to segment the population of test subjects such that the subjects the first generation population subset A have a probability to have/develop the health condition higher than a predefined rule-in cut-off probability;
the second model M2 is configured to segment the first generation population subset B such that the subjects in the second generation population subset A have a probability to have/develop the (health) condition lower than a predefined rule-out cut-off probability; and the third model M3 is configured to segment the second generation population subset B such that the subjects in at least one of the third generation population subsets have a probability to have/develop the health condition higher than a predefined rule-in PPV cut-off.

7. A computer implemented method according to Claim 5 in which:
the first model M1 is configured to segment the population of test subjects such that the subjects the first generation population subset A have a probability to have/develop the health condition lower than a predefined rule-out cut-off probability;
the second model M2 is configured to segment the first generation population subset B such that the subjects in the second generation population subset A have a probability to have/develop the health condition higher than a predefined rule-in cut-off probability; and the third model M3 is configured to segment the second generation population subset B
such that the subjects in at least one of the third generation population subsets have a probability to have/develop the health condition lower than a predefined rule-out NPV cut-off.

8. A computer implemented method according to Claim 6 in which:
the first model M1 is configured to segment the population of test subjects such that the subjects the first generation population subset A have a probability to have/develop the health condition higher than a predefined PPV rule-in cut-off;
the second model M2 is configured to segment the first generation population subset B such that the subjects in the second generation population subset A have a probability to have/develop the health condition lower than a predefined rule-out cut-off probability; and
the third model M3 is configured to segment the second generation population subset B such that the subjects in at least one of the third generation population subsets have a probability to have/develop the health condition higher than a predefined rule-in PPV cut-off.

9. A computer implemented method according to Claim 4 in which:
the first model M1 is configured to segment the population of test subjects such that the subjects the first generation population subset A have a probability to have/develop the health condition lower than a predefined rule-out NPV cut-off;
the second model M2 is configured to segment the first generation population subset B such that the subjects in the second generation population subset A have a probability to have/develop the health condition higher than a predefined rule-in cut-off probability; and the third model M3 is configured to segment the second generation population subset B such that the subjects in at least one of the third generation population subsets have a probability to have/develop the health condition lower than a predefined rule-out NPV cut-off.

10. A computer implemented method according to Claim 1, including one or more additional segmentation steps in which each segmentation step comprises:
when the nth generation population subset B comprises a sufficient number of subjects, providing a (n+1)th model Mn+1 configured to predict the presence or risk of the health condition in the nth generation population subset B, the model comprising 1 to n variables, wherein the model Mn+1 is configured to segregate the nth generation population subset B into (n+1)th generation population subset A and B such that the subjects in the (n+1)th generation population subset A have a probability selected from one of: a probability to have/develop the health condition lower than or equal to a predefined rule-out NPV cut-off; or a probability to have/develop the health condition higher than or equal to a predefined rule-in PPV cut-off; and; and segmenting the nth generation population subset B based on the (n+1)th multivariable model into (n+1)th generation population subsets A and B.

11. A computer implemented method according to Claim 10, in which a final segregation step employs a final model configured to predict the presence or risk of the health condition in the penultimate generation population subset B, the model comprising 1 to n variables, wherein the final model is configured to segregate the penultimate generation population subset B into final generation population subset A and B such that the subjects in the final generation population subset A have a probability selected from one of: a probability to have/develop the health condition lower than or equal to a predefined rule-out NPV cut-off; or a probability to have/develop the health condition higher than or equal to a predefined rule-in PPV cut-off;

12. A computer implemented method according to Claim 1, including one or more additional segmentation steps in which each segmentation step comprises:
(a) when the nth generation population subset B comprises a sufficient number of subjects, generating a (n+1)th model Mn+1 comprising 1 ton variables, configured to predict the presence or risk of developing a health condition in the nth generation population subset B, wherein the model Mn+1 is configured to segregate the nth generation population subset B into (n+1)th generation population subset A and B whereby when the subjects in the (n+1)th generation population subset A are added to the combined earlier generation population rule-in subsets A, the composite rule-in population has a probability higher than or equal to a predefined rule-in PPV cut-off to have/develop the condition; or when the subjects in the (n+1)th generation population subset A are added to the combined earlier generation population rule-out subsets A, the composite rule-out population have a probability lower than or equal to a predefined rule-out NPV cut-off to have/develop the condition; and.
(b) segmenting the nth generation population subset B based on the (n+1)th multivariable model into (n+1)th generation population subsets A and B.

13. A computer implemented method according to Claim 10, 11 or 12 in which successive segmentation steps are repeated until a population subset is generated that cannot be further segmented.

14. A computer implemented system for generating a model M for detecting or predicting risk of a health condition in a subject, comprising:
an input device; a processor;
a memory;an output device;
said processor operatively coupled to the input device, the memory and the output device, said processor configured with:
a module or means for providing a population of test subjects and measurement data for a plurality (n) of variables for each of the test subjects selected from biometric, life-style and/or physiological characteristics;
a module or means for providing a first model M1 configured to predict the presence or risk of the health condition in the population of test subjects comprising 1 to n variables, wherein the model M1 is configured to segregate the population of test subjects into first generation population subsets A and B in which the subjects in the first generation population subset A have a probability selected from one of: a probability to have/develop the health condition lower than or equal to a predefined rule-out cut-off probability; or a probability to have/develop the health condition higher than or equal to a predefined rule-in cut-off probability; **characterised by**:
a module or means for segmenting the population of subjects based on the first model M1 into first generation population subsets A and B;
optionally, a module or means for determining when the first generation population subset B comprises a sufficient number of subjects;
a module or means for providing a second model M2 configured to predict the presence or risk of the health condition in the first generation population subset B, the model comprising 1 to n variables, wherein the model M2 is configured to segregate the first generation population subset B into second generation population subsets A and B in which the subjects in the second generation population subset A have a probability selected from the other of: a probability to have/develop the health condition lower than or equal to a predefined rule-out cut-off probability; or a probability to have/develop the health condition higher than or equal to a predefined rule-in cut-off probability;
a module or means for segmenting the first generation population subset B based on the second model M2 into second generation population subset A and B;
wherein the model M comprises the first model M1 and the second model M2; and
means for outputting a value indicative of a detection, or a prediction of risk, of the health condition in the subject based on said model M and wherein the health condition is selected from: a hypertensive disorder, a cardiovascular disease, a proliferative disorder, an inflammatory disease, an autoimmune disease, a metabolic disorder, a neurological disease, a hepatic disorder, or a pulmonary disease, and excluding a disorder of pregnancy.

15. A computer program product stored in a non-transitory storage medium, said storage medium operatively coupled to a processor and said computer program product causing the processor to generate a model M for detecting or predicting risk of a health condition in a subject, the processor configured to implement the steps of:
providing a population of test subjects and measurement data for a plurality (n) of variables for each of the test subjects selected from biometric, life-style and/or physiological characteristics;
providing a first model M1 configured to predict the presence or risk of the health condition in the population of test subjects comprising 1 to n variables, wherein the model M1 is configured to segregate the population of test subjects into first generation population subsets A and B in which the subjects in the first generation population subset A have a probability selected from one of: a probability to have/develop the health condition lower than or equal to a predefined rule-out cut-off probability; or a probability to have/develop the health condition higher than or equal to a predefined rule-in cut-off probability;
segmenting the population of subjects based on the first model M1 into first generation population subsets A and B;
**characterised in that** the first generation population subset B comprises a sufficient number of subjects, providing a second model M2 configured to predict the presence or risk of the health condition in the first generation population subset B, the model comprising 1 to n variables, wherein the model M2 is configured to segregate the first generation population subset B into second generation population subsets A and B in which the subjects in the second generation population subset A have a probability selected from the other of: a probability to have/develop the health condition lower than or equal to a predefined rule-out cut-off probability; or a probability to have/develop the health condition higher than or equal to a predefined rule-in cut-off probability;
segmenting the first generation population subset B based on the second model M2 into second generation population subset A and B; and
wherein the model M comprises the first model M1 and the second model M2; and
outputting a value indicative of a detection, or a prediction of risk, of the health condition in the subject based on said model M and wherein the health condition is selected from: a hypertensive disorder, a cardiovascular disease, a proliferative disorder, an inflammatory disease, an autoimmune disease, a metabolic disorder, a neurological disease, a hepatic disorder, or a pulmonary disease, and excluding a disorder of pregnancy.
